# EUROPEAN PATENT SPECIFICATION

(11) **EP 1 384 141 B1**
(45) Date of publication and mention of the grant of the patent: **16.01.2019**
(21) Application number: 02729072.5
(22) Date of filing: 29.04.2002
(51) Int. Cl.: G16H 40/20, G16H 10/40, A61B 5/00, A61M 1/38, A61B 5/15, A61B 5/155, G06F 19/00, G06Q 10/08, G06Q 50/22

(54) **A SYSTEM AND METHOD FOR MANAGING INVENTORY OF BLOOD COMPONENT COLLECTION**
SYSTEM UND VERFAHREN ZUR VERWALTUNG DES INVENTARS EINER BLUTKOMPONENTENSAMMLUNG
SYSTEME ET PROCEDE PERMETTANT DE GERER L'INVENTAIRE D'UN PRELEVEMENT DE COMPOSANTS SANGUINS

(30) Priority: 28.04.2001 US 287122 P; 24.05.2001 US 865196
(43) Date of publication of application: 28.01.2004
(73) Proprietor: Fenwal, Inc., Lake Zurich, IL 60047 (US)
(72) Inventor: NG, Kok-Hwee, Elkhorn, WI 53121 (US); FORD, Ian, Albuquerque, NM 87106 (US); CONLEY, Alfons, Evanston, IL 60202 (US); MARCH, Edward, Barrington, IL 60010 (US); FREDERICKS, Chris, N, Libertyville, IL 60048 (US); PETERSON, Grant, A., Chicago, IL 60645 (US)
(74) Representative: Geary, Stephen
(86) International application number: PCT/US2002/013620
(87) International publication number: WO 2002/088930

(56) References cited:
- WO-A-96/36923
- JP-A- 8 240 589
- US-A- 4 922 922
- US-A- 5 812 419
- US-A- 5 854 423
- US-A- 5 970 423
- US-A- 6 104 939
- US-B1- 6 233 525
- US-B1- 6 256 643

## Description

### Technical Field

The present invention is directed to a system and method for automating a laboratory process. More particularly, the present invention is directed to a system and method for managing inventory of blood component collection soft goods in a blood component collection facility and for preventing the use of quarantined blood component collection soft goods for use in the blood collection industry.

### Background of the Invention

Blood component therapy is a popular method of treating medical conditions or diseases. Components may be removed from a patient's blood circulation to eliminate unhealthy components. Blood components may also be inventoried for future transfusion into an ailing patient. Rather than transfusing a patient with whole blood, the patient is given packed red cells, platelets, or plasma depending on the condition that is being treated. For example, a patient suffering from anemia needs red cells, and treating that same patient with whole blood would be wasteful and could actually be detrimental to the patient.

Blood components are removed from a patient's/donor's blood circulation by a process called apheresis. The removal of red cells and white cells is called cytapheresis or hemapheresis; removal of plasma is called plasmapheresis; and removal of platelets is called plateletpheresis.

During apheresis, I. V. lines are inserted into a donor's/patient's veins. Blood is drawn from the veins and transferred to a cell separator machine where it is spun in a centrifuge to separate the components of the blood for collection. The speed of the centrifuge can be varied to isolate specific components by their density. Unneeded components are transferred back to the patient and reinfused.

There are a number of facilities/organizations that are specifically set up to collect blood and blood components. Tracking donors, component handling, donor registrations, and the like are important aspects of the blood component collection industry. However, the blood component
collection industry has long recognized a void in the automation of collection processes at collection centers.

For instance, the current blood donation process generally comprises the following steps. First, when the donor arrive at the facility, he/she checks in at the front desk. The donor's paper chart is pulled, and his/her DMS record is pulled and checked. Next, the donor signs in, his/her weight is measured, and the weight is recorded on the paper chart. A name tag with a barcode is printed for the donor. The donor proceeds to a screening area where he/she is asked a series of medical questions. The answers given by the donor are recorded on the paper chart (new donors have additional questions). The donor's vital signs are taken, and these results are recorded on the paper chart as well. A small blood sample is taken from the donor and the protein level is measured. Again, these results are entered on the donor's chart.

Next, the donor proceeds to a waiting room where he/she waits to be called to a control desk. At this time, the donor's DMS record is updated with information from the paper chart. The collection facility designates a blood collection container and a blood collection kit for the donor. Bleed numbers are taken from a roll of preprinted barcode labels. There are three rolls of labels, each having different number ranges representing different blood types. The donor's name, donor number, and the date are printed on the appropriate label. Many labels are produced for each donor. One label goes on the donor's chart and several are applied to the collection bottle.

Finally, the donor is escorted with his/her chart and collection bottle/kit to an assigned bed where the donor's blood is drawn. The actual volume of blood drawn from the donor is manually recorded on the collection bottle. After the donation is completed, the donor's chart and the full blood container are transported to a processing desk.

Most blood center operators have managed to automate processes such as donor registration, blood sample, and product handling but are unable to do much about the record keeping and tracking of the blood collection process itself.

The ability of the centers to automate blood collection data depends on the built-in data communication capability of the blood or blood component collection device. Unfortunately, most collection devices currently in use in this field have very rudimentary and/or proprietary data communication interfaces. This makes the task of connecting the devices to standard computing equipment cost prohibitive.

For example, some of these devices transmit periodic binary data out of a proprietary pin-type interface. The periodic data rate and the maximum burst rate of these devices often approach or exceed the limits of most serial networks. Thus, in order to connect these existing collection devices to a serial network, new adaptor hardware, which is capable of interfacing with the proprietary interface of the collection device, is needed. Moreover, such a hardware add-on would require a communication driver for the host computer that receives the data from the collection device. This aspect would be even more complicated when high level messaging protocols are utilized for the communication.

Healthcare devices have been included in computer networks. For instance U. S. Patent No. 5,857,967 (Frid et al.) is directed to a healthcare device which comprises a web server and medical information thereon, and which generates HTML files on the fly for providing such information to a client at a remote location using a standard browser interface. The healthcare device of the'967 patent is universally accessible via a communication network using open standard network protocols. The healthcare device includes the web server that exchanges messages with web clients using the HTTP and HTML open standard protocols on the communication network. The web server handles HTTP commands received via the communication network that specify a predetermined Universal Resource Locator (URL) for the healthcare device. The HTTP commands are used by web clients such as a web browser to read medical information including measurement data and optional related information from the healthcare device. The web server packages the medical information into the HTML format and transfers the information to requesting web clients on the communication network using the HTTP protocol.

U. S. Patent No. 5,891,035 (Wood et al.) is directed to an ultrasonic diagnostic imaging system. The imaging system is capable of accessing images and information from internal and external databases by means of a web browser. Access to the images or information may be over a local network or over the Internet. The browser may be used to pull in system preset data or reference images from a reference image library.

WO 96/36293 is directed to a care management system for medication administration and patient care management and is concerned with ensuring the appropriate administration of care to a patient. The parameters which are monitored relate to the therapeutic process of delivering treatments to patients such as timings for terminating infusions, providing infusion parameters to pumps and providing alarms when there is an unscheduled suspension of infusion.

US 5970423 describes a blood component collection system with optimizer. Process parameters are derived from input /configured predetermined blood component yield based upon the maximisation of at least one process parameter. The blood collection procedure is then performed using these derived process control parameters.

### Summary of the Invention

The present invention is directed to a system and a method as defined in the claims.

The system and method may be used for networking a blood component collection facility in which the facility includes a plurality of independently operable blood component collection instruments. Suitably, at least one of the plurality of blood component collection instrument is an existing collection instrument having a first communication protocol. Suitably, the system includes a system computer, a communication conduit, a second communication protocol, and a protocol converter.

The system computer comprises a memory and a code segment. The code segment defines at least a portion of a blood component collection process. The communication conduit operably connects each of the plurality of collection instruments to the system computer. The second communication protocol facilitates communication on the communication conduit between the system computer and each of the collection instruments. The protocol converter operably connects to the communication conduit between the existing collection instrument and the system computer.

The protocol converter also converts the first communication protocol to the second communication protocol for communicating between the existing collection instrument and the system computer.

The protocol convertor can be a programmable communication controller. For example, the protocol convertor can be a Picoweb' communication interface, as is described below.

The invention is described by reference to the following description and the non-limiting accompanying drawings.

### Brief Description of the Drawings

Figure 1 is a block diagram of an embodiment of the present invention;
Figure 2 is a structural diagram of an embodiment of the apparatus of the present invention;
Figure 3 is a structural diagram of a second embodiment of the apparatus of the present invention;
Figure 4 is a flowchart of a daemon incorporated within the device of the present invention;
Figure 5 is an illustration of a system login computer screen;
Figure 6 is an illustration of a system welcome computer screen;
Figures 7 through 34 are illustrations of multiple computer screens which are used during setup and administration of the system;
Figures 35 and 36 are illustrations of computer screens which are used during the search function of the present invention;
Figures 37 through 76 are illustrations of various computer screens which are used during the reporting function of the present invention;
Figure 77 is an illustration of a computer screen used to monitor the instruments within a facility;
Figure 77a is an illustration of information provided on the computer screen of Figure 77;
Figure 78 is an illustration of a computer screen used during the dispatch function of the present invention;
Figure 78a is an illustration of information provided on the computer screen of Figure 78;
Figures 79 through 105f are illustration of various computer data input screens used with the wireless interfaces of the present invention. to 24a are illustrations of computer screens used during administration and setup of an embodiment of the present invention; and

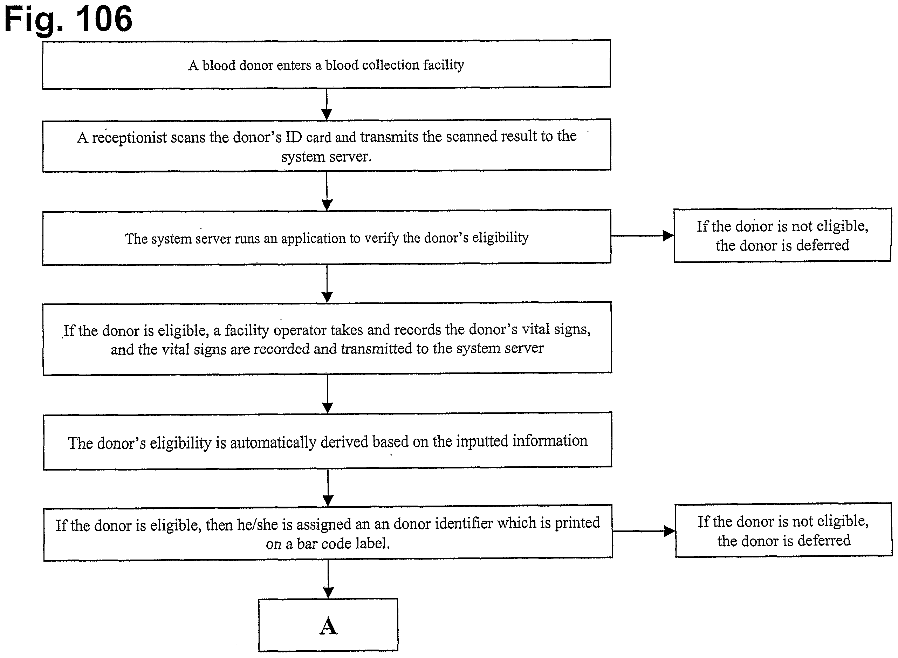

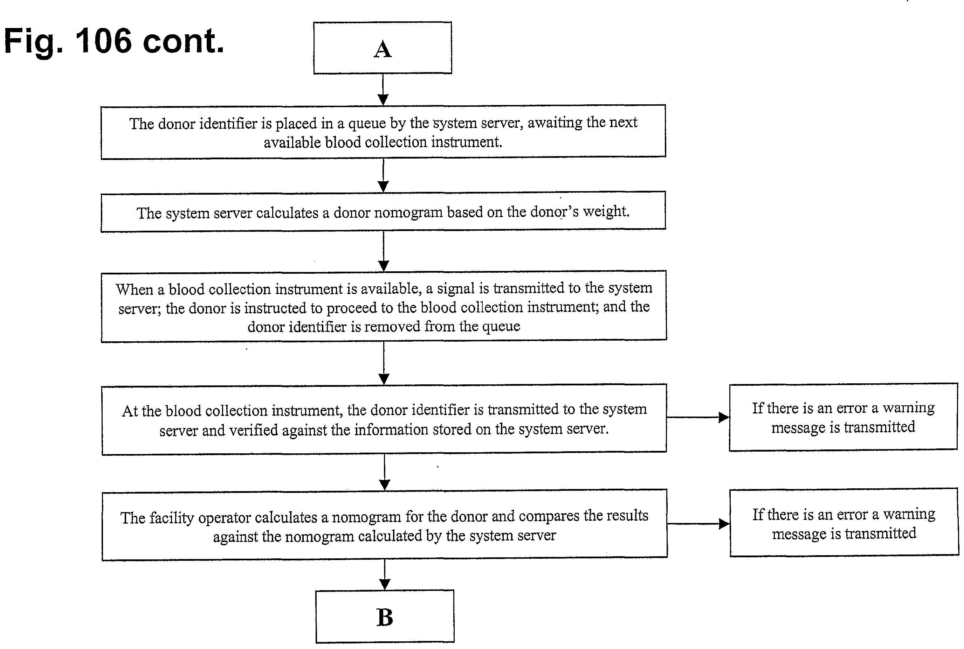

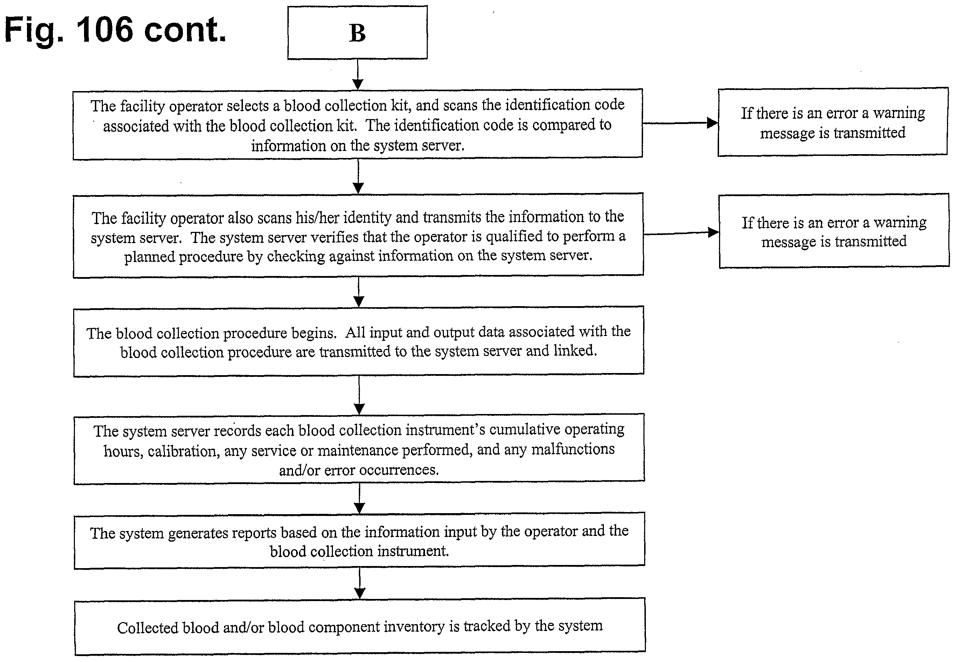

Figure 106 is a flowchart of a method for using the device of the present invention.

### Detailed Description

The present invention is directed to a system for collecting, using, and storing information in a biological fluid collection and/or processing facility ("facility"). The present invention can be incorporated into an existing facility's system via an upgrade to existing hardware and software. The present apparatus provides a data connection between laboratory instruments, including, but not limited to, existing blood and blood component collection instruments, such as the Autopheresis-C' instrument which is supplied by the Fenwal Division of Baxter International, Inc. located in Deerfield, Illinois, those described in PCT Publication No. WO 01/17584, U. S. Patent Nos. 5,581,687 and 5,956,023, and U. S. Serial No. 09/037,356, and biological treatment instruments, such as the pathogen inactivation instruments described in U. S. Serial. 09/325,599, and the collection facility's management information system which lends itself to automated tracing and/or tracking of donors and biological fluids data logging. Traceability is provided via integration of donor, operator, soft goods, and instrument data. The present invention further automates event reporting which is required for regulatory compliance.

The system is designed for a biological fluid collection and/or processing facility as an accessory to the instruments used in those facilities. The general purpose of the system is to increase the efficiency of processing biological fluids and aid in the regulatory compliance process.

This purpose is fulfilled principally through the collection of more information and more accurate information. Currently, facility staff must manually keep track of information such as by writing information on a clipboard, but the present system allows the staff and operators to skip the paper/manual steps. The system may also provide some of the following benefits: more accuracy and completeness in the data that is already being collected manually; more data collected for diagnostic use, which may give rise to better information with which to design or troubleshoot laboratory instruments ; more data collected for use by the center for generation of ad-hoc statistical reports, which could relate any number of variables such as donors per day/per time of day, rate of errors, collection amount by type of donor, etc.; more data collected for use by the center to determine the efficiency and error rate of different operators, which in turn can inform decisions to institute better training or could substantiate a complaint against a facility operator; greater efficiency on the floor, due to less paperwork; lower costs due to less office paperwork; ability to research all the detailed information on a single procedure, or on the history of a single donor, as a way to find information pertaining to a donor complaint, or something wrong with the product, or any other complaint or error; more complete records and statistical and trend reports to help ease compliance reviews; accurate monitoring of the facility procedures; collection of information that may help the facility's staff improve their efficiency/workflow.

The present invention also improves collection facility workflow by providing portable data input and monitoring devices and a method for tracking soft good utilization and coordinating restocking and reordering of the soft goods through remote access service.

The present invention is suited for medical facilities where product integrity and traceability are critical quality factors. Typically, the instruments, laboratory equipment, as well as data input
devices are connected to an Ethernet along with other data processing applications. The present invention is also suited for connecting legacy instruments that automatically transmit or can be configured to periodically transmit data via a serial or parallel interface and protocol converters. A computer acting as a server/gateway runs applications to receive the transmitted data and route them to database and hypertext markup language (HTML) applications. Each data packet bears a unique identifier which identifies the source of the data.

Users can perform data query and reporting on a local area network, through a wide area network, over the Internet, or a combination of two or more of these, using a standard browser application interface. Real-time viewing and updating of device operation can be configured for any number of devices on the browser. In addition, the server also presents abbreviated data to a wireless personal digital assistant (PDA) also running a standard application browser interface for portable information and viewing and alarm and event notification. The PDAs are also used for data input (through a keypad touch screen, scanning, or other entering method - all used interchangeably herein) in association with an apparatus operation. Thus, the present invention includes open standard architecture in a heterogeneous apparatus environment with real-time update and access of data, and portable data viewing, reporting, notification, and inputting.

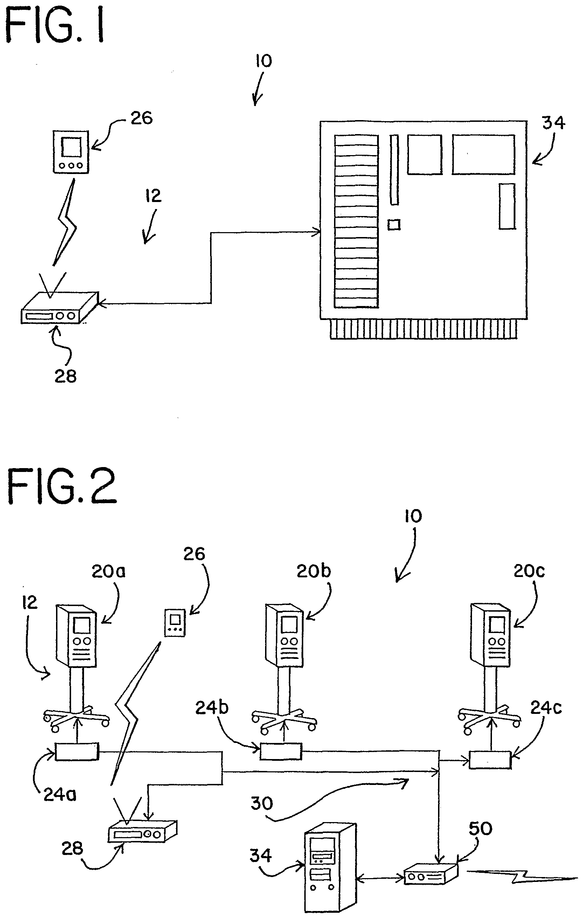

Referring to Figure 1, the system/apparatus 10 comprises a first network 12 comprising a system server 34 including a memory, a communication driver and an HTML application capable of running embedded javascript code and at least one wireless data interface, preferably a PDA and/or scanner 26, but more preferably enough PDAs and scanners to accommodate several facility operators and/or donors at a time and a wireless access point 28.

In a second embodiment illustrated in Figure 2, the apparatus 10 comprises hardware and software component parts and provides for inter-process communication. Figure 2 shows a first network 12. The first network 12 includes laboratory instruments 20a, 20b, 20c, serial/parallel to Ethernet converters 24a, 24b, 24c, such as a PicoWeb™ device by Lightner Engineering located in San Diego, California or a NetDev™ device by Fenwal Division of Baxter International, Inc., where needed, a first Ethernet 30, and a system server 34 including a memory, a communication driver for the apheresis instruments, a communication protocol converter, and an HTML application with embedded javascript code.

The first network 12 can communicate via the Internet through a network switch 50. The network switch 50, which can be incorporated within the system server 34, includes a processor which allows the switch to distinguish the sources of the information which it receives.

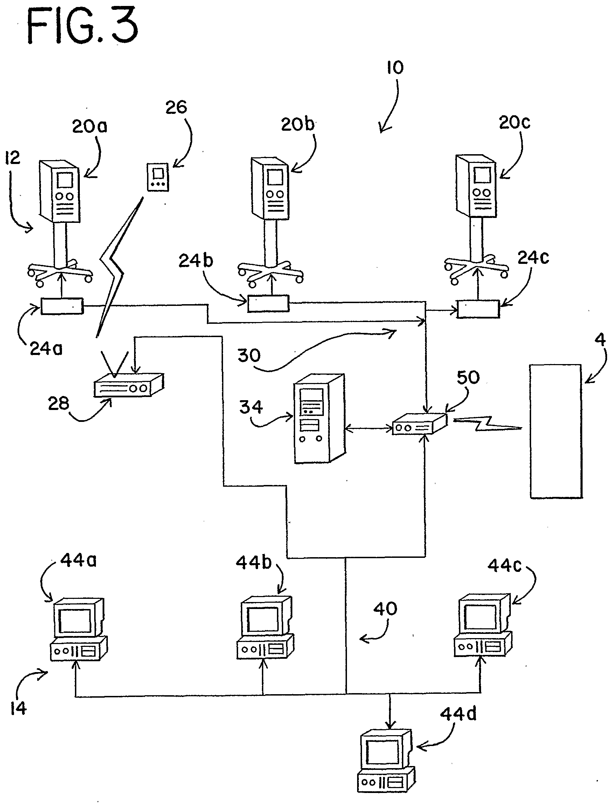

Figure 3 shows a pair of networks 12, 14. The network switch 50 provides the communication link between the networks 12, 14. Again, the network switch 50 includes a processor which allows the switch to distinguish the sources of the information which it receives. The first network 12 includes laboratory instruments 20a, 20b, 20c, serial/parallel to Ethernet converters 24a, 24b, 24c where needed, a first Ethernet 30, and a system server 34 including a memory, a communication driver for the instruments, a communication protocol converter, and an HTML application capable of running embedded javascript code.

The second network 14 includes a second Ethernet 40 and data interfaces 44a, 44b, 44c, 44d, e.g. personal computers to run server and browser software. At least one of the data interfaces 44a, 44b, 44c is equipped with a barcode scanner for setting up facility operators and associating them with preprinted badges. The second network 14 also comprises at least one wireless data interface, preferably a PDA and/or scanner 26, but more preferably enough PDAs and scanners to accommodate several facility operators and/or donors at a time and a wireless access point 28.

A central server 48, generally located at a remote site, may communicate with the first and second networks 12, 14 via the Internet using a communication link such as a modem, digital subscriber line, or the like with the network switch 50. The central server 48, therefore, can access data regarding the instruments 20a, 20b, 20c that are stored in the system server 34.

The first network 12 is primarily established between the system server 34 and the instruments 20a, 20b, 20c. This first network 12 is not directly connected to the Internet or any other subnetwork except through the network switch 50. The network switch 50 is adapted to prevent unwanted communication with external servers and/or other means of data communication while at the same time being configured to forward useable Ethernet datagrams broadcast packets (UDP) to all ports.

The system server 34 controls the distribution of data throughout the system 10. The system server 34 runs an operating system, such as a Linux machine running SuSE 6.4 or more preferably a personal computer running Microsoft 2000. The system server 34 receives data from an instrument 20a via one of the serial/parallel to Ethernet converters 24a and/or other interfaces within the apparatus 10. Accordingly, the system server 34 includes one or more Ethernet cards to connect sets of apheresis instruments 20a, 20b, 20c to the system server 34 and at least one additional Ethernet card to connect the system server 34 to the facility's office network which is also connected to the central server 48. The system server 34 also runs a web server, such as Apache or more preferably Microsoft Internet Information Server provided with Microsoft 2000.

Each instrument 20a, 20b, 20c connected to the apparatus 10 is identified by a unique number such as an internet protocol (IP) address and a serial number. Certain legacy instruments provide framing bytes on data packets coming through a parallel port. The serial/parallel to Ethernet converters 24a, 24b, 24c gather data from the instruments 20a, 20b, 20c and deliver the data into an Ethernet frame buffer. The data is transmitted via the first Ethernet 30 to the system server 34. Server software takes the data and outputs web pages of information. It should be noted that the Ethernet converters 24a, 24b, 24c are necessary for certain legacy devices and may not be needed in every application of the present system 10.

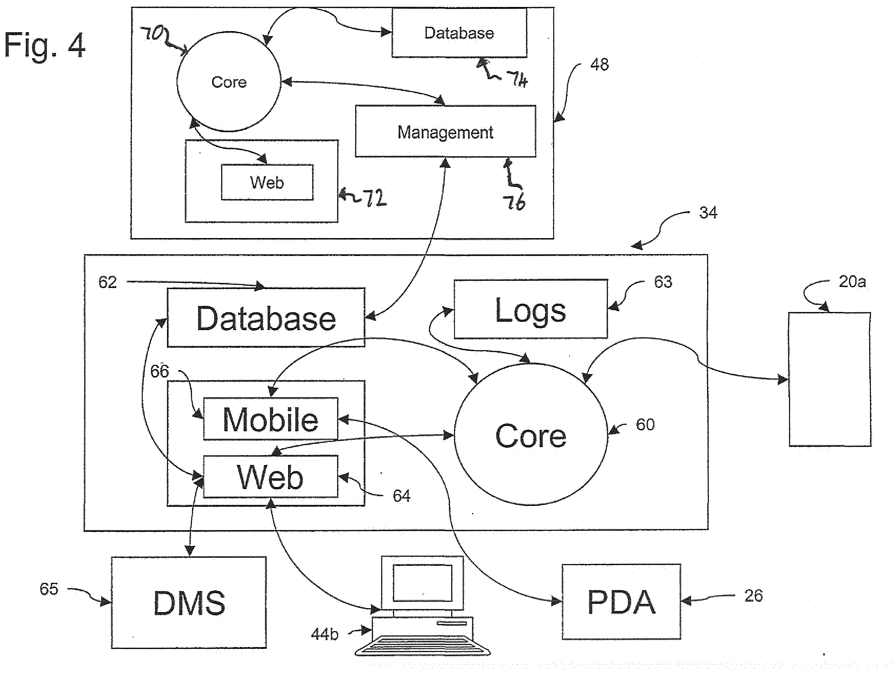

Referring to Figure 4, the instrument 20a is the primary source of data for the system 10. The instrument 20a may provide parallel data packets to the serial/parallel to Ethernet converter 24a which converts the packets to useable Ethernet datagrams (user datagram protocol/internet protocol (UDP/IP) packets). The first Ethernet 30 transmits the UDP data packets to the system server 34.

The software within the system server 34 performs two separate functions. The first function gathers data from the instruments 20a, 20b, 20c. This function receives the UDP packets from the first Ethernet 30. The second function outputs HTML files to web clients by sending and receiving remote method invocation (RMI) data. Accordingly, the server software comprises separate modules for performing these functions.

Still referring to Figure 4, a core module 60, comprising a java program, communicates with the first Ethernet 30 and also communicates with the other modules within the system server 34. The core module 60 handles access with a database module 62 and caches information from the instrument 20a that is monitored on a frequent basis via data interface 44b and/or the PDAs 26. The core module 60 also writes to a high resolution log filing system 63 and performs the bulk of the business logic.

First, the core module 60 receives UDP packets from one of the instruments 20a and tracks the instrument's process. A converter network protocol module contains a protocol describing network communications between the instruments 20a, 20b, 20c and the system server 34 and a converter boot procedure used in conjunction with a bootp server which contains the IP addresses for the instruments 20a, 20b, 20c. The bootp server contains the Internet protocol that enables a diskless workstation to discover its own IP address, the IP address of a bootp server on the network, and a file to be loaded into memory to boot the machine. This enables the workstation to boot without requiring a hard or floppy disk drive. The converter network protocol and the converter boot procedure modules are specifications and not software.

The data transferred from the instruments 20a, 20b, 20c to the core module 60 can be used to create HTML web pages for monitoring the instruments via a structured query language (SQL) open database connectivity interface (ODBC). The core module 60 writes to the database module 62, which includes a SQL database server, to save and manage the instrument data. Javascript is used to create database tables on the SQL server and creates definitions for each table and field. The SQL database server stores all apparatus data except for high resolution logs.

The SQL database server preferably uses MySQL and more preferably Microsoft SQL Server. The SQL database server saves the data into a disk array. Java code within the HTML files provides a SQL interface to the SQL database server 62.

A web module 64, comprising the web server, can access the SQL database server using the ODBC interface. The web module 64 serves the web pages on the second Ethernet 40 so that the instruments 20a, 20b, 20c on the first Ethernet 30 are not interfered. The second Ethernet 40 allows standards such as javascript and hypertext preprocessor (PHP) codes to be viewed. The javascripts and/or PHP can be used to query and search the database.

The web module 64 communicates with the core module 60 via RMI data transmission. The core module 60 sends RMI data to the web module 64. Hypertext transfer protocol (HTTP) data generated by the web module 64 are served to and received from the web browser 44b via the web module 64 and the second Ethernet 40. The web browser 44b can act as a central workstation for monitoring the workflow within the blood collection facility. HTTP data can further be served to and received from the facility's donor management system (DMS) 65.

A mobile module 66 controls the system server's 34 communications with the PDAs/scanners 26. Thus, PDAs/scanners 26, such as the Palm Pilot™ by Symbol, are also a source of data to the system server 34. Preferably, each PDA/scanner 26 includes a wireless RF link and a built-in bar code scanner. The wireless feature of the PDA/scanner 26 allows the users to move freely in a room such as a blood center and scan barcoded material knowing it was logged into the database. The human error from manually writing down a number onto a log sheet is, thus, eliminated.

The core module 60 communicates with the PDAs/scanners 26 via the mobile module 66 by transmitting and receiving RMI data to and from the mobile module 66. The core module 60 can also serve data regarding the instruments 20a, 20b, 20c, such as an instrument's screen display or status, to a PDA/scanner 26 in real time or near real time. Thus, the wireless access point 28 provides the link between the system server 34 and the PDAs/scanners 26.

The mobile module 66 communicates HTTP data to and from the PDAs/scanners. The . PDA/scanner 26 can be used to scan the barcodes of plastic disposable kits, bleed numbers, donor ID cards, operator ID cards, and the instrument itself, and transfer that information to the core module 60 via the mobile module 66. Data that was historically manually recorded at blood centers can now be barcoded and logged electronically and wirelessly via the PDA/scanner 26. Date and time are automatically logged with such information. Finally, a downtime module contains a java program that performs downtime tasks, including software updates.

The central server 48 is generally located at a remote site and preferably runs a Windows 2000 operating system. The central server 48 is also referred to as a headquarters (HQ) server. The central server 48 is connected to facility networks through an IP network and is, therefore, necessarily more powerful than the facilities' system servers 34 due to the larger database size. The central server 48 must be capable of contacting any remote server at any time. There is not a wireless base station 28 or instrument 20a, 20b, 20c at the HQ level. Personal computers at the headquarters office connect to the central server 48 through HQ office network (IP). Personal computers at the facilities may also connect to the central server 48. Other computer devices with a browser interface and internet/networking capability can also connect to the server with proper security passwords and/or identification.

Similar to the system server 34, the central server 48 includes modules that perform predetermined functions, including a central core module 70, a central database module 74, and a central web module 72. In addition, the central server contains a central management module 76, a database connect file, and an installation procedure.

The central management module 76 is an interactive java program used by HQ management to perform continuous backups and software updates while the database connect file is a file containing the password for the SQL server database. The installation procedure is a procedure for installing server networking and files necessary to start the initial facility network upgrade process, including a setup program.

The central database module 74 houses a database composed of all the facilities' databases merged together. The central database module 74 is designed to facilitate the database merge by insuring that the definitions of unique keys do not conflict. All data is collected by and lives in the facilities' database modules 62. There can be many such facility database modules 62 in communication with the central server 48. The system servers 34 are the servers for all communications with the donor management systems 65.

Optionally, a company operating several facilities, each having its own system server 34, may also have a dedicated central database. This dedicated central database is equivalent to the database module 62 except: (1) many of the functions of the database module 62 cannot be used because the central server 48 is not connected to any wireless devices or apheresis instruments ; and (2) an additional program is needed to run the dedicated central database with the contents of the several system databases. This synchronization program communicates directly with the system servers and updates any changes from the system server 34 to the central server 48.

In use, the facilities provide inputs to the system server 34 through an HTTP call for each procedure which is initiated from their donor management system before the system server will store data for the procedure. The facilities may issue HTTP requests for data from their system servers 34 for limited bleed summary fields, using a programmatic interface, in addition to the HTTP browser-based reporting interface from the central server 48.

The present apparatus 10 may be called a "distributed system ;" however, the system server 34 operates independently as if it were not part of a distributed system. The central or HQ server 48 takes initiative to copy data in both directions as needed.

The system server 34 always operates in server-mode with respect to communications with headquarters and other systems, and never operates in client-mode. The donor management system and the central server 48 operate in client-mode. In server mode, the system server 34 waits for requests and does not initiate transactions with other servers. This achieves the benefits of centralizing data management functions (like backups) while retaining the robustness of independent servers. The following is an illustrative example of the functioning of a system 10 of the present invention. The illustrative example described herein is adapted for use in an apheresis facility; however, it should be noted that the present invention can be used in any biological fluid collection and/or processing facility

### SOFTWARE

The system 10 of this embodiment includes software components for reporting, business administration, and technical facility requirements.

### Reporting

On the reporting end, the system 10 provides interactive reporting capability to any common computing platform, essentially web-based. Specific reports will be discussed below. The reports
are intended to be optimized for on-screen viewing, but are also printable. All reports may require user selection of a location or region when accessed from the headquarters database; however, this step may be skipped when accessed from the center database. Although the report requirements below do not include the location name each time, it should be part of the header of each report. The local time zone of the database server must be known by the system 10, and all time outputs in reports are stored and reported in the local time of the site where the facility procedure took place.

Data reporting can be customized for specific time periods, e.g. by week where a week is defined as Monday-Sunday, Sunday-Saturday, etc. The total hours of instrument operation may be displayed. The total hours of instrument operation references a pre-defined table of weekly operation hours which consists of seven data items representing hours, as in this example: mon=8, tue=8, wed=9, thu=9, fri=9, sat=6, sun=0.

Other reports may reference collection and other goals compared to actual values. There must be a center-wide goal entry screen to allow data input of goals to handle these cases. Goals are different for each center, and different for each day, week, month, and year.

### Business Administration

On the business administration end, the high resolution log is stored to get a time history, e.g. second-by-second, of an instrument's output variables. The log is filed so that it can be easily located in the file system, but not interactively within the software. The facility management has the option of turning the high-resolution logging feature on or off.

The high resolution log is generally stored at one second intervals for each facility instrument while the instrument is in run mode; i.e. during a operation, and is not stored at all while not in a bleed. This is accomplished by suspending logging if the instrument's mode code remains at 1 (indicating paused or not in a operation) for more than one minute. When it changes to another value, logging is resumed. The high resolution log is stored in a FIFO-type queue as disk space permits, at least one month of logging.

The following variables must be stored for each log interval: local date and time contents of the instrument's run packet (as described below).

Further to the business administration end, a medium-resolution log is stored to provide an occasional "snapshot" of the instrument's output. The medium resolution log is stored for facility purposes and is available the generation of reports.

The medium resolution log entries are generally made at these times: when any alarm/alert is issued by the instrument during any phase of a procedure; at every instrument keystroke; at every minute, if there hasn't been any log entries in the last minute, but only if the instrument is running (i.e. mode is not 1). The one-minute recording feature is controllable (on/off) at the site level.

In the case of an apheresis instrument, the following variables are stored in the medium-resolution log: date/time; reservoir sensor; PI pressure; P2 pressure; cuff pressure; M2 flow rate; plasma flow rate; cycle #; display; plasma volume; clamp status (4 clamps); mode code; keystrokes.

The administration end also includes a setup and procedure-related log (e.g., a bleed-related log). In order to meet the purposes of cost reduction and information availability, information on each procedure is stored in an interactively available form for a period of at least two years. The facility has the option of purging old data stored at the facility level. Meanwhile, the headquarters system staff has the option of archiving old data stored at the HQ level. Every log entry includes the absolute date and time (i.e. GMT, not local time). These general data requirements apply equally to the medium resolution logs (above).

In the case of an apheresis facility, the stages of the procedure are the instrument setup, procedure-program, arm-prep, remove-plasma, and disconnect donor. Instrument setup is performed without reference to any specific donor, and is, therefore, not linked to the donor or bleed in the database, (although it may have to be linked retroactively). The bleed procedure (from procedure-program through disconnect donor) is identified by a unique bleed number provided by the facility's system.

The events that are collected and logged include the those shown in Table 1. In addition, the system allows event types to be added with a minimum of redesign, because the selection of events to be logged is a requirement that could conceivably change after initial testing.

**Table 1: Event Logging**

| Event Type | O/A | Data to Log for This Event |
|---|---|---|
| Machine Setup | O | Operator ID |
| | | Instrument ID |
| | | Products |
| Procedure Program | O | Operator ID |
| | | Instrument ID |
| | | Bleed No. |
| | | Donor ID |
| | | Plasma Volume |
| Prep Arm | O | Operator ID |
| | | Instrument ID |
| | | Arm - Left or Right |
| Venipuncture | O | Operator ID |
| | | Instrument ID |
| Response to Remove plasma alert (donor still connected) | A | Operator ID |
| | | Instrument ID |
| | | Plasma Volume Collected (user entered) |
| | | Reason-List for UON or UUN (over or under draw) |
| Response to Disconnect Donor Alert | A | Operator ID |
| | | Instrument ID |
| | | Donor Reaction - yes or no |
| | | Free-Form Notes |
| Response to Reboot-Resync Alert | A | Operator ID |
| | | Instrument ID |
| | | Bleed No. |
| | | Donor ID |
| Response to All Other Alarm/Alerts | A | Operator ID |
| | | Instrument ID |
| | | Action List |
| | | Original Alarm/Alert Text |
| | | Time of Original Alarm/Alert |
| | | Procedure Continued? |
| | | Outcome List |
| | | Free-Form Notes |
| | | Other Actions |
| Product Performance (used when any part of a kit is bad and is discarded - provides information for the kit supplier) | O | Operator ID |
| | | Instrument ID (optional) |
| | | Product Failure? |
| | | Process Step |
| | | Product ID |
| | | Lot No. |
| | | Problem Code and Description (used only if failure is Yes) |
| | | Component Code and Description (used only if failure is Yes) |
| | | Videojet No. (only used if component is "separation device") |
| | | Reason (used only if failure is No) |
| | | Notes |
| Move Donor to a Different Instrument | O | Operator ID |
| | | Instrument ID (the new one) |
| | | Bleed No. |
| | | Donor ID |
| | | Reason-List |
| | | Was Collection Container Transferred? |
| | | Plasma Volume Collected (only if answer to previous question is Yes) |
| | | Outcome-List |
| | | free-form notes/reasons |
| Second Venipuncture (used only as a subroutine of other event types - see screen layouts) | O | Operator ID |
| | | Instrument ID |
| | | Outcome-List |
| Manual Saline | O | Operator ID |
| | | Instrument ID |
| | | Reason-List |
| | | Outcome-List |
| | | Free-Form Notes |
| Donor Reaction | O | Operator ID |
| | | Instrument ID (optional) |
| | | Outcome-List |
| | | Free-Form Notes |
| Procedure Termination (Abnormal disconnect donor) | O | Operator ID |
| | | Instrument ID |
| | | Donor Reaction? |
| | | Reason-List |
| | | Reservoir contents Manually Reinfused? |
| | | Cell Loss Volume (only ask if the answer to the previous question is No) |
| | | Free-Form Notes |
| Other | O | Operator ID |
| | | Instrument ID (optional) |
| | | Bleed No. (optional) |
| | | Free-Form Description |
| | | Free-Form Resolution |
| Override Log | O | Operator ID |
| | | Instrument ID |
| | | Free-Form Notes |
| Accuracy Messages | O | Operator ID |
| | | Message |

In regard to Table 1, in the O/A column, an "O" indicates that the operator initiates the logging from a menu, and it is optional. An "A" indicates that the logging is a required response to an alarm/alert.

The operator is identified by a unique badge number and log-in ID.

The "products" entry is a list of up to ten components of two barcode scans for each component. The first barcode identifies the product number. The second barcode identifies the lot number and expiration date. In some cases, only one component will be used, and in other cases, five will be used. The system allows up to ten for future needs.

The bleed number is printed on a barcode label generated by the DMS, and is scanned only once. The bleed number is tied to all future events until the completion of the remove-plasma event occurs or any instrument-setup event occurs. If the donor is moved to a different instrument, uses a different kit, is seen by a different operator, or any other changes occur, it is still the same bleed number as long as the same collection container is used.

The donor is identified by unique donor number. Donor tags may be printed with a photo ID upon entry to floor, or the donor number may be scanned from a paper chart. The ID number is a constant and unique identifier for the donor.

The Apheresis instrument is identified by serial number. For the O-type events, it must be operator entered (scanned), but it is system derived for the A-type events.

The 'action list' for alarm/alerts is a different list for each alarm/alert type, including such items as "check line", "check pump", etc. The user must be able to select one or more items from the action list. The 'reason list' works the same as action lists, but is used when a reason for an operator action is required. The 'outcome list' works the same as action lists, but it includes occurrences following the event in question (e.g. procedure continued, terminated, etc.). There is only one customer-defined outcome list which is used for all types of log entries where listed.

The 'Override log' event is only available in recovery mode, not as a general purpose log entry. It is intended for supervisor use to log a change to an existing log entry. It does not change the bleed summary or erase any data, or change what is shown in reports.

The 'Accuracy message' log is not operator initiated. It is automatically logged when exception messages are shown as listed under 'Operational Accuracy' requirements. It logs the message that is shown to the user without user intervention.

In addition to and separate from the log of events listed above, summary data for each bleed or partial bleed is stored. These records are not specific events, but are totals or averages over the whole bleed. The summary values are calculated either from (1) data provided by the apheresis instrument (the same data that optionally forms the high-resolution log); (2) the log of operator events; or (3) from the customer donor management system. The summary includes the following data items: bleed number, donor number, date and time of most recent instrument-setup logging, date and time donor arrived on floor (captured from donor system), date and time of arm prep logging, date and time of venipuncture logging, date and time procedure started (from apheresis machine), date and time of remove-plasma logging date and time of disconnect-donor logging, all products/lots used in the bleed, apheresis instrument (if more than one instrument, store latest instrument used), flag if more than one apheresis instrument used, operator who performed instrument-setup (if multiple, store latest), number of alarm/alerts associated with instrument-setup, number of instrument-setups performed, operator who performed the procedure program (if multiple, store latest), number of alarm/alerts associated with procedure-program, the operator who performed venipuncture (if multiple, store latest), number of alarm/alerts associated with the run, the operator who performed remove-plasma (if multiple, store latest), the operator who performed procedure-end (if multiple, store latest), total number of alarm/alerts, the latest AC rate set (it could be changed mid procedure), the total plasma volume collected as reported by the apheresis machine, total plasma volume collected as reported by the user, the remove-plasma event, target plasma volume as determined by a calculation based on the facility system, total VP time - calculated by disconnect time minus VP time, or use a value from zeropage, total AC used - based on volume of AC pumped for the current bag + (number of bags used completely * volume of each bag), any overdraw? (defined by site-entered value, e.g. 8% over the programmed volume), any underdraw? (defined by site-entered value, e.g. 8% under the programmed volume), any no-take? (defined by site-entered value, e.g. less than 100 ml) cell-loss volume (if abnormal disconnect), donor reaction - yes or no (based on the existence of a Donor-reaction event, or the donor-reaction field set in the Disconnect-donor or Procedure-termination events), reviewer, review date, review notes, whether the bleed is classified as an exception - yes or no (in order to determine this, a center-level setup function must be available to select which of these things indicates an exception: 2nd VP, procedure termination, cell loss, donor reaction, over/under-draws, misprogrammings, "other"-type log entry, reboot-resync log entry, and the reason that the bleed is classified as an exception (e.g. "cell loss" or "2nd VP"). Additional fields may need to be stored in the summary in order to produce the required reports.

Only the procedure-program, change-instrument, and reboot-resync associate the bleed number to a instrument. All other events event types are logged in relation to the instrument. Therefore, in order to satisfy all of the above requirements, the system tracks the events associated with the instrument(s) in use, and calculates the bleed summary based on that information. The bleed summary is be calculated after the fact without user intervention. In other words, there is not a user input required to signal the end of a bleed. For example, if a venipuncture event is recorded, and the next event for the same instrument is an instrument setup event, then it is assumed that the previous bleed ended in some unknown way (perhaps a power failure), and that the bleed summary record should be stored with incomplete information.

The bleed summary has the option of an attached review (reviewer, date, and notes), which an auditor with the appropriate privileges shall be able to add and edit. The review information must be visible to all users who have access to the bleed summary.

Each logged event may be edited by a supervisor who has appropriate access to the system. However, the old version of the record must also be stored and visible for comparison. Only the new edited version will be used for calculations in reports.

Outside of the bleed events detailed above, the system must log these maintenance events, along with the operator who performed them, the absolute date and time, and the Apheresis machine.

**Table 2: Maintenance Event Logging**

| Event Type | O/A | Data to Log for This Event |
|---|---|---|
| Daily Maintenance | O | Operator ID |
| | | Instrument ID |
| | | Actual Weight for Weight A |
| | | Actual Weight for Weight B |
| | | Cleaned Instrument? |
| | | Free-Form Notes |
| Weekly Maintenance | O | Operator ID |
| | | Instrument ID |
| | | Step That Failed, if any |
| | | Free-Form Notes |
| Monthly Maintenance | O | Operator ID |
| | | Instrument ID |
| | | Step That Failed, if any |
| | | Free-Form Notes |
| Field Service Report (FSR) | O | Operator ID |
| | | Instrument ID |
| | | Field Service Completed? |
| | | Reason-List (only stored if FSR NOT completed) |
| | | Free-Form Notes |
| Out of Service | O | Operator ID |
| | | Instrument ID |
| | | Reason-List |
| | | Free-Form Notes |
| Back in Service | O | Operator ID |
| | | Instrument ID |
| | | Resolution Technician - Free-Form |
| | | Reason for Service (pick list) |
| | | Part Replaced (pick list) |
| Audit Log (to be logged only by | O | Operator ID |
| auditors with appropriate | | Instrument ID |
| permissions) | | Free-Form Notes |

In regard to Table 2, Weight A and B for daily maintenance references two pre-defined calibration weights, such as 500g and 1000g, which may be customer defined. When an out-of-service event is logged, the system automatically removes the instrument from the inventory so that attempts to use it will result in the appropriate alarm/alerts as described elsewhere. However, the back-in-service event does not reinstate the instrument. This step is done separately.

The system also stores the history of each instrument added to or subtracted from the facility's inventory. The inventory function is not associated with the maintenance event logging of the out-of-service and in-service events.

For each instrument, the manufacturer identity, the model name/number, the serial number, and the enabled/disabled flag are stored. Each time an instrument is added to the inventory, the following information is also stored: the location (center), the date added, the bed number (e.g. 1, 2, 3..., as opposed to the long serial number), and any notes. Each time an instrument is removed from the inventory, the following information is stored: location - from where it was removed, the date removed, and the location or description of where/why it was moved. An instrument that is not in inventory is not considered an "authorized (or enabled) device."

The system also maintains a log of the disposable kits inventory. All disposable lots that are received by the facility are logged. The following information is stored: the product number, the lot number, the record added by the operator, the lot quantity, the lot expiration date, the date that the lot was received, whether the lot was released for use, the date that the lot was released for use, whether the lot was quarantined, if quarantined, the reason for quarantine, whether a sterilization certificate is on file, and the directions for use. Facilities may select whether they want to use the fields "sterilization certificate on file" and "directions for use" during setup.

In order to handle logging of product performance issues and reporting of products by descriptions, the following fields must be stored in a product look-up table which may be edited via a setup function: the product category - e.g. "needles", "kits", "solutions", etc., the manufacturer's product number, the UPN (universal product number - this is the number that appears on barcodes), the product description.

In order to handle reporting of product performance issues by problem code, a lookup table of problems must exist. Each specific problem must belong to a general problem group (e.g. "leaks in tubing" belongs to the general group of "leaks".) The lookup table is not edited by the facility.

This section details the requirements for alarm/alerts, which are messages presented to operators asynchronously, which is to say that they can occur at any time unrelated to operator inputs. (There are also warnings given in the course of operator input.

There are two general types of operator alarm/alerts: (1) alarm/alerts originating from the instrument; (2) alarm/alerts originating from the system. Alarm/alerts that have not been dismissed by an authorized operator are considered active in an alarm/alert queue. An alarm/alert may not be assumed to have been read until the operator dismisses it by some action (such as pressing an OK button).

Operators can be authorized for handling alarm/alerts on a fine-grained level by the center supervisor; i.e. each operator can be authorized for any subset of alarm/alerts. In addition, an operator may be flagged for training for a certain alarm/alert. This affects reporting only.

If an operator is not authorized to handle an alarm/alert, he can override the restriction by getting the approval of any supervisor, in the form of a badge-scan. To handle this requirement, the administrator must be able to select which operators are supervisory during setup.

Each alarm/alert type can be set up by the center supervisor to be one of three status levels: ignored, auto log, or manual log. The 'manual log' type requires a log entry to be entered to dismiss the alarm/alert. The 'auto log' type allows the operator to dismiss the alarm/alert and automatically makes a log entry.

Each alarm/alert type can be flagged by the facility supervisor to be any combination of the following states: operator-related, disposable-related, instrument-related, instrument-setup-related, procedure-program-related, procedure-run-related, critical, count-for-reports (makes it appear on alarm/alerts by operator and instrument report).

Single-user devices (PDA devices) may be available for each operator to interactively input data and process alarm/alerts. These devices are operator-specific, not facility-wide. The items that are displayed in this context are: full alarm/alert text for alarm/alerts routed to the current operator.

Routing alarm/alerts will depend on the hardware to be used. If mobile devices are used, then alarm/alerts will be routed to operators whenever the system knows that the operator is using a certain device. The alarm/alert must not interrupt the current entry screen. All operators may view all alarm/alerts (but only authorized operators may dismiss or log resolutions).

At login, whenever a password is required, the operator must either scan a set of instruments or press "All" to associate a certain set of instruments (or all instruments) with the operator. Alarm/alerts are initially routed only to operators associated with the instrument, but after a site-selected timeout period, the alarm/alert routing is widened to go to all operators, and the signaling (beeping or flashing) becomes more pronounced.

As an exception to the above routing requirements, a nomogram check alert will always be routed to the operator who logged the procedure-program instead of the operators associated with the instrument.

Instrument alarm/alerts are predefined by the instrument firmware. The present system will use the same alarm/alerts that the instrument generates.

System-generated alarm/alerts will include the following type. If a bleed is apparently in progress (based on logging of events by the operator) and no procedure log data has come from the instrument in the three seconds prior to the operator's log, then an alarm/alert must occur to warn the operator that the instrument is not working, or the network is not working or not connected.

If an instrument is detected to be in run mode but the instrument is not an authorized device, an alarm/alert will occur to warn the operator that an unauthorized device is in use. An alarm/alert is generated when the instrument is powered up, as a diagnostic tool to verify that it is connected properly.

An alarm/alert is generated if there is a mismatch between an instrument record and its previous record, such as could occur if the same Ethernet MAC address is used on two devices, or if someone swaps the networking connections or devices. This requirement is only necessary if the system design would potentially permit such an error to occur, such that the actual source of the data is unknown.

If the system is rebooted during one or more bleeds, it must figure out which instruments are in a bleed process after it comes back on-line. To associate the running instruments with the data that may have been collected before the crash, it generates a reboot-resync alarm/alert for each instrument that is running. The operators' responses to this alarm/alert re-associate the instrument with the donor and bleed numbers.

The facility has the option of running as many as ten independent display screens at the same time, and is able to choose which type of display to show on each screen. These are intended for mounting where all floor staff can easily view the screens. All displays support standard monitor sizes and resolutions.

One status screen is the overview screen. The overview screen includes highly summarized compacted data items shown for each instrument. Depending on the monitor size and number of instruments, the overview screen may be scrollable, or two or more screens may be needed to display the status for all instruments. The overview screen should match the floor layout of the instruments in groups or areas.

Another status screen shows the instrument number (e.g. 1, 2, 3, not the serial number), alarm/alert conditions - just a highlight if there is an active alarm/alert, instrument display text, the amount of plasma collected in this procedure, a bar chart of plasma volume per programmed amount, the operating state of the instrument (out of order, not in use, in use, or on reserve), and an indicator if the instrument is associated with at least one operator.

Another status screen displays the full alarm/alert text for all active alarm/alerts, with the related instrument

Another status screen is a dispatch screen. The dispatch screen is similar to the overview screen. However, the dispatch screen adds an interactive function. The dispatch screen shows these data items in the same layout as the overview screen: instrument internal number (e.g. 1, 2, 3, not the serial number), a
bar chart of plasma volume per programmed amount, the operating state of the Apheresis machine (out of order, not in use, in use, or on-reserve)

The dispatch screen includes a function which allows an instrument to easily be placed on reserve. "On-reserve" is a pseudo-state of the instrument that is only relevant within the dispatch screen itself, and is never communicated or stored in any other part of the system. When on reserve, a instrument stays on reserve for 20 minutes or until its actual state changes to a run state.

The system comprises two levels of accuracy checks: (1) dual entry - the most stringent, and (2) confirmation - slightly less stringent but quicker. The system requires dual entry of the target collection volume because accuracy of this entry is paramount. One entry is inputted into the instrument as is currently done. The second entry is calculated based on the donor weight, which is passed automatically from the donor management system (DMS). The calculation is based on a mapping of weight-ranges to volumes (nomograms), which will be controlled by a setup function. If the calculated nomogram and programmed value do not match, an alarm/alert is issued.

The system also requires dual entry of the donor number and bleed number. These numbers are originated by the DMS, printed on barcode labels, and also inputted pushed into the system. The system compares barcode scans with the directly passed values and prevents entry of the scanned barcodes if they do not match. The comparison of donor and bleed numbers is done as a unit, not each separately, since there is only one correct donor number for each particular bleed number.

If an event is logged, but it is out of sequence with the previous event on an instrument, then a warning is displayed but the events are logged as usual. Sequencing is determined by a table of A-B pairs where "A" and "B" are two events and "B" normally follows "A". The table may be determined at design. For example, if a venipuncture follows a donor-disconnect event, this signals a warning because the entry (disconnect, venipuncture) is not in the sequence table. An exception is the order of the arm-prep and procedure-program events which may be interchanged.

If an expiration date is scanned in any context, and the date is earlier than the current date, then a warning is displayed and the operator may continue or go back and scan a different item.

If an instrument is scanned, and that instrument number is not an authorized and enabled device, then a warning is presented and the operator may continue or cancel.

If a lot number is scanned, and that lot number has not been entered as a released lot, then a warning is presented and the operator may continue or go back and scan a different item. In order to handle this, the facility management must enter released lot information as defined under data storage requirements - disposable lots.

If a lot number is scanned and that lot has been entered as a bad lot, then a warning is presented and the operator may continue or go back and scan a different item. In order to handle this, the facility management must enter lot numbers and associated messages in a bad-lot list, with product numbers added by operator, and dates. The list may also be broadcast to all other facilities.

If a bleed number is scanned/entered, and that number is already in the database, a warning is presented and the operator may continue or cancel.

Scanning a barcode is an insufficient action to commit permanent data storage of information. Any scanned input must require the operator to use keyboard or touch-screen input to confirm/verify the scanned values.

If an instrument setup is logged, and the instrument has not had a daily weight scale verification logged within the last 24 hours, then a warning is presented and the operator may continue or cancel.

If an instrument setup is logged, and the instrument has not had a field-service-report logged within the last year (or time period specified by customer), then a warning is presented and the operator may continue or cancel.

When an abnormal-disconnect event is logged, the computed value of cell loss volume must be suggested to the user, who may accept or change the suggested value.

In order to assist the operator in making the correct log entries, the operator must be able to view all the log entries that have been made for any bleed that is in progress. For bleeds that have ended, this information is available in reports.

If a calibration weight is entered in the daily maintenance log, and the measured weight is more than some pre-defined percent variance, then a warning is presented and the operator may continue or cancel.

If a venipuncture event is logged and the donor requires a blood sample, a message is given to this effect.

When an Hb detect alarm is logged, the operator is notified what number Hb detect this is for the bleed, e.g. first, second, etc.

When a venipuncture event is logged, the operator must re-scan the bleed number, and if the bleed number does not match the bleed number scanned for the procedure-program event on the same instrument, an error message must be presented, and the operator may not continue.

The system database server is secured by password protection (at least), and the passwords are different for each installation and changeable by the administrator at any time. Read and write access to each of several different areas of data are separately controllable.

Users are identified by a user ID, password, and scannable badge number. The user must log on using the user ID and password. Passwords are 4 or more characters in length and may not be the same as the login ID.

Relaxed security is tolerated in the case of a mobile device, under this condition: If the device was used by an operator in the past hour and the same operator logs in again on the same device, no password is required, only the operator's ID number is required.

Relaxed security is also tolerated in connection with a mobile device where an operator logs on using the user ID and password, and scans his badge number, then when he/she logs on again during the same shift (a center-selectable time period), only the badge number is required to validate the operator.

Users and passwords are maintained at both the facility level and the headquarters level, separately. Facility-level users will only have login access at that facility. Headquarter-level users will have login access at all facilities. The distinction of the two levels does not grant any privileges to the user. Rather, it only specifies at what site the user records are managed.

Operators are able to choose their own passwords and change them at any time, but not on a mobile device.

To handle forgotten passwords, the system includes a method for an administrator to reset a selected user's password to a random-4-digit number, then the operator must be forced to change his password on the next login, and within 15 minutes. After 15 minutes the account will be unusable until the password is reset again by the administrator.

To handle occasional password changes, the system includes a means for allowing an administrator to flag all accounts to force a password change on the next login.

All network devices must be authorized by manually entering their serial numbers or MAC addresses, including wireless devices, such that a new, unrecognized device cannot successfully send or retrieve data.

The system may support as many as 100 instruments and up to 20 operator devices. These will generally be in a 6:1 ratio.

The headquarters system must support merging the databases from up to 90 facilities.

The system monitors operator performance. At maximum scale when all devices are in operation, the system should never enter into a sustained period of slow performance lasting more than one minute, where slow performance is defined as a 2-second lag between operator input and the next prompt. A lag of up to one second is acceptable under normal operations.

Because of the possibility that the facility will develop software linking to the system, and that software could place unforeseen demands on the system, such as excessively large or non-optimized database queries, facility extensions must be kept as an exemption to the performance requirements. The facility's extensions can be run during hours of low-use or non-use of the instruments if this turns out to be a problem.

The system must be able to operate continuously for at least 18 hours at full utilization, out of each 24-hour period. The system may be designed to run internal downtime procedures, in which case the instruments may not operate during that time. The downtime procedures may not last more than 4 hours in any 24 hour period. The customer must have the option to schedule the downtime procedures at a time appropriate for them. Each facility has the option to continue running the system during the remaining hours in order to run software extensions that interface to the system.

### Technical Facility Requirements

Underlying many of the technical requirements is the fact that, typically, there will not be IT staff available at the facilities, and so the system must be relatively self-correcting and require few inputs.

A diagnostic mode is provided to ensure that the mobile devices can communicate full-circuit to the instruments.

The operational state of the system server (i.e. running or down) is detectable by the instruments.

There are a number or reliability requirements. For instance, the mobile devices and instruments reliability do not have an impact on system reliability because they are redundant and replaceable. The number of extra mobile devices needed to account for failures can be determined after installation. However, the reliability of non-redundant commodity equipment, such as the database server, is not considered a formal requirement

In the event of theft, fire, or irreparable physical damage to the database server, such as disk failure, the system must be able to be rebuilt with new components and loaded with archived data within 12 hours, and the number of hours of data loss in this case may not exceed 4 hours. The high resolution logs are exempt from the data loss requirement.

In the event of unexpected shutdown of the server, or crash, or power loss, the server reboots and continues operations with a maximum data loss of the period one minute prior to the shutdown. When the system is rebooted, it resumes logging data related to bleeds that were started or were continued during the downtime.

The system is be fail-safe between components, i.e. failures in one component may not cause data loss or the need to reboot the rest of the system. In particular, communication failures to a mobile device are recoverable without user intervention, and power loss of any component including the central server should not require user intervention other than restoring power.

As conceived, the instruments and NetDev devices (where necessary) use UDP/IP network packets. Therefore network packets are verified at the UDP level. As a result, there is some data loss of the high-resolution procedure logs. The allowable losses may be one sample per 60 seconds from each device. Therefore, at least 59 samples per minute are be stored. These 59 samples must be perfect.

False-positive network packets may occur where the packet is read incorrectly but the checksum happens to match so it is presumed to be correct. Reliability in this case is generally 99.99 %.

A set of entry screens is provided to allow the input of bleed-related log information and maintenance log information, to be used after a period of downtime during which paper logging was used. Medium-resolution and high-resolution logs will not be reconstructed in the event of downtime; they will be considered expendable in this situation.

Further, the system has the following compatibility requirements. Donor and bleed number barcodes that have been printed by another customer system in code 39 or UCCEAN 128 formats, are readable by the system. Up to 20 alphanumeric characters are supported. If using UCCEAN 128, the facility must enter the barcode field identifiers for bleeds and donors in a setup function.

Permanent donor number barcodes are generally 9 characters, e.g. A00001888 - where A00001 is a consecutive number (i.e. all the way thru Z99999) and 888 is a 3 position unique facility number.

Bleed number barcodes are generally ten characters, e.g. 00MMIA0001 - where 00 is the last 2 digits of the year, MMI is a unique facility identifier, and A0001 is a consecutive number thru Z99999.

Instrument barcodes that have been printed in UCCEAN 128 format, are be readable by the system. Instrument serial numbers allow up to 30 alphanumeric characters, and use field identifier 21.

Operator badge barcodes are printed by Baxter in UCCEAN 128 format and are readable by the system. Badge numbers allow up to 15 alphanumeric characters, and use field identifier 91.

Product-related barcodes are printed by any product manufacturer in UCCEAN 128 format and are readable by the system. Up to four fields may be combined in any combination up to three separate barcodes, or all together in one barcode. Field identifiers are as follows: universal product code (UPN) - field 01, lot number - field 10, expiration date - format YYMMDD - field 17, and quantity - field 30.

Still further, the database server should be accessible by widely used methods, so that the facility can access data for interfacing with other existing or new systems. Unless other methods are proven adequate, this implies that the database will use standard SQL accessible through, for example, Microsoft ODBC and/or a database driver that runs under Microsoft Windows. Direct database access is the primary method to allow the system to interface with other systems. Also, the security considerations may limit the manner and level in which a facility can access the database.

Instructions are be provided to enable a trained database administrator to export portions of the headquarters database, selected by date range, to a transportable file format, which, for example, may be a Microsoft Access database format. This will be used to export data for review by regulatory agencies.

The system provides a method for a donor management system to send a new bleed number, time donor entered floor, donor number, whether or not a blood sample is required, and donor weight. The system does not the use of donor and bleed numbers that have not been sent by the facility system. The facility system initiates this transfer. If the DMS does not pass the donor and bleed numbers, no data will be logged for that bleed.

The system also provides a way for a donor management system to obtain summary data items for each bleed. The facility system also initiates this transfer by requesting the summary for a given bleed number. The items are: bleed finished - yes/no (if no, then the summary data should not be considered accurate), arm used, donor reaction - yes or no, plasma volume collected, instrument number, over- or under-draw, and reason if so, cell loss volume, and donor number.

The system server includes a mechanism for sending messages to a particular instrument.

The system also includes requirements for data transfer between the facility database and the HQ database. The facility database must be designed with a location identifier in each applicable record so that multiple system databases can be merged without key violations (e.g. an ABRA 4-digit code).

All management software functions and reports are available from the application server at the facility, and also from the application server at the facility database. When running off of the HQ database, each function and report must request user input to determine which location or region to affect. When running at the facility, the functions do not request such input. It is not necessary for the operator logging functions, alarm/alert functions, or any other real-time functions to run against the facility database.

The system server is be backed up to the HQ server at intervals assigned by the facility, and only the changed records will be transferred. The backup includes the resolution logs (bleed-related data), the instrument-related data, maintenance-event data, and disposable lot data.

The facility databases are polled every 15 minutes by HQ (or another user defined interval) and any changed records will be copied to HQ. Facility databases are, therefore, only temporary stores, and do not need any other backup scheme. The HQ database may be backed up with a tape backup system or any other suitable means.

The provider has access to the high resolution procedure logs, ad-hoc one at a time. This is accomplished by sending the logs automatically from the system server to the HQ server, and allowing the provider access to the area of the HQ server where logs are stored. The high-resolution logs may be copied during each night from the facilities to HQ.

Software upgrades are handled by action at the HQ level only, and must not be designed to normally require center-level user interaction.

The system and HQ servers typically run on Microsoft Windows NT 4.0 or later. The database servers typically run on Microsoft SQL Server 7.0 or later.

### DATA INTERFACING

The present system relies upon data interfacing from three primary sources: the workstation(s), the wireless PDAs, and the facility instruments. The interfacing is accomplished via web browser technology. Therefore, the data interfacing comprises several web pages for data input and verification.

### System Workstation

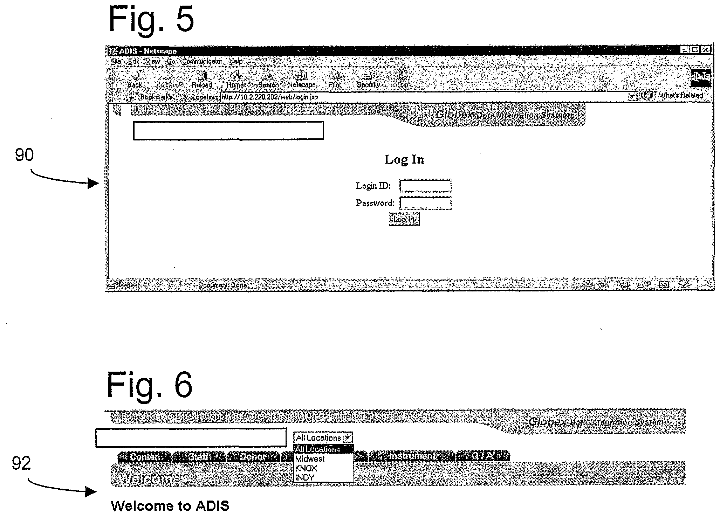

Referring to Figures 5-78a, the screens for operating and monitoring the system from a web-based workstation are illustrated. These screens are navigated very simply. Since every screen includes a menu of options, the user may access any particular screen by simply clicking on a menu item.

From any screen, any one of the following options may be selected: search (search by various criteria for bleed numbers), location (HQ only: change the location or region to supply data to the current and subsequent reports), setup (leads to a number of setup screens), monitor (shows live machine monitor), dispatch (shows machine dispatch status), help (a help system), and logout (ends session, there is also a timeout which can be defined in setup).

Referring to Figure 5, the login screen 90 is where the user logs into the system. Once the user has accessed the server through their browser, they will be asked to log into the system. If they are at a facility ISP, they will have access to facility data only. If they are at headquarters, they can see data for all facilities. On this screen, the user enters his/her user ID and password, and the system verifies the ID and password. If the ID and password match the records in the system database, the user is logged into the system until they timeout (defined in setup) or logout of the system.

The user enters the user ID and presses tab to move the cursor to next field. The user enters the password and clicks OK, then return. The system checks entries against the database. If there is a match, the system goes to a welcome screen 92 (See Figure 6). If the user's credentials are incorrect, the system shows a message, e.g. "Your ID or password is incorrect. Please try again." On the fourth try, the system shows another message, e.g. "Your ID or password is incorrect. See your system administrator for help."

The welcome screen 92, illustrated on Figure 6, comes up when a user logs into the system. If the user has logged into headquarters, they can choose to see all data, data for a particular center, or data for a particular region by selecting the location data from a pick list at the top of the screen. The pick list will drop down from the top with all data in one column, then all setup groups (from the setup screen, i.e. region 1, region 2, region 3, custom 1, by center), the "Center" option brings up a list on the right with all centers as options. Default will be all data.

If a user has logged into a facility, the facility title will appear as a second heading and no location pick list will appear.

From the welcome screen 92, the user can click any of the section tabs, "Search," "Administration," "Reports," "Monitor," "Dispatch," "Help,", or "Logout," to go to any section.

### Administration

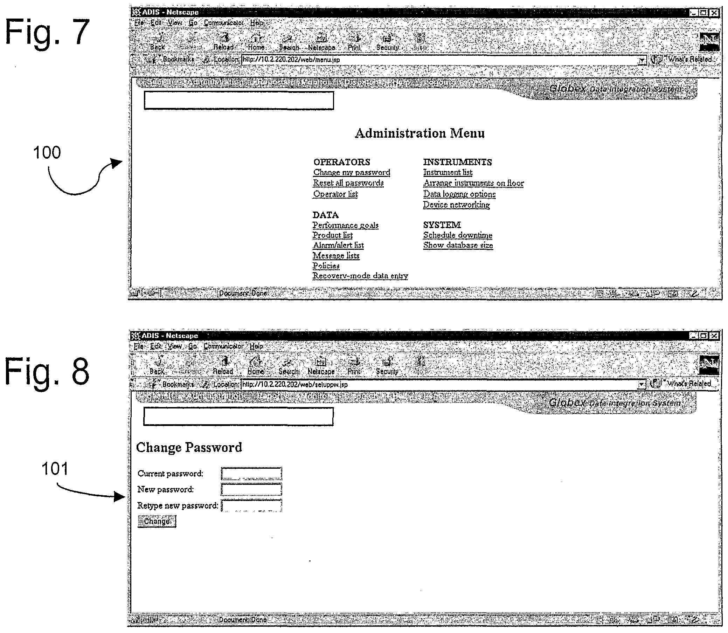

First, referring to Figures 7-26, the system 10 comprises a setup and an administration operation. The setup and administration operations are accessed by clicking on the "Administration" tab at the top of any screen. When the "Administration" tab is clicked, an administration menu 100 appears (See Figure 7). From the administration menu 100, information regarding facility operators, instruments, data, and the overall system can be input or edited. The information entered during setup is used during operation of the system 10 to verify operations, monitor procedures, monitor inventory, create reports, schedule maintenance, and the like. The administration menu 100 includes links to screens or pages where the information is inputted or edited.

The administration menu 100 includes a link to a change password screen 101. As shown in Figure 8, the change password screen allows the user to change his/her password by simply typing their current password, their new password, and repeating their new password for verification.

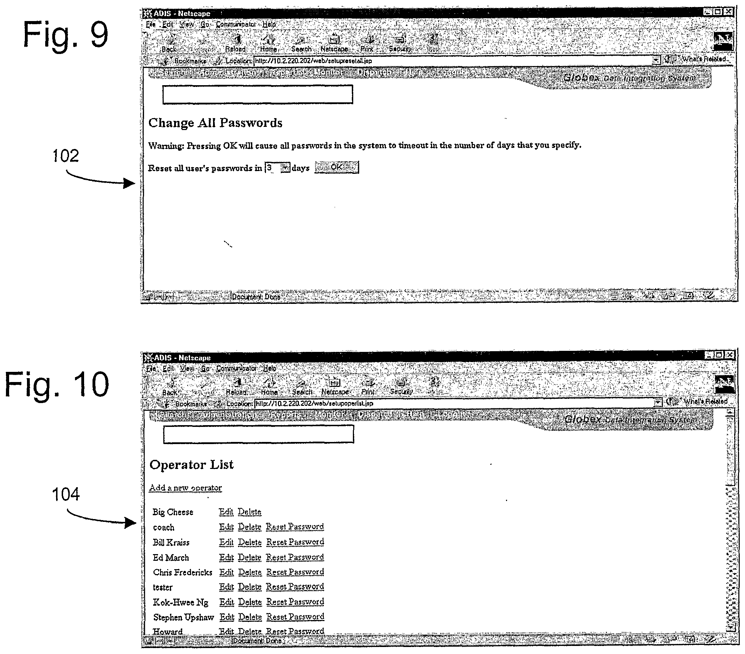

The administration menu 100 also includes a link to a reset passwords screen 102, shown in Figure 9. To reset all user passwords, a timing for reset is entered on the screen, and the user passwords will reset accordingly.

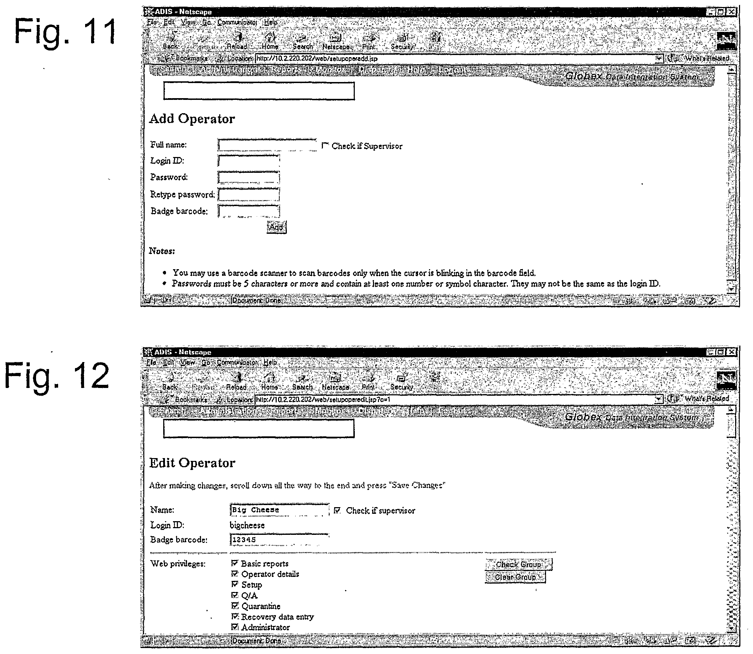

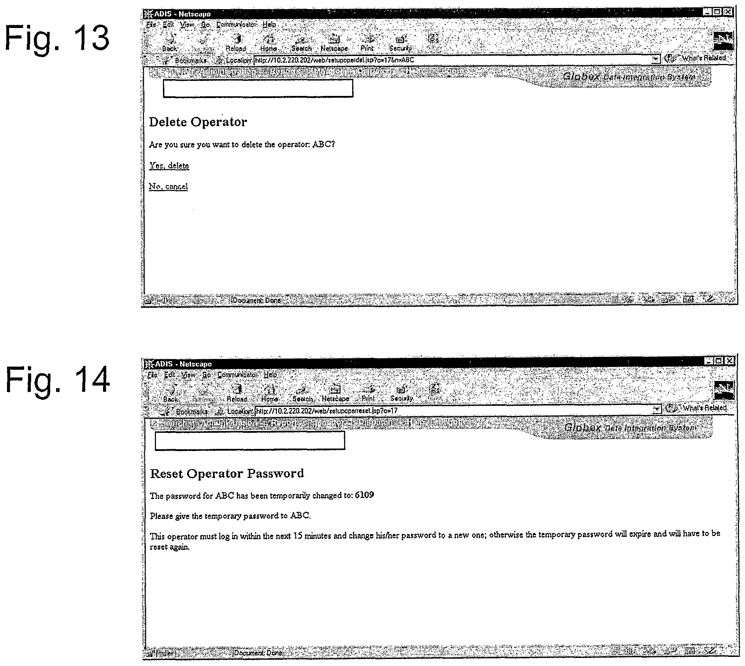

Another link on the administration menu 100 takes the user to an operator list page 104. Referring to Figure 10, the operator list page 104 comprises a list of the operators who work within the facility and links to other pages for adding new operators (see Figure 11), editing operator information (see Figure 12), deleting an operator (see Figure 13), and resetting an operator's password (see Figure 14).

Information regarding the operators qualifications to perform certain tasks or procedures or to use certain instruments is inputted by accessing the individual operator information page (Figure 12) from the operator list page 104. To assign privileges to an operator for all the options within a category (e.g. web privileges, mobile privileges), the user clicks on "Check Group." The "Clear Group" button clears or deselects all the options within that category.

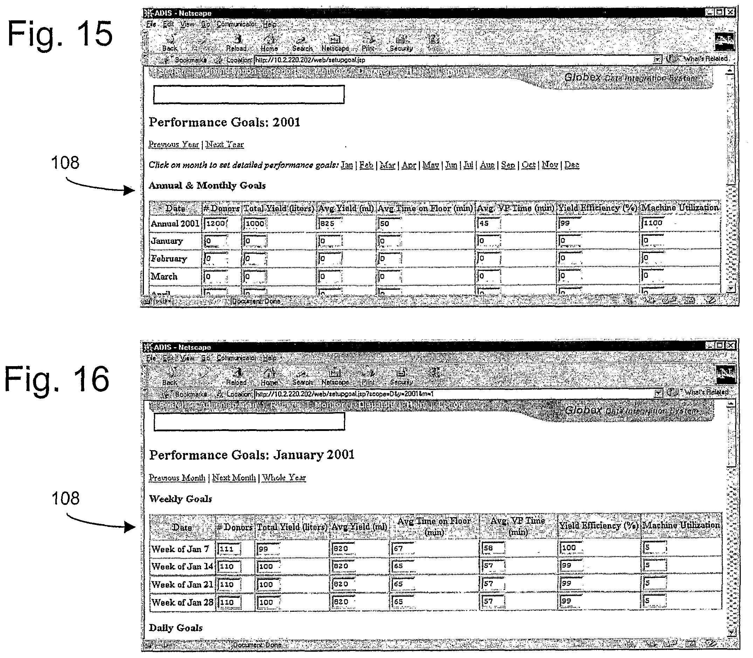

Clicking on "Performance goals" on the administration menu 100 takes the user to a performance goals page 108. Referring to Figures 15 and 16, the performance goals page 108 comprises fields where goals for a specific time period of the facility's specific operations can be inputted. In the example illustrated, the time periods are for annual and monthly targets. The base information that is inputted into these fields can be compared against actual data generated as facility functions in its normal day-to-day operation. The performance goals page 108 further comprises links to pages that comprise the performance goals from the previous year and for the next year.

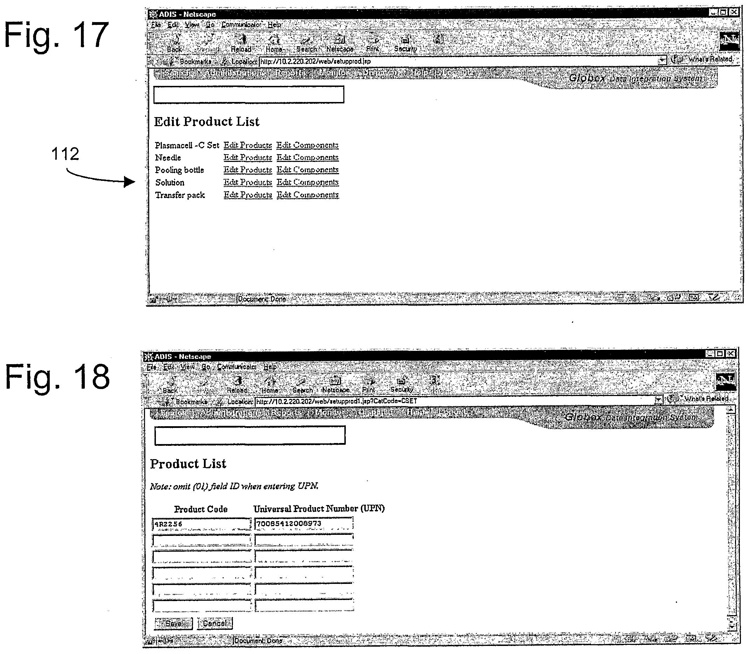

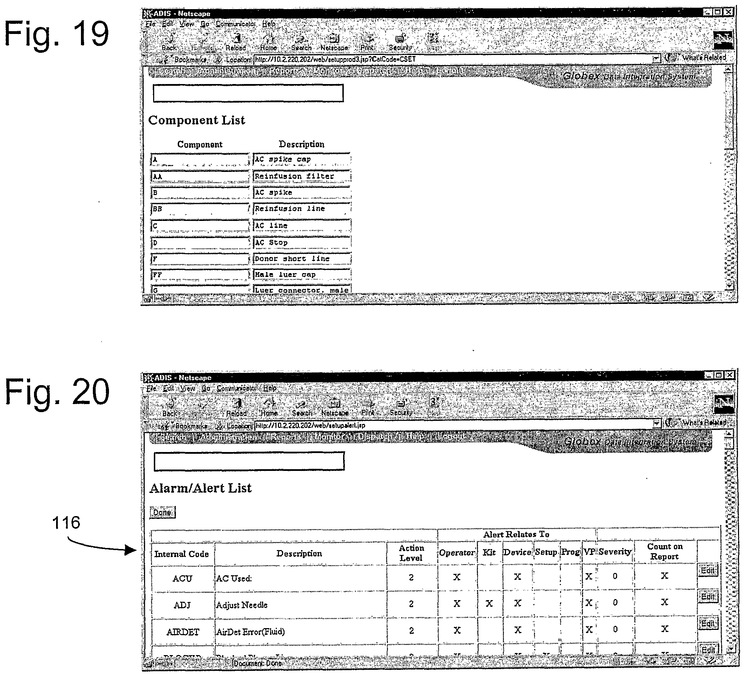

Another link on the administration menu 100 takes the user to a product list page 112. The product list page 112 is illustrated in Figure 17 and comprises a list of the products/soft goods, disposable and otherwise, used during the facility's operations as well as links to pages for editing the product list (Figure 18) and the product components associated with each product (Figure 19).

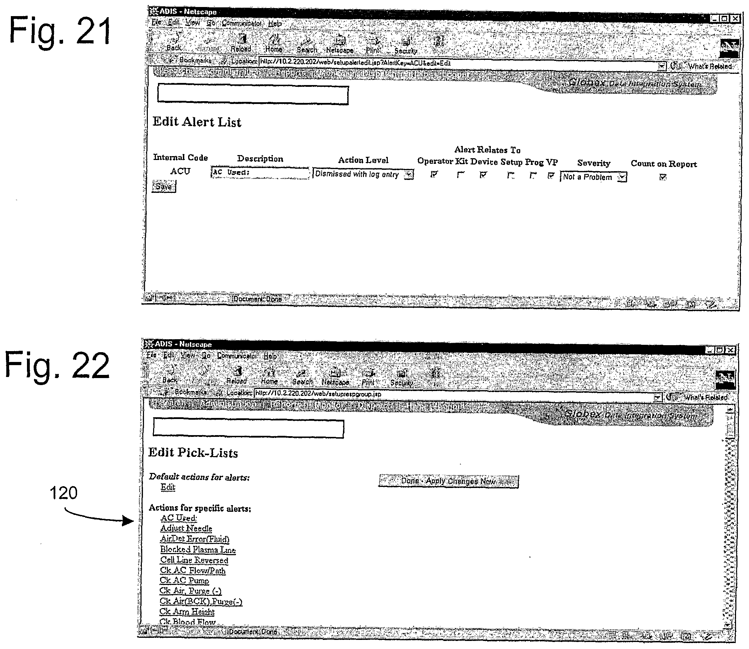

The administration menu 100 further comprises a link to an alarm/alert list page 116. As shown in Figure 20, the alarm/alert list page 116 includes information regarding alerts, alarms, or errors associated with the system's and facility's operations and further includes links to pages for editing alarm/alert list information (see Figure 21). This page can be used to associate an alert/alarm with a level of severity and a particular cause, actions to be taken, and whether the alert should be reported. Referring to Figure 21, the "Action Level" field has 3 options. The "Severity" field determines the color in which the alarm/alert is displayed on screen. For example, the color can be gray, yellow, or red depending upon low to high severity.

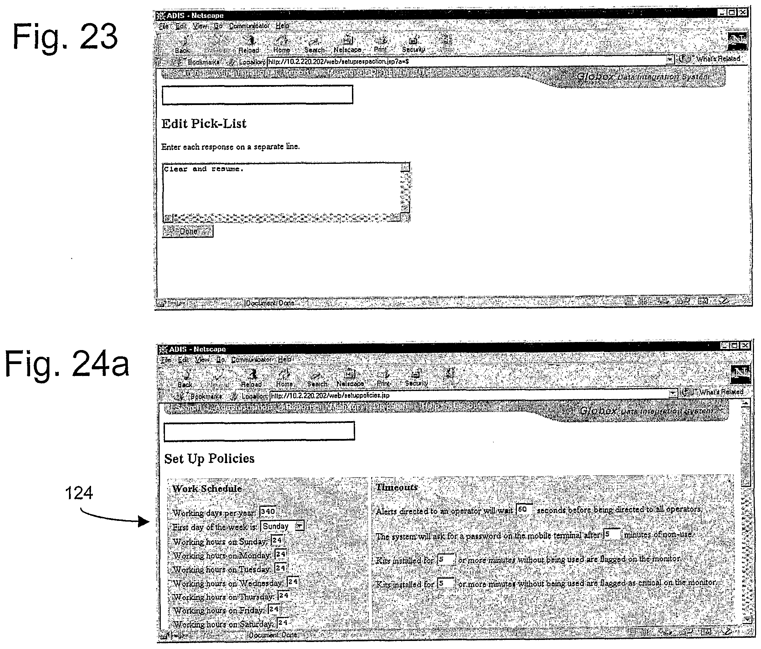

Another link on the administration menu 100 takes the user to a message list page 120. Referring to Figure 22, the message list page 120 comprises a list of the alerts/alarms. The actions taken in response to the alerts/alarms can be edited through links for each alert/alarm listed (see Figure 23). Alternatively, default actions can be edited by via a default edit link. These messages can be displayed on the system 10 when an alert/alarm is encountered in the system 10 and used in a pick list format.

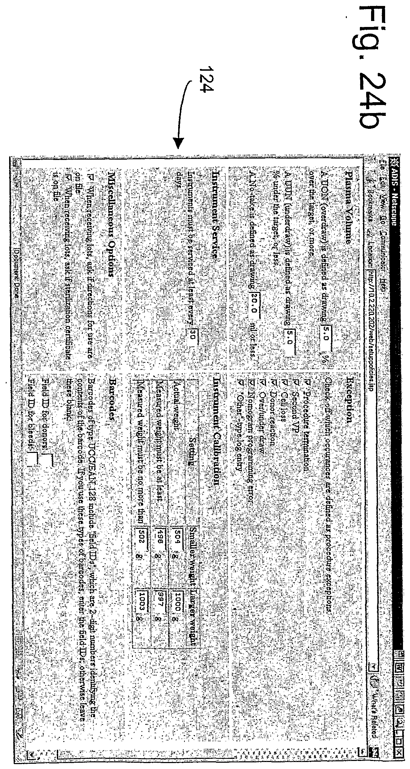

The administration menu 100 is also used to access policy page 124. As illustrated in Figures 24a and 24b, the policy page 124 allows the system 10 to be configured to expect a certain operations or procedural occurrences within the facility. For instance, the work schedule for the facility, production targets, exceptions, instrument timeouts, instrument service schedule, instrument calibration, types of barcodes expected, and other miscellaneous options can be inputted and stored. The system 10 eventually uses the data in setting system parameters or in a comparison to actual data collected during facility procedures.

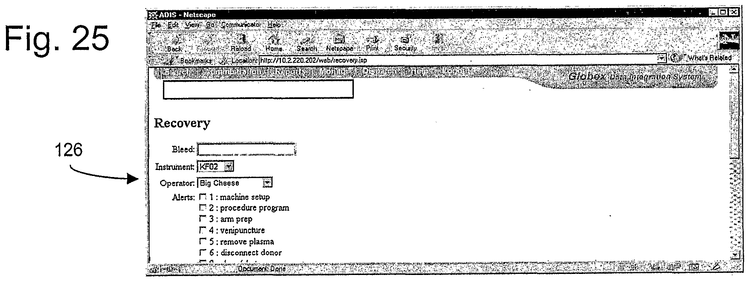

The administration menu also comprises a link to a recovery screen 126. The recovery screen 126 is illustrated in Figure 25. This screen is used to create bleed records or enter edits to existing bleed records with specific procedure information that was collected manually. Also, this function is used if the system 10 crashes and the facility procedure (e.g., an apheresis procedure) is continued.

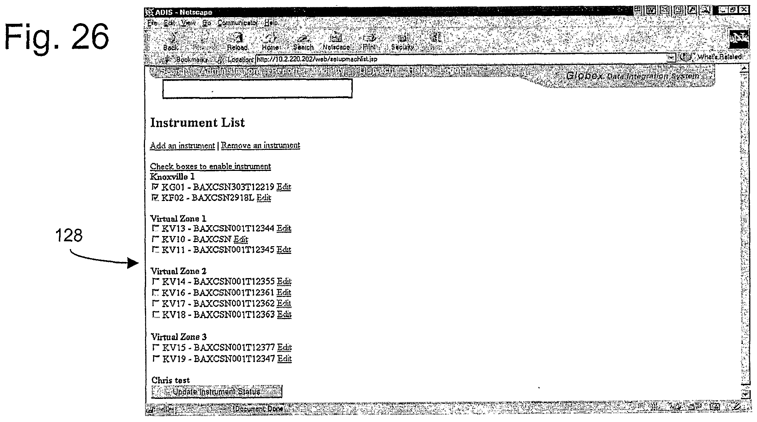

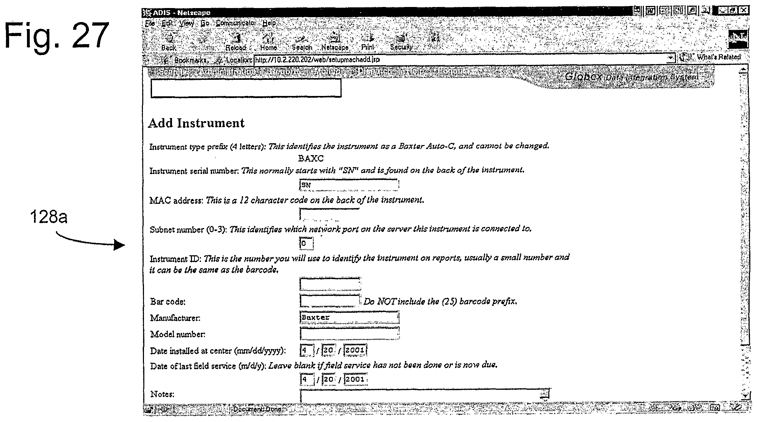

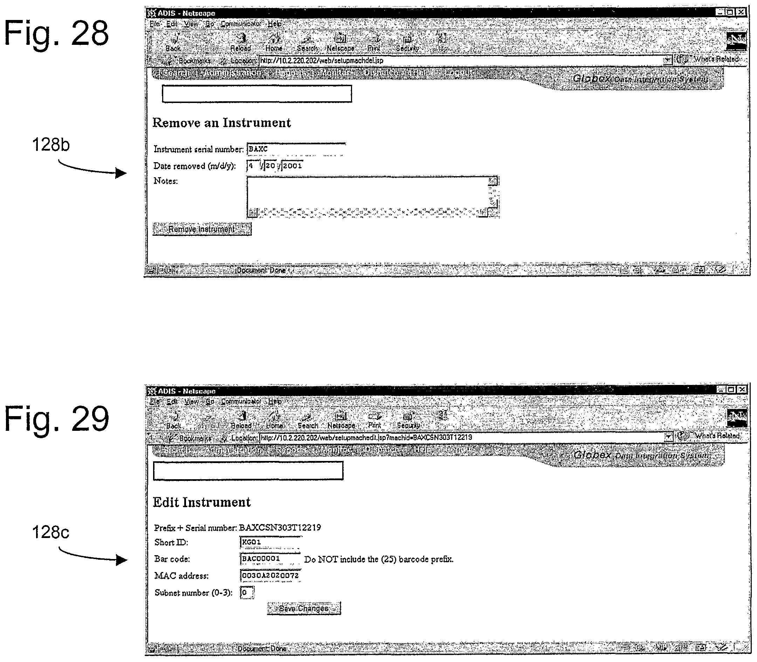

The administration menu 100 further comprises a link to an instrument list page 128. The instrument list page 128 is illustrated in Figure 26. It provides a list of instruments within the facility, an input portion to enable the instruments on the list, and links to pages 128a (Figure 27), 128b (Figure 28), and 128c (Figure 29) where instruments can be added, removed, and edited, respectively.

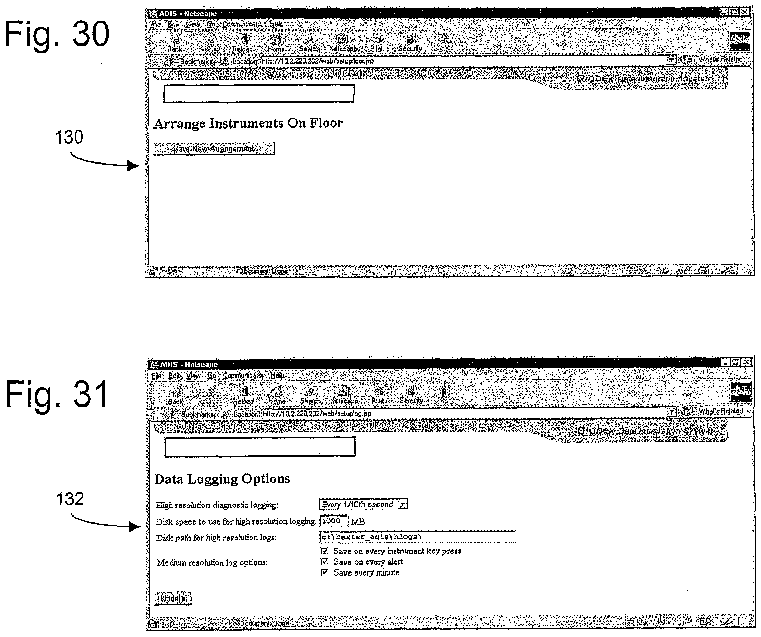

Another link on the administration menu 100 takes the user to facility configuration page 130. This page, shown in Figure 30, is used to assign the instruments from the instrument list to a zone/bay/area within the facility.

Yet another link on the administration menu 100 takes the user to a data logging options page 132. Referring to Figure 31, this page is used to tell the system 10 how often to log data. For instance, the data logging page may comprise fields for high resolution diagnostic data logging interval, disk space reserve for high resolution logging, and the disk path for the high resolution logs. The data logging page may further comprise fields associated with medium resolution data logging options, such as when to save the medium resolution log data, e.g. on every key press of an instrument, on every alert, or after a specific duration of time.

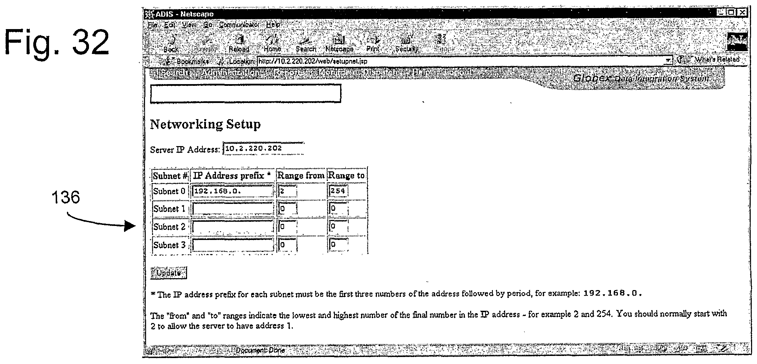

The administration menu 100 also comprises a link to a network setup page 136. As shown in Figure 32, the network setup page 136 is used assign the IP address to the system server 34 and any subnetworks.

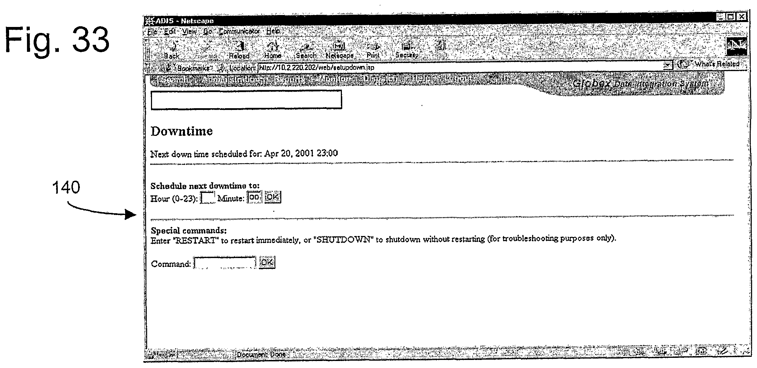

The administration menu 100 further comprises a link to a downtime page 140. As illustrated in Figure 33, the downtime page 140 is used to input the timing of a scheduled shutdown of the system 10. The shutdown can be for a variety of reasons, including maintenance, troubleshooting, software updating, and the like. A command may also be entered on this page to tell the system 10 to remain in the shutdown mode or to restart immediately.

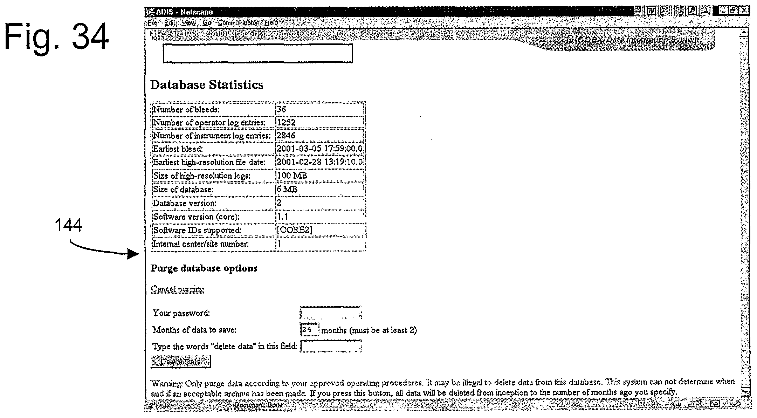

Finally, the administration menu 100 comprises a link to a statistic page 144. The statistic page 144, shown in Figure 34, includes a summary of the procedures performed within the facility. The statistic page 144 further includes fields for inputting the length of time to save data and whether the data should be deleted. For the system to accept information from these fields, the user must verify the information by inputting his/her valid password.

### Database Searching

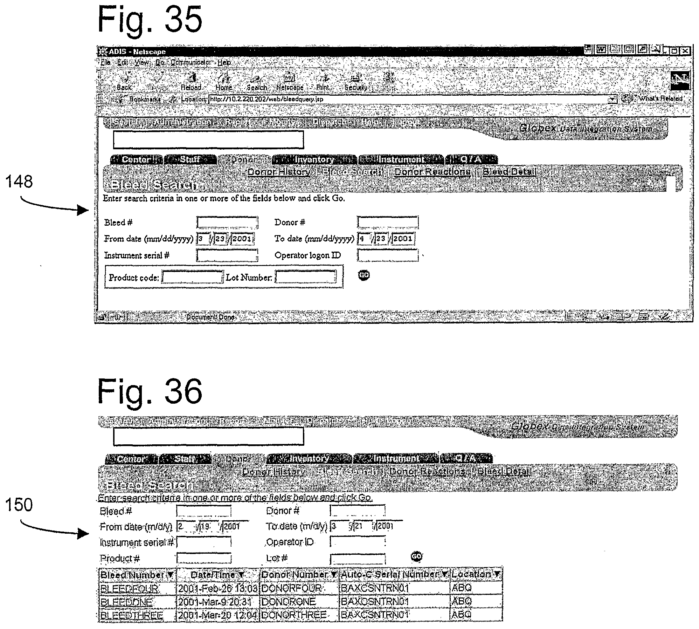

Next, referring to Figures 35 and 36, the system 10 comprises a search function. The search function is accessed by clicking on the "Search" tab at the top of any screen. When the "Search" tab is clicked, a search page 148 appears. Referring to Figure 35, the search screen 148 allows the user to find information pertaining to any bleed that is stored in the system database. The user can enter search criteria for as many fields as needed, click the "GO" button, and view the resulting records at the bottom of the screen. The search results can be sorted according to column headings by clicking the corresponding arrow.

The search function allows the user to search for: a specific bleed number, a group of bleed numbers within a specific date range, all bleeds that occurred on a specific instrument (within a specific date range), all bleed numbers from one donor (within a specific date range), all bleeds that were performed by a specific operator (within a specific date range), and all bleeds that were performed with specific components or components from a specific lot (within a specific date range).

To perform a search, the user enters any search criteria necessary and clicks the "GO" button. A Bleed List Report 150 (see Figure 36) sorted by bleed number is displayed. To change the sort order, the user may click on the arrow for that column heading. All bleed numbers are links to bleed detail reports (described below).

### Reporting

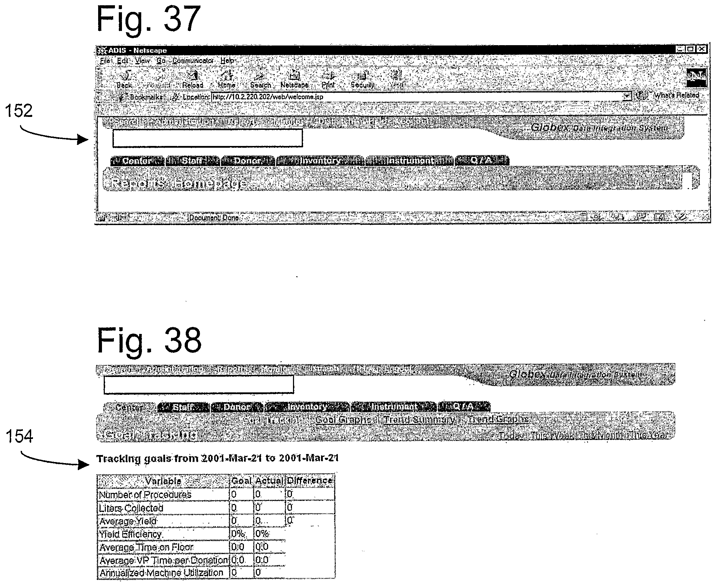

Figures 37-76 illustrate the system's reporting function. The reporting function is accessed by clicking on the "Reports" tab at the top of any screen. When the "Reports" tab is clicked, a reports homepage 152 appears. Referring to Figure 37, the reports homepage 152 includes tabs for each of the report types available in the system. The report types include center, staff, donor, inventory, instrument, and Q/A (quality assurance).

The reports are easily navigated. A user clicks on "Reports" from the options menu to display the report menu screen with selection tabs. From there, the user can select a report section tab and report type. The user clicks the appropriate report section tab for the report he/she wishes to view. The default report for that section displays. To select a different report in that section, the user clicks the appropriate report type from the choices below the report section tabs. The name of the report being displayed is highlighted in white.

If desired, the user can select a new time frame to generate a new report. Most reports can be displayed for one of several time frames: today (this is the default time frame); week to date; month to date; year to date; or custom. There are two methods of selecting a time frame, by clicking the desired time frame to generate a new report or by entering specific start and end dates and clicking the "GO" button.

The information in some reports can be sorted to display the data by a specific column in descending order. If there is a down arrow in a column heading, then the report is sortable by that column variable. Generally reports are displayed sorted by the variable in the left-hand column. To re-sort a report, the user clicks the arrow in the heading of the column that he/she wishes to sort by.

Many reports contain links to other reports. A link is any operator, bleed number, donor number, or other variable that is displayed underlined and in blue. When the user clicks a link within a report, a new report is displayed. Generally, links provide great detail on a specific item within a report.

Now, referring to Figures 38-41, the reports in the center section display statistics for all facility performance variables. For each variable, the reports indicate the actual value, the goal, and the difference between the actual value and the goal. The reports can be displayed as goal summaries and graphs, and as trend summaries and graphs.

The goal tracking report 154 (see Figure 38) is used to compare target volumes collected with actual values collected for specific items on a day-to-date, week-to-date, month-to-date, or year-to-date basis. If a value is displayed in the "Difference" column, the value indicates that the goal for the selected item is larger or smaller than the actual count.

The "Number of Procedures" field shows only the goals that have been entered in the system through the administration setup function. The "Yield Efficiency" field indicates the actual yield per target volume. The "Actualized Machine Utilization" field indicates procedures per machine, extrapolated to one year.

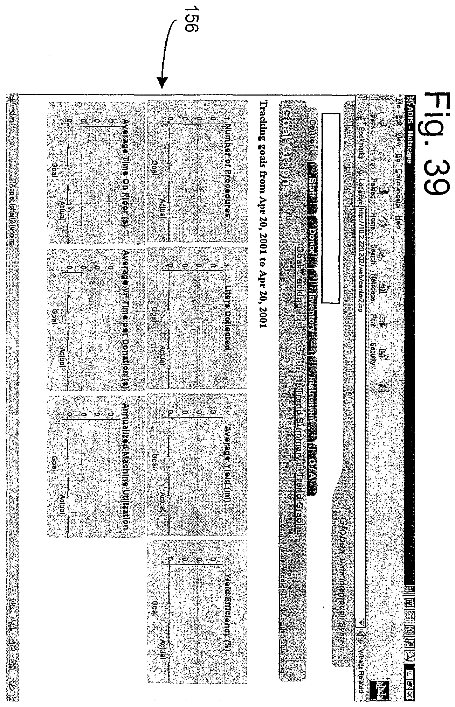

The goal tracking graph 156, illustrated in Figure 39, is a graphic representation of the actual efficiency items compared to their goals on a day-to-date, week-to-date, month-to-date, or year-to-date basis. The default time frame for this report is week-to-date. The user can click on a graph to enlarge the report and click again to revert to summary format.

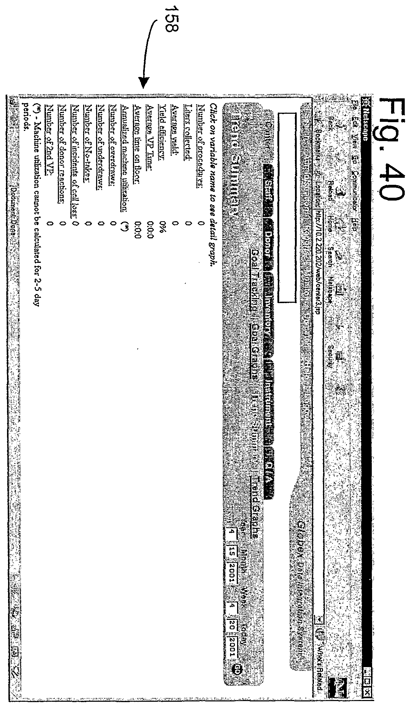

Referring to Figure 40, the trend summary report 158 displays relative values for any entered data to help identify predictable trends. The default report range is week-to-date. The user may click on any variable to view a graphic image of the corresponding data on the right side of the screen.

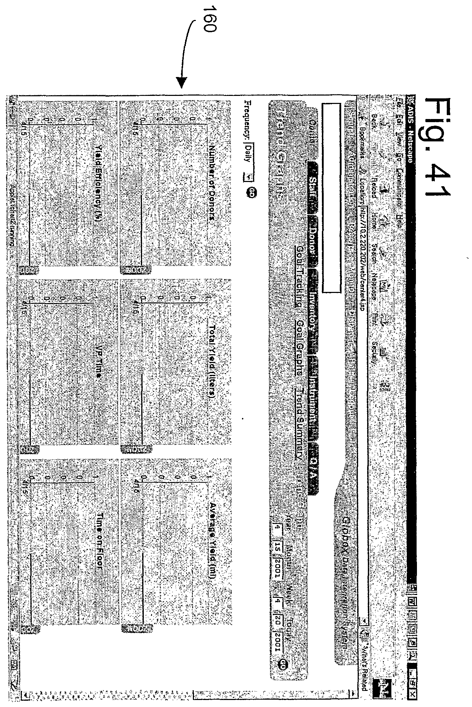

Figure 41 illustrates the trend graphs page 160. This report displays key indicators for any date range by week, month, day and year. The user may click on any graph to zoom in and click again to revert to summary format. The scroll bar allows the user to view additional graphs that do not fit on a single screen. screen.

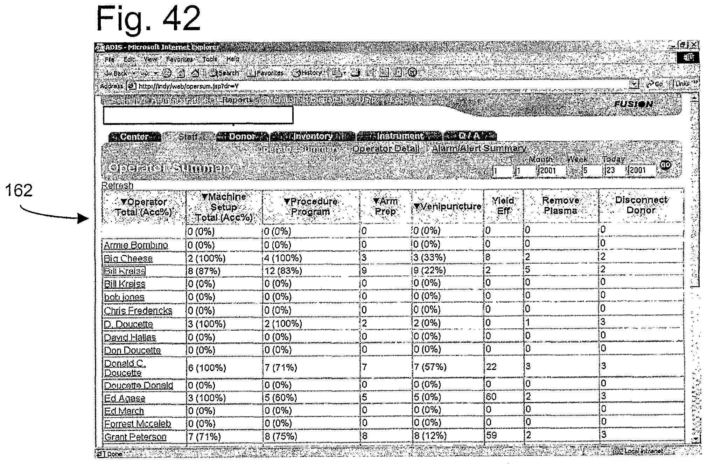

Referring to Figures 42-47, the staff reports are illustrated. The summary report 162, Figure 42, is only available to facility administrators. This report provides a summary of the relative rates of accuracy for the operators. The user may click on the arrows next to the column headings to change the sort order.

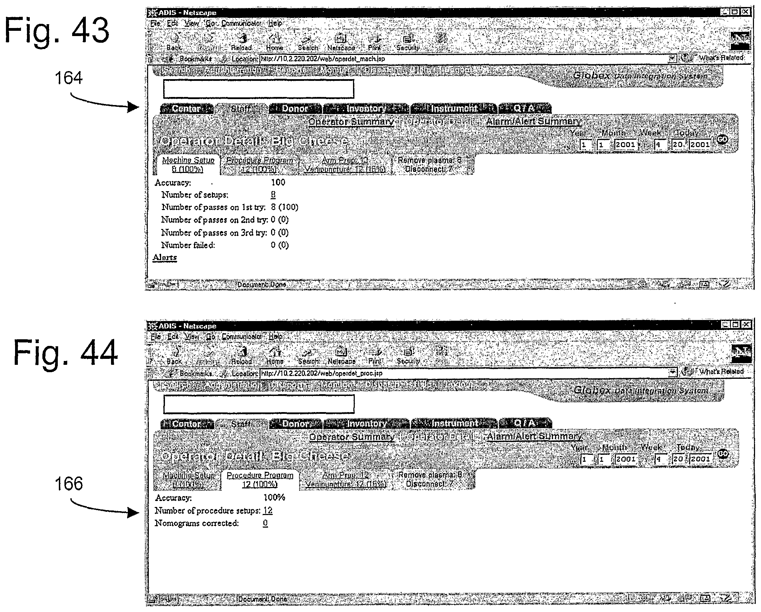

Referring to Figures 43-45, the operator detail report is a detailed version of the data found in the summary report. It provides in depth operator details with an additional set of links. Operator detail reports can be viewed for machine setup, procedure program, and arm prep venipuncture.

Figure 43 illustrates the machine setup report 164. This report displays the operator detail for machine setup for a specified period. The user can click on the alerts link at the bottom of this screen to display details about the alerts. The accuracy field indicates the accuracy percentage on the operator's first try. The number of setups field indicates the number of machine setup events logged by the operator. The number of passes on 1^{st} try field indicates the number of machine setups that had no associated alarms/alerts. This number is used to calculate the accuracy percentage. The number of passes on 2^{nd} try field indicates the number of machine setups that had just one associated alarm/alert. The number of passes on 3^{rd} try field indicates the number of machine setups that had exactly two associated alarms/alerts. The number failed field indicates the total number of machine setups less the setups that had exactly two associated alarms/alerts.

The procedure program report 166 is illustrated in Figure 44. This report displays the operator detail for procedure program for a specified period. The accuracy field is displayed as a percentage based on the frequency of setups that resulted in alarms/alerts. The number of procedure setups field indicates the number of machine setup events logged by the operator. The plasma nomogram checks field indicates the number of times that there was a mismatch between expected and programmed plasma volume. The percentage field is derived from the total counts per completed setup. The bleed number list field displays the bleed numbers that were included in the count of nomogram checks.

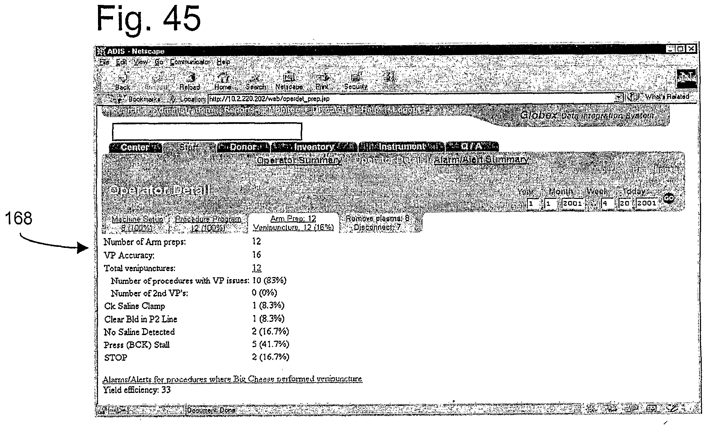

The arm prep/venipuncture report 168 is shown in Figure 45. This report provides an overview of the arm preparation and venipuncture for each operator. The user clicks on the alerts link at the bottom of this screen to display details about the alerts. The VP frequency field indicates the frequency of venipuncture alarms/alerts associated with the operator. The total venipunctures field indicates the number of venipunctures logged by the operator.

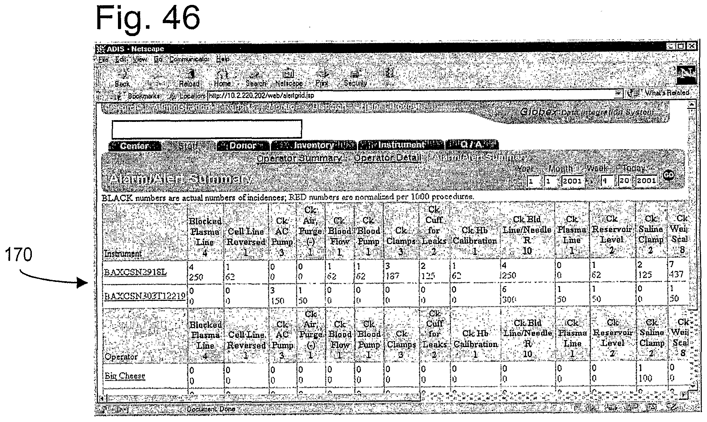

The operator alarm/alert reports are illustrated in Figures 46-48. This report provides an overview of the alarm/alert details and can be viewed for operator summary, operator detail, and alarm/alert summary. The reports can be viewed in normalized or raw data mode by clicking on the toggle switch on this screen. The values in the report that appear in black color indicate actual raw data and the numbers that appear in red are the normalized data per 1000 procedures. The normalized data is used as a form of comparison to determine the averages.

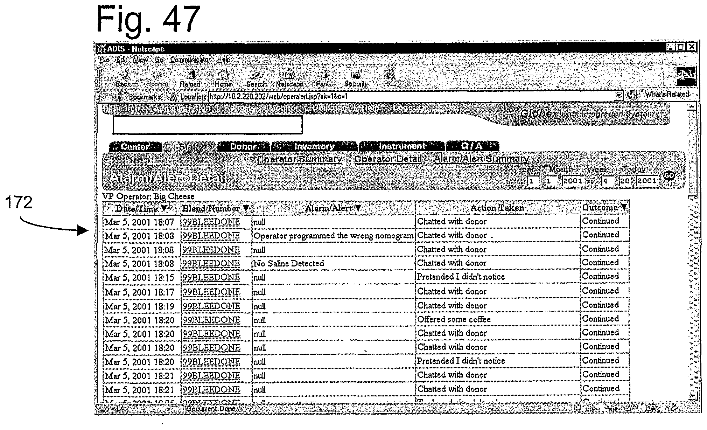

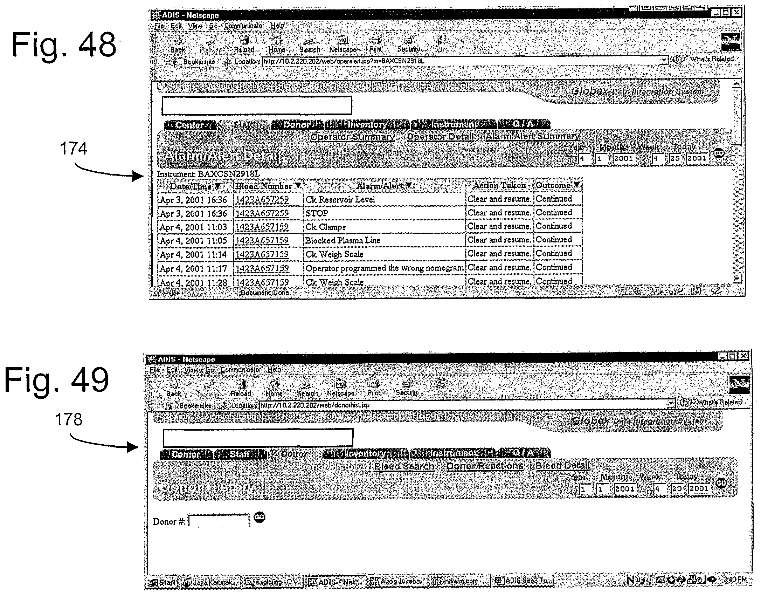

Referring to Figure 46, the alarm/alert summary report 170 is illustrated. This report displays all of the alarms/alerts across the screen. All of the instruments where the alarm/alert occurred are listed down the screen. All of the operators associated with the alarms/alerts are also listed down the screen. The instrument numbers and operator IDs listed in this report link to the alarm/alert detail report (Figures 47 and 48) that lists the associated bleed numbers.

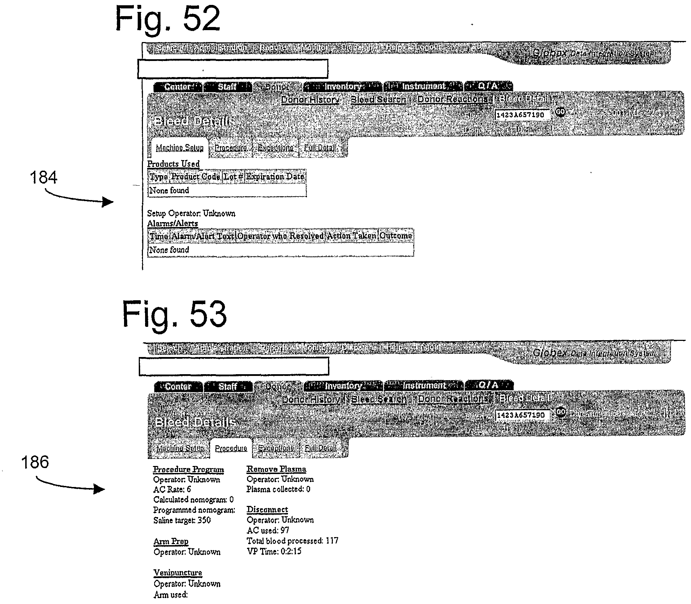

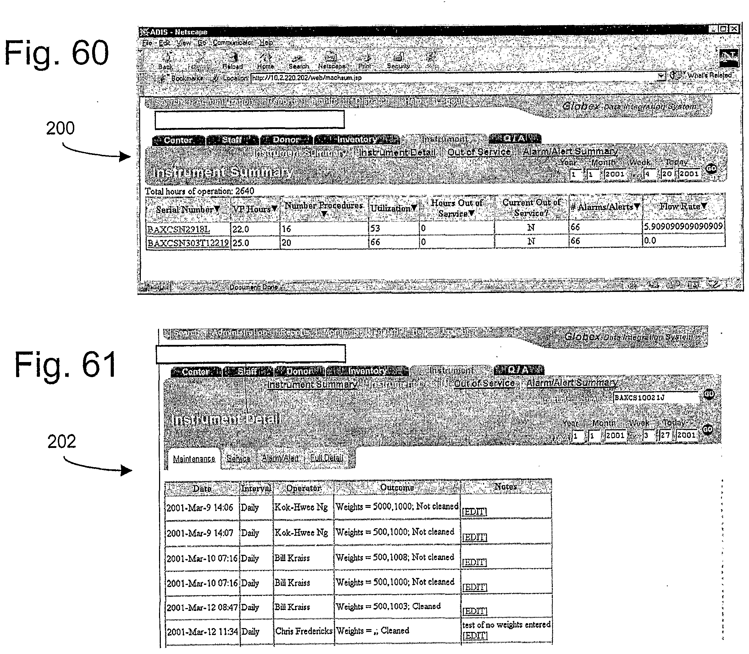

The alarm/alert detail report 172, 174 is shown in Figures 47 and 48. This report provides details of the alarm/alert by instrument or by operator depending upon the selection made in the alarm/alert summary report 170 (Figure 46). The user may also access this screen by clicking the links from the operator summary report 162 (Figure 42) or the instrument summary report (Figure 60). This report is typically used by supervisors to identify the areas in which operators may need training. The bleed numbers listed on this report may be used to link to the bleed detail report (Figures 52-55) .

The donor reports are illustrated in Figures 49-55. All reports related to donors and bleeds are available in the donor reports. Reports can be generated about specific donor histories and donor reactions, specific bleeds (summary and details), and bleed information over a specific time frame or a range of bleeds. The user enters a valid donor number at the "Donor #" field to begin generating these reports.

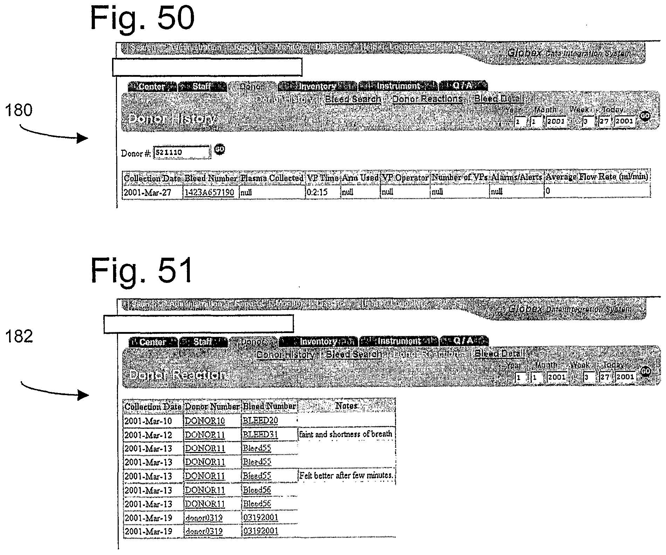

Referring to Figure 50, the donor history report 180 is illustrated. This report provides a summary listing of all bleeds for a single donor in chronological order. To view full details about a bleed, the user clicks on the bleed number. Also, if any alarms/alerts are displayed for the bleed, the user may click on the alarm/alert link to display a list of bleed numbers that had that an alarm/alert. This report also has a link to the bleed details report (Figures 52-55).

Next, Figure 51 shows the donor reaction report 182. This report provides details about all donor reactions that occurred during a specific time frame in chronological order. To view donor histories, the user clicks on the donor number. To access bleed details, the user clicks on the bleed number.

In Figures 52-55, the various bleed detail reports are shown. The bleed detail reports include machine setup reports, procedure reports, exception reports, and full detail reports.

The machine setup report 184 is illustrated in Figure 52. This report is a summary of donor and bleed details for machine setup. This report lists the products used during the bleed and displays any alerts/alarms that occurred during the setup. The user may click on the operator ID to view the operator details report (Figures 44-48).

Referring to Figure 53, the procedure report 186 is a summary of donor and bleed details for the procedure setup obtained from the logging records.

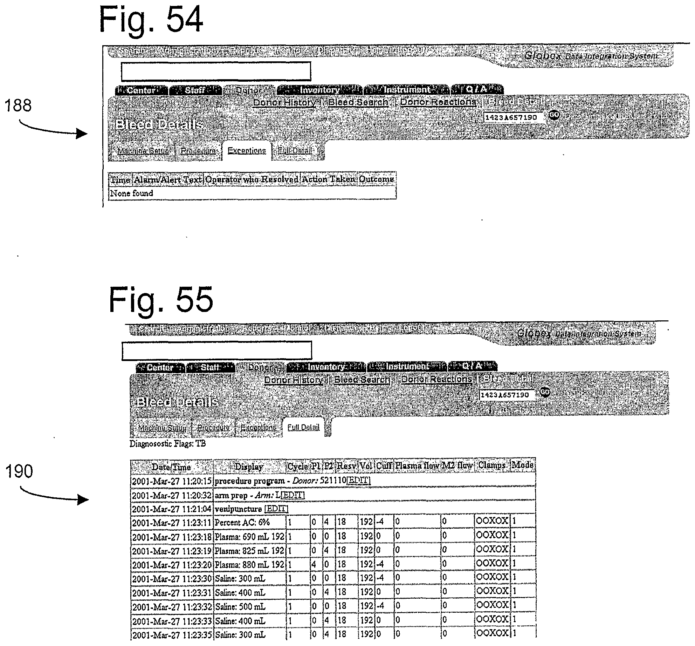

Figure 54 is an illustration of the exceptions report 188. This report displays all of the exception resolutions logged during the bleed, except for those displayed under the machine setup report.

Figure 55 shows the full detail report 190. This report provides a medium resolution log of instrument variables in chronological order. It includes a log of operator-initiated events. The report shows the date, time, and a description of the event that was logged.

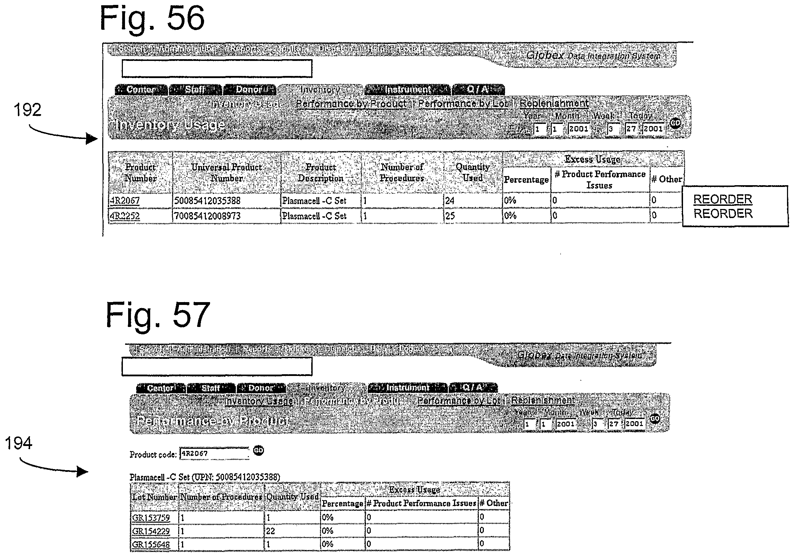

The inventory reports are illustrated in Figures 56-59. The inventory reports allow the user to track information related to products used in the center and search and sort by product and lot number. The inventory reports include the following reports: inventory usage, performance by product, performance by lot, replenishment.

The inventory usage report 192 is illustrated in Figure 56. This is a summary report that lists the product numbers used in any bleed for a specified period. The user may click on the product number to see details for that product. The default time frame for this report is month-to-date. When this report is generated from a regional or higher level (central server), the report includes an additional button for disposable reordering.

The performance by product report 194 is shown in Figure 57. This is a summary report listing all of the lot numbers found for the selected product and used within the specified time frame.

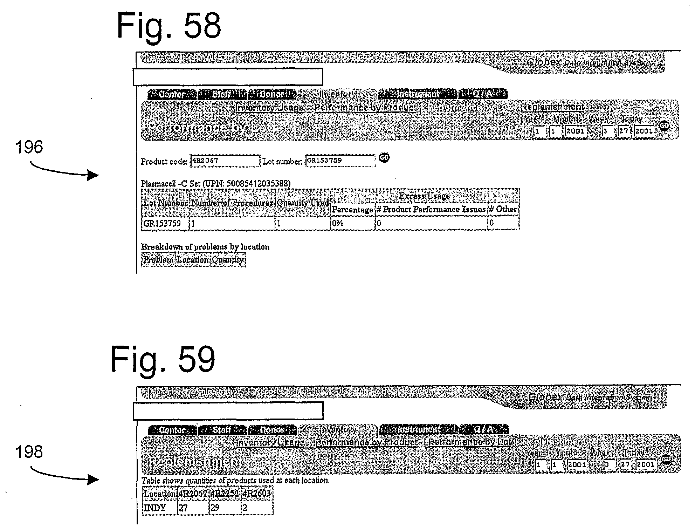

Referring to Figure 58, the performance by lot report 196 is illustrated. This is a detailed report that provides information of disposables by lot number.

Figure 59 shows the replenishment report 198. This report provides an overview of products used across locations within the currently selected region. The default time frame for this report is month-to-date.

The instrument reports are illustrated in Figures 60-66. These reports provide summary and detailed statistics for the instruments. This information includes all procedures performed on a machine, maintenance and servicing records, and the alarm/alert summary report.

Referring to Figure 60, the instrument summary report 200 is shown. This is a summary report with information about the run hours for each instrument. For details about the instrument, the user can click on the instrument number links.

The instrument detail reports are illustrated on Figures 61 and 63-65. These are reports sorted by instrument serial number. The instrument detail reports include the maintenance, service, alarm/alert, and full detail reports. If this report is accessed from a facility, only data from that facility is displayed. If this report is accessed from a headquarters, a complete history may be viewed.

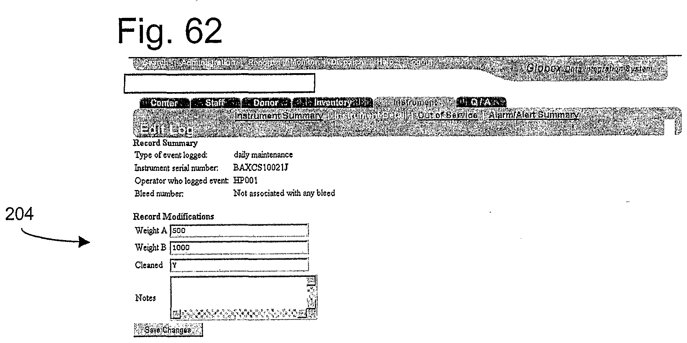

First, referring to Figure 61, the instrument maintenance report 202 is illustrated. The report lists details about the maintenance activities performed on an instrument. The records can be edited by clicking on the "EDIT" button. When the "EDIT" button is clicked, an edit log page 204 is displayed (see Figure 62). The edit log page 204 allows the user to edit maintenance records.

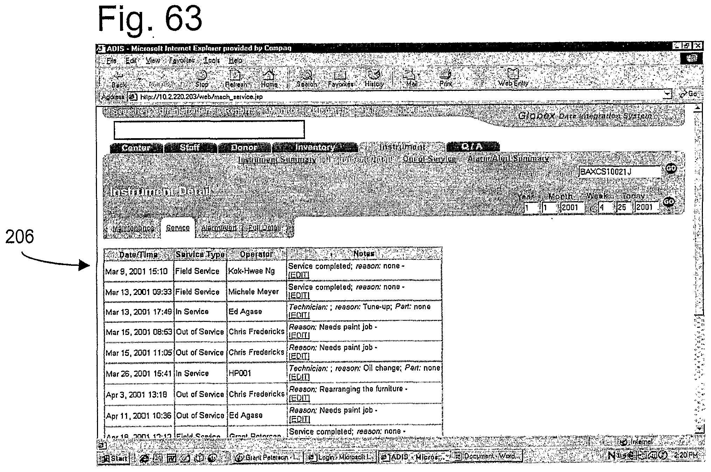

Next, Figure 63 is an illustration of the instrument service report 206. This report lists the out-of-service events logged for this instrument.

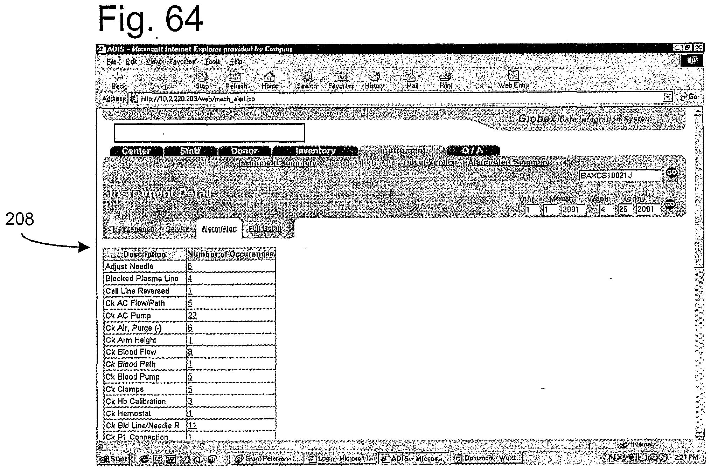

Further, Figure 64 is an illustration of the instrument alarm/alert detail report 208. The report lists alerts and alarms produced by an instrument. The user can access the bleed details (Figures 52-55) by clicking on the bleed number link.

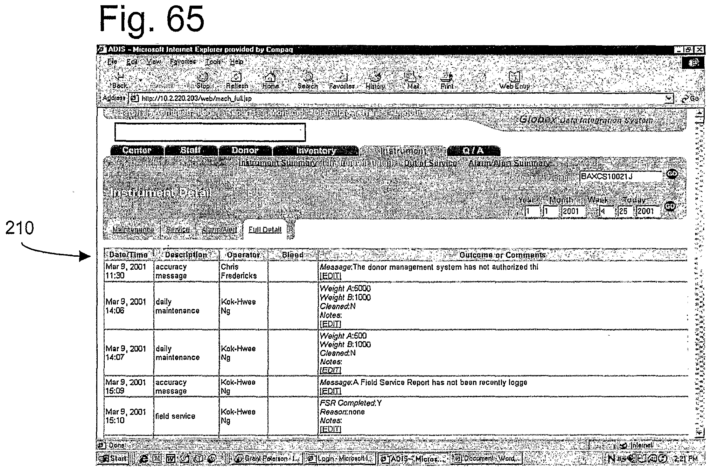

Figure 65 is an illustration of the instrument full detail report 210. This report lists details about all actions performed on an instrument.

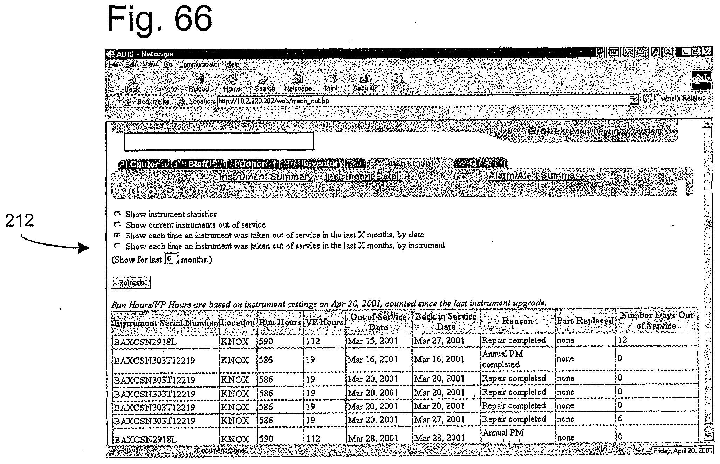

Finally, Figure 66 is an illustration of the instrument out of service report 212. This report lists the instruments that are out-of-service and the details related to these instruments. For a detailed history, the user can click on the instrument's serial number link.

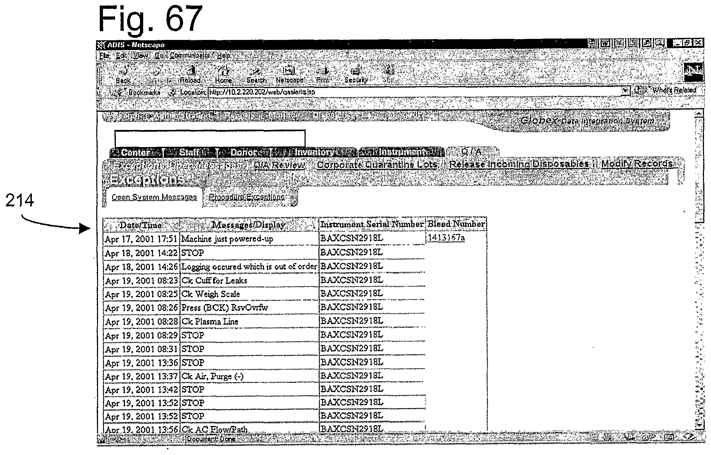

The quality assurance (Q/A) reports are illustrated in Figures 67-76. The Q/A reports comprise a variety of reports and data entry functions. The user can view lists of messages and procedure exceptions by instrument or bleed, and see information about quarantined and released product lots. In this section it is also possible to edit or modify records stored in the system database.

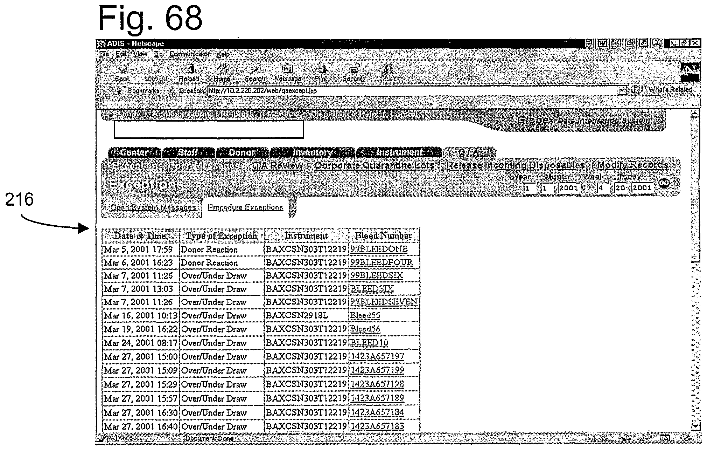

Figures 67 and 68 illustrate the Q/A exception reports. There are two types of Q/A exception reports, the open system message report and the procedure exception report.

Referring to Figure 67, the open system messages report 214 is illustrated. This report lists all of the open alarms/alerts within the system. This allows the supervisor to identify all open alerts/alarms. To clear the open alerts, the user logs the event on a mobile device (see below) or under setup. The user can link to the bleed details from this report.

Figure 68 is drawing of the procedure exception report 216. This report lists all of the bleeds that are marked as exceptions. The criteria for classifying a bleed as an exception is determined by the facility and is entered into the system through the system setup function (see above).

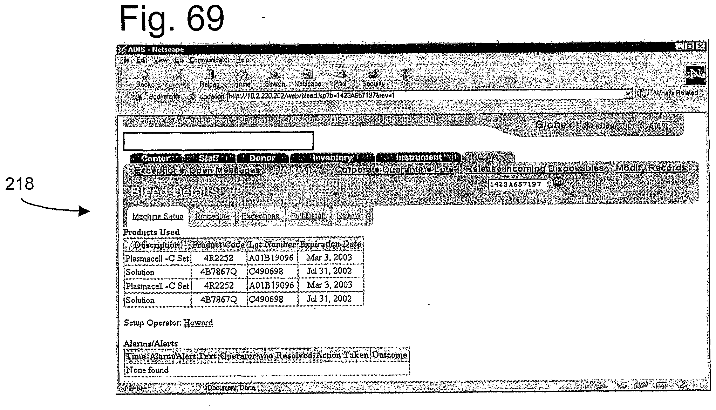

Figures 69-70 are the Q/A review reports. There are three types of Q/A review reports, the bleed number review, instrument review, and the all reviewed records report.

Figure 69 is a Q/A bleed details report 218. This report is usually used by supervisors to query records by bleed number and mark them as reviewed. The user enters a bleed number in the bleed number field to access the bleed details. To review a record, the user clicks on the "Review" tab and enters any comments, if appropriate and clicks on "Update."

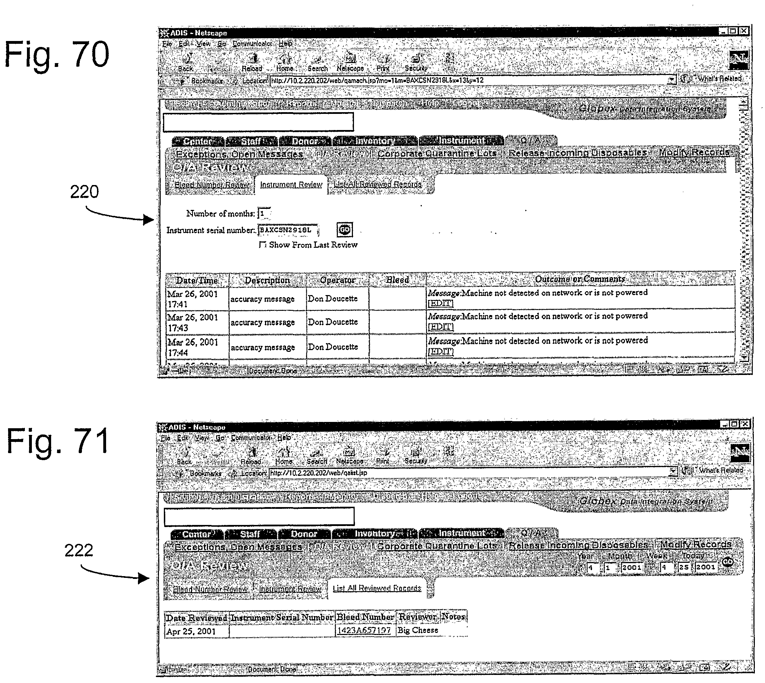

Figure 70 is the instrument review report 220. The user enters an instrument number that needs to be reviewed. This report allows supervisors to retrieve records by instrument number, mark them as reviewed, or edit them (see Figure 62).

Figure 71 is an illustration of the all reviewed records report 222. This report is usually used by supervisors to query all reviewed records. The bleed numbers on this screen link to the bleed details report. The instrument numbers on this screen link to the instrument details report.

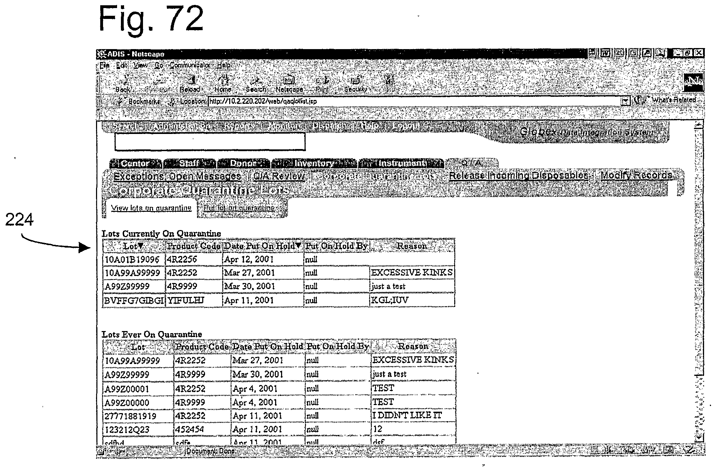

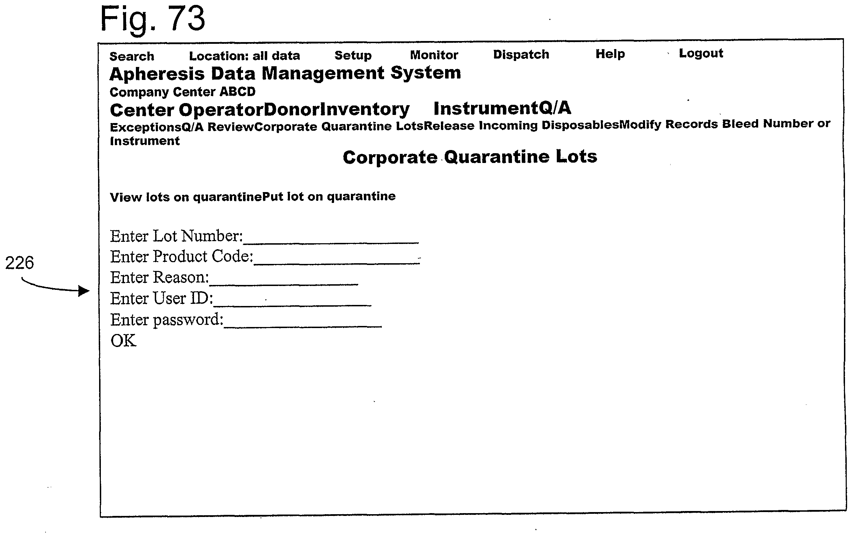

Figures 72 and 73 show the Q/A corporate quarantine lots reports. Figure 72 is a view the disposable/soft good lots on quarantine report 224. This report is usually used by supervisors to query lots on quarantine. Figure 73 is the put lot on quarantine report 226. This report can only be used from HQ and is used to place a lot on quarantine.

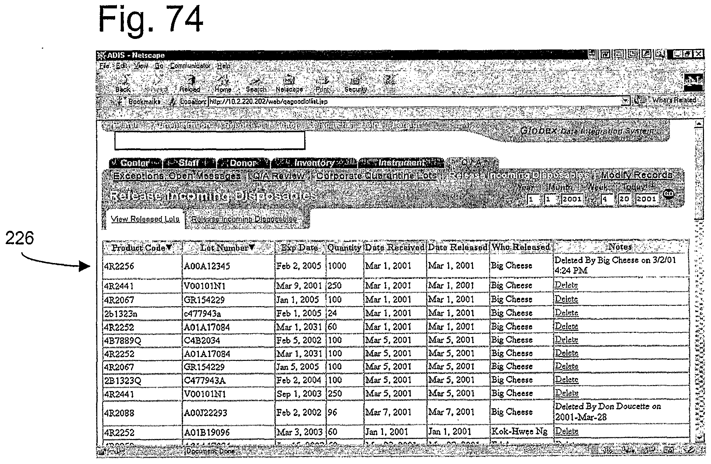

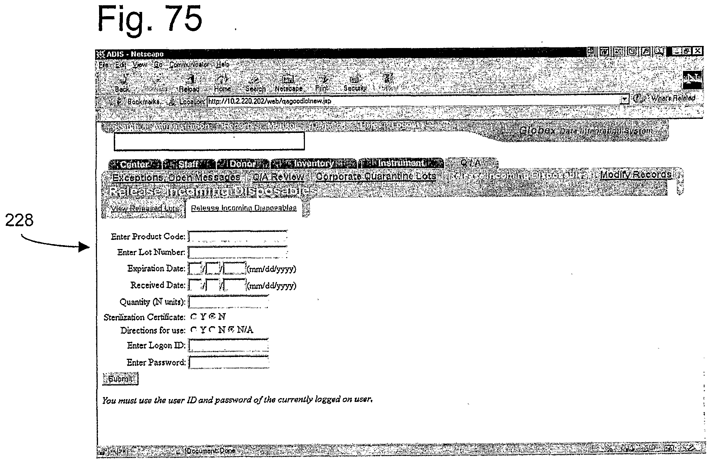

Figures 74 and 75 are the Q/A release incoming disposables reports. Figure 74 is a view released lots report 226. The supervisors use this report to view a list of products that have been released for use. Figure 75 is the release incoming disposables report 228. This report allows facility supervisors to release incoming products for use.

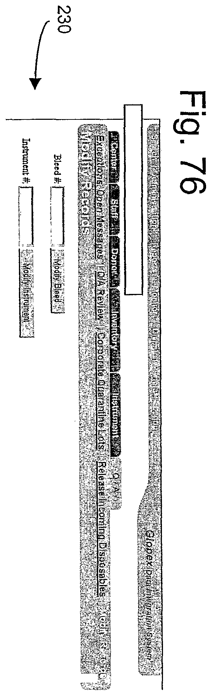

Finally, Figure 76 is an illustration of the Q/A modify records report 230. The supervisors use this report to modify log entries in a record. The user enters a bleed number or an instrument number to modify its record. The user must enter their user ID and password for the changes to take effect.

### Instrument Monitor

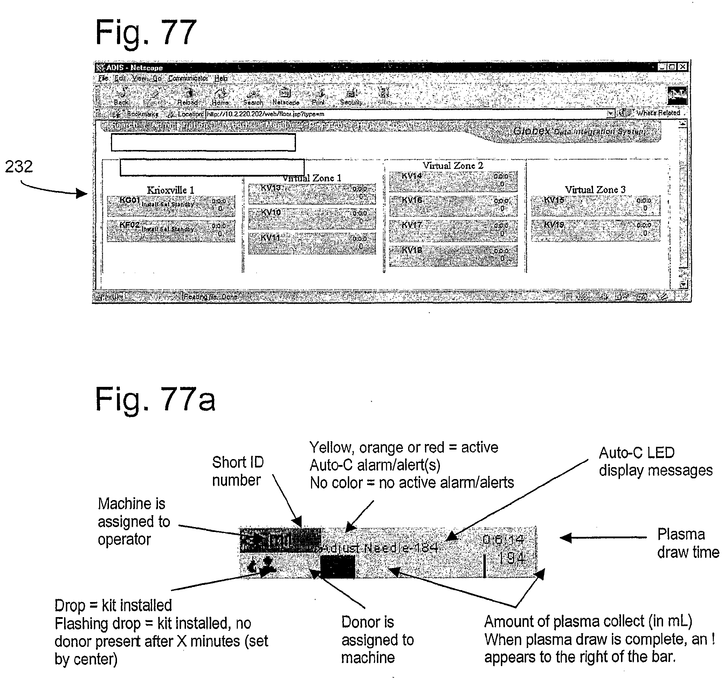

Referring to Figure 77, the monitoring page 232 is accessed by clicking on the "Monitor" tab. The monitoring screens 232 provide information on the current state of the machines in the center. The screens show all of the instruments arranged by area/bay within the facility. Each instrument is represented on screen by a bar. It may be necessary to scroll down or to the right to view all of the instruments in the facility. Figure 77a shows potential information that is available for each instrument.

### Instrument Dispatch

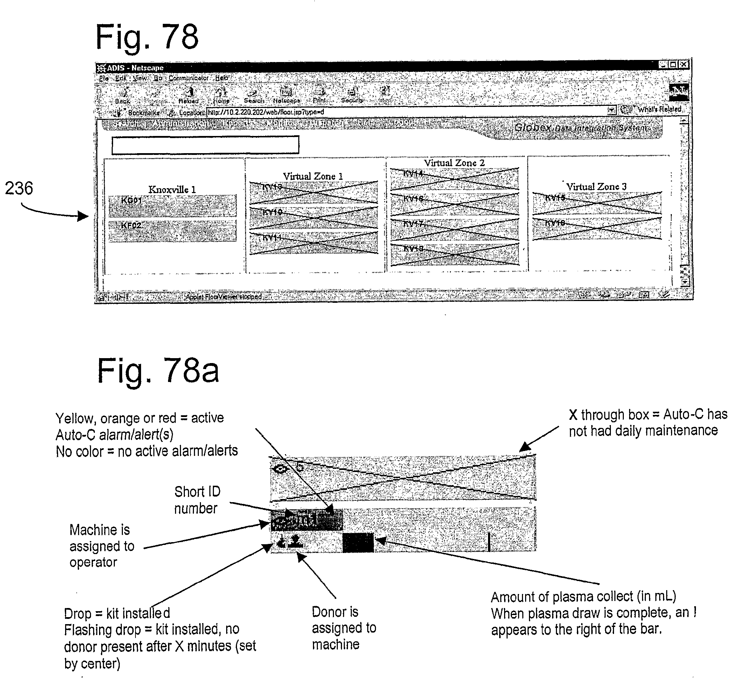

The instrument dispatch screen 236 is illustrated in Figure 78. The dispatch screen 236 is used to monitor the activities in the donor facility. It may be used in the following situations: to dispatch donors to the donation floor; help control work flow in the facility; or provide a quick view of the donor floor. To access the dispatch function, the user clicks "Dispatch" from the options menu. Figure 78a shows potential information that is available on the dispatch screen.

### Wireless Interfacing

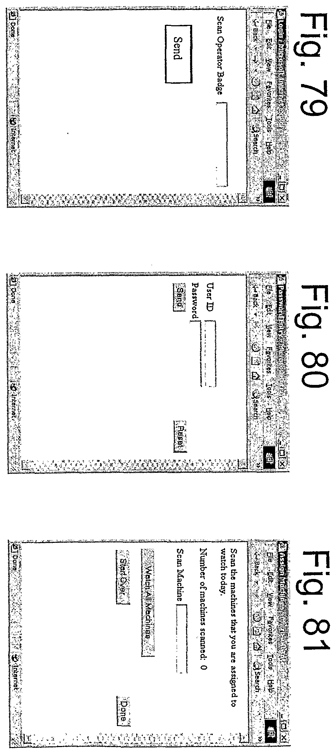

Figures 79-105 illustrate the various screens associated with the wireless interfaces or PDAs. The wireless interfaces are used by the facility operators to interface with the other components of the system. Each wireless interface includes buttons, data entry fields, pick lists, login and logout formats, and multiple input and output screens.

A button is defined as a visual graphic accessed either by a mouse-click or by Tab-Enter/Return. All buttons produce an audible click, and have up, down and disabled states. The up state is the resting state. When a button is down-clicked, the down state appears. When the button is released (the up-click), the button event is processed. Note that when an overlay appears on the screen, underlying buttons are temporarily disabled.

Every data entry field has an active state and an inactive state. Active fields appear brighter than inactive fields. Data input screens come up with the first field active. Only one data entry field is active at a time. An active highlight can be moved to a new field by pressing Enter/Return or Tab, or by clicking the mouse on another field.

Pick lists are offered whenever possible. Pick list entries have an arrow on the right side of the field. When that arrow is clicked, the entire pick list appears with a highlight on its first item. If a pick list is to long to fit onscreen in a single column, it is scrollable. A pick list appears right-justified to the data entry field, and its highlight moves with the cursor up and down the list.

Users can login to a specific URL for each facility or for the headquarters. Once at the site they will be asked for their ID and password to access the database. Users can also log out by pressing "Logout" on the menu. After logging out, the interface returns to the login screen.

Each wireless interface screen is described with reference to a Figure, and an itemization of the objects on screen and their individual functions. However, the actual screens will be high-quality graphic images, whose placement of elements may differ from that of the Figures.

Referring to Figure 79, a login screen is used to log an operator onto a particular palm device. The user scans his/her bar coded ID and presses OK. If the user has used device within several hours (definable variable), the device goes directly to a main menu (but goes to alerts if there are any active alerts). If not, the device goes to a password screen. If the user had previously pressed "Logout" to log out, then the device goes to the password screen. If the user ID does not match fields in the database, the device shows an error message such as "Your badge is unrecognized, please try again"; then allows the user to try again.

Referring to Figure 80, a password screen is used to validate the user's password. The user enters his/her password. The system verifies the user ID and password against the database and checks that these match the previously scanned barcode. If there is a match, the device proceeds to an assign machines screen (Figure 81). If the user ID and password do not match fields in the database, an error message is displayed, and the user is returned to the login screen.

The assign machines screen, Figure 81, is used to assign one or more instruments to a particular operator. Once the assignment is made, alarms/alerts from that instrument may be associated with the operator, for the duration of that operator's shift. The user scans any number of instruments, or the user presses "Watch All Machines" to associate himself/herself with all of the instruments in the facility.

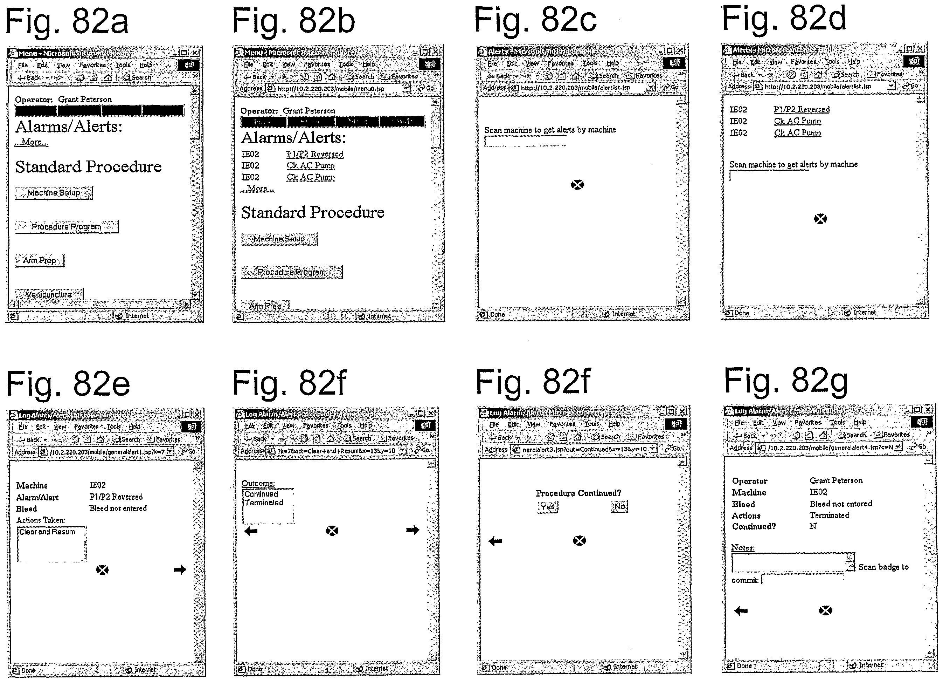

A number of machines scanned so far field displays the number of distinct machines that have been scanned while on this screen. If the user scans an instrument machine, the device adds the machine to the association list, increments the "Number scanned so far" field, and redisplays the same page. If the user clicks "Start over," the device clears the associations and redisplays the page. If the user clicks "Watch All Machines," the device associates all enabled instruments with the operator and goes to the main menu. If the user click "Done," the device goes to the main menu (Figures 82a and 82b) (Note: this can be done even if no machines have been scanned).

The main menu allows the user to select a function. This screen appears if a user has just logged on, or completed a prior action. The main menu is the primary screen in executing any logging sequence. At the end of the various routines, the system automatically returns to this screen.

The routines available through the main menu are categorized into the following four groups which appear as tabs on the upper portion of the screen and as headings as the user scrolls down the main menu: Standard Procedure; Exceptions; Maintenance; and Commands. The user can use the four tabs on the main menu to jump to that menu item. The items associated with that menu tab are then displayed. Alternatively, the user may scroll down the display window to view all the options.

Referring to Figures 82a and 82b, if the user clicks "Alert," the device goes to an "Alert" screen. If the user clicks "Logout," the device goes to the login screen, and clears the association of machines from the operator.

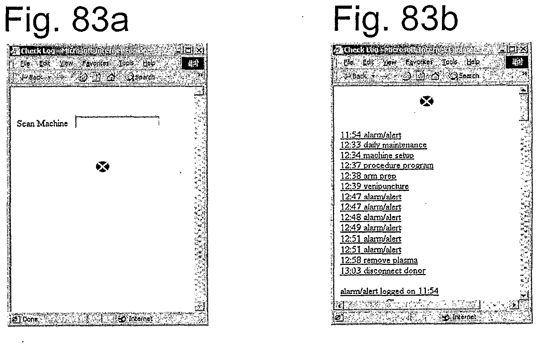

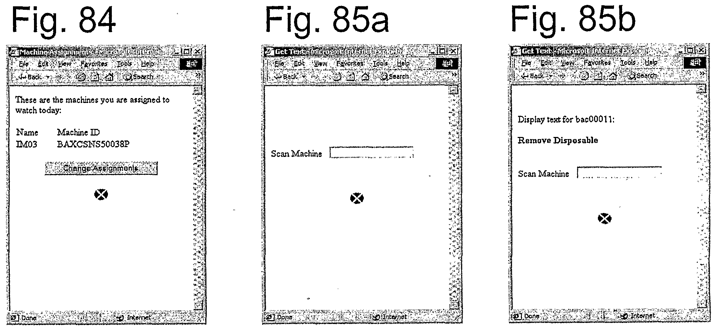

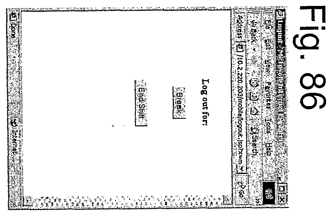

Under the Commands group, if the user clicks "Check Current Bleed Log," the device goes to a show log screen (Figures 83a-83b); if the user clicks "Check Machine Assignments," the device goes to a check machine assignments screen (Figure 84); if the user clicks "Display," the device goes to a check text screen (Figures 85a-85b); and if the user clicks "Logout," the device goes to the logout screen (Figure 86).

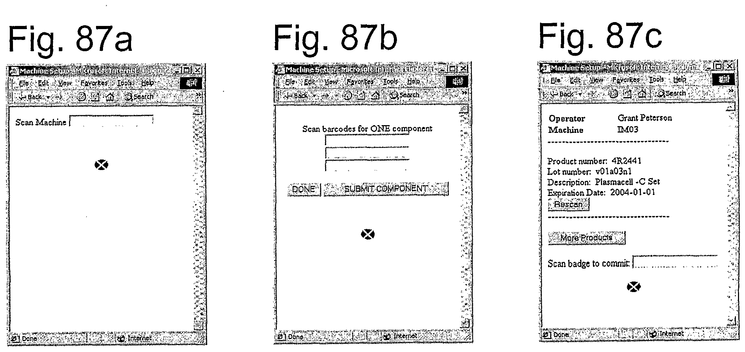

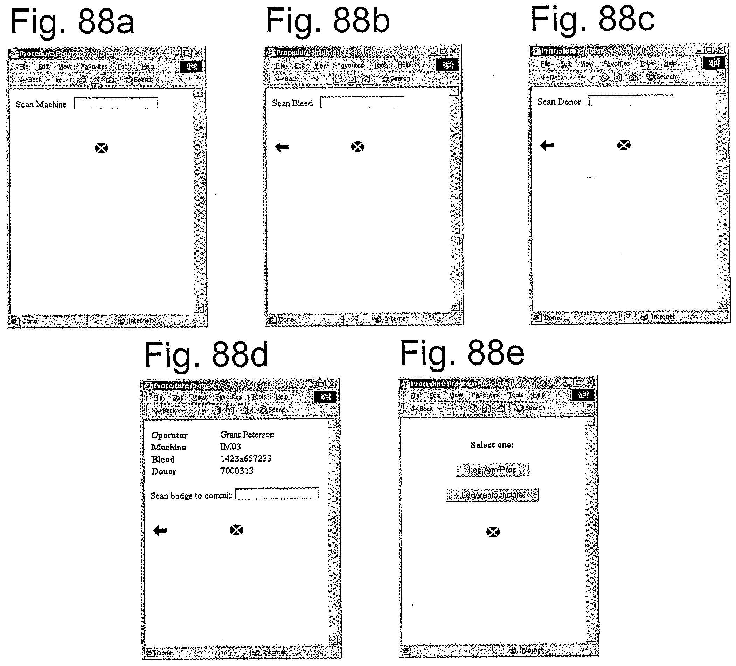

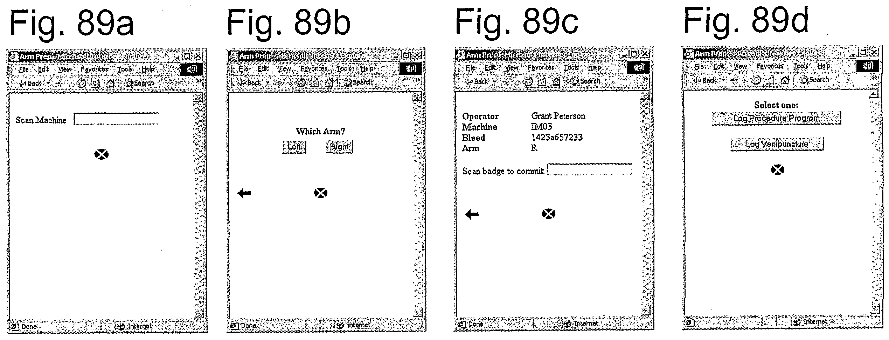

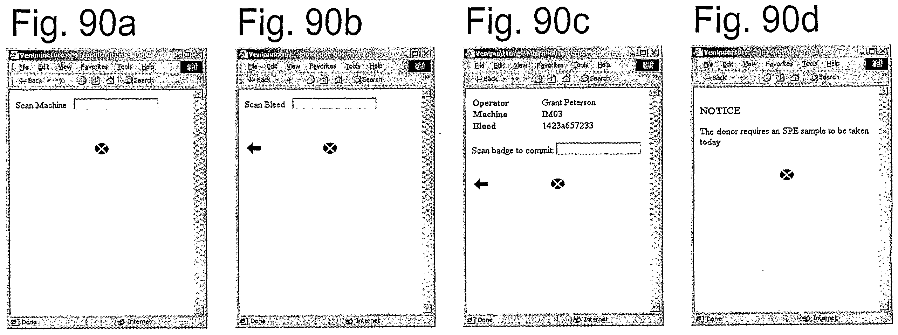

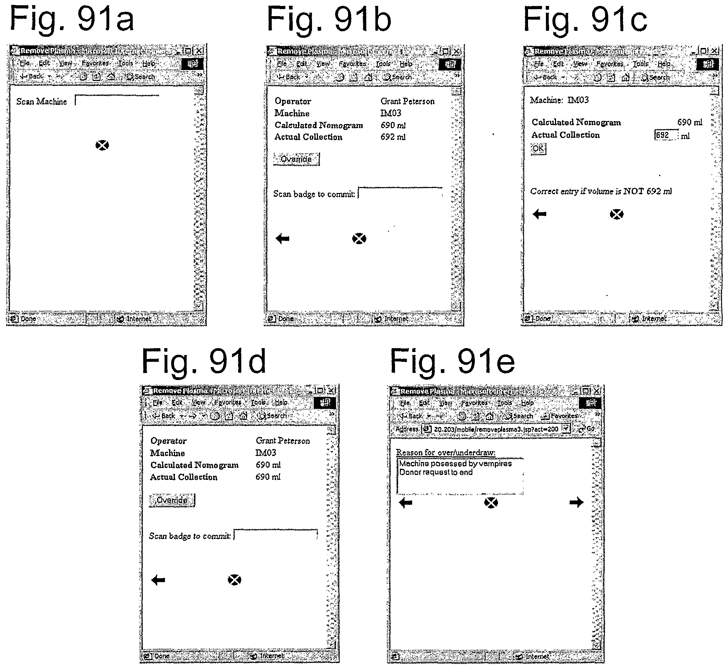

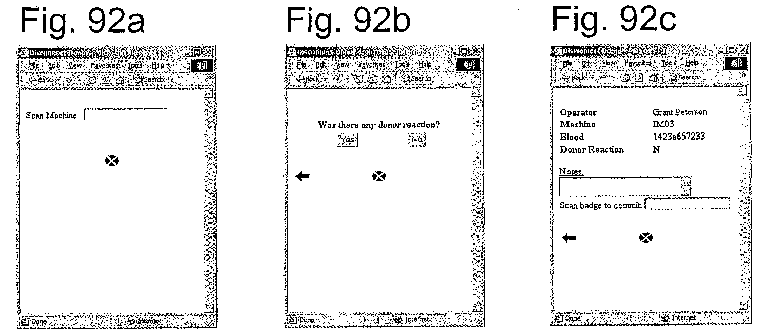

Under the Standard Procedure group, if the user clicks "Machine Setup," the device goes to a machine setup logging screen (Figures 87a-87c); if the user clicks "Procedure Program," the device goes to a procedure program screen (Figures 88a-88e); if the user clicks "Arm Prep," the device goes to the an arm prep screen (Figures 89a-89d); if the user clicks "Venipuncture," the device goes to a venipuncture screen (Figures 90a-90d); if the user clicks "Remove Plasma," the device goes to a remove plasma screen (Figures 91a-91e); and if the user clicks "Disconnect donor," the device goes to a disconnect donor screen (Figures 92a-92c).

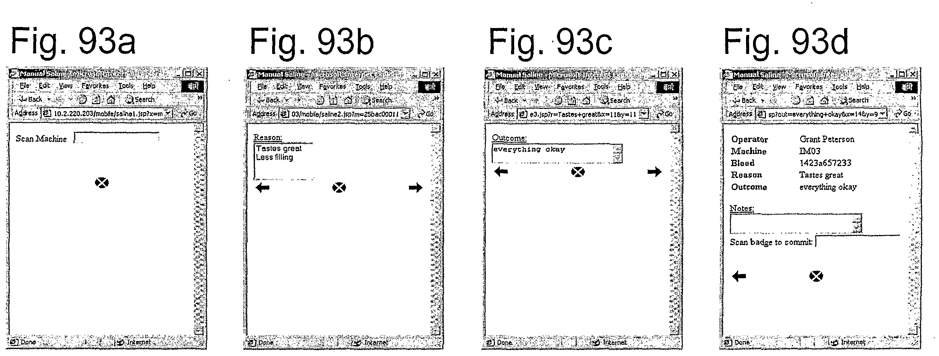

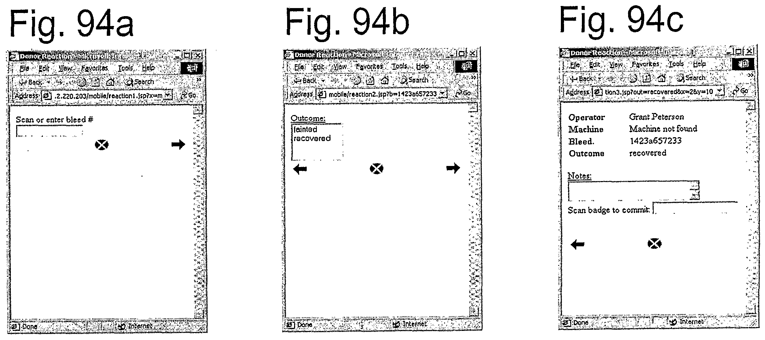

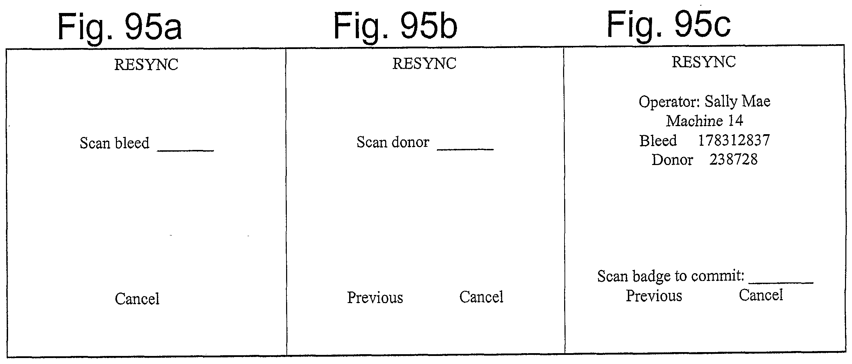

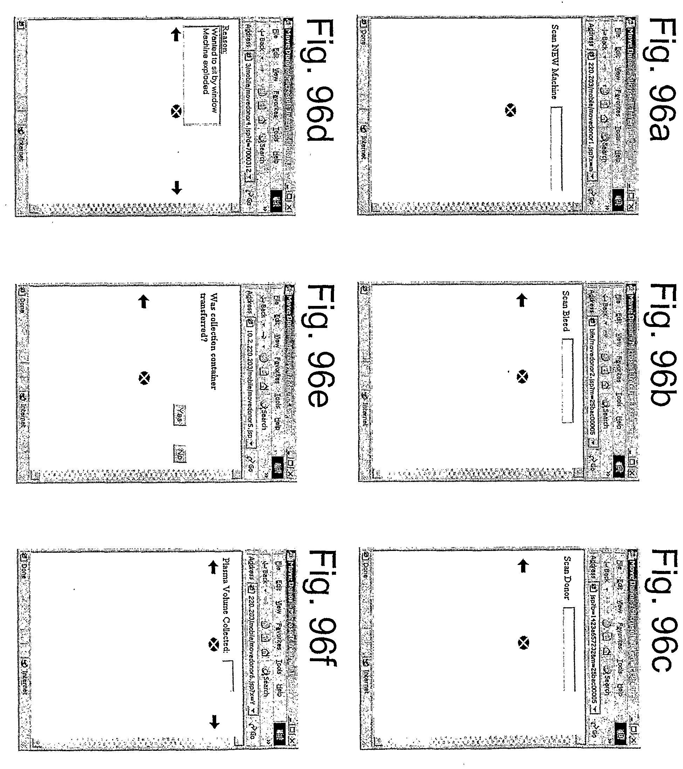

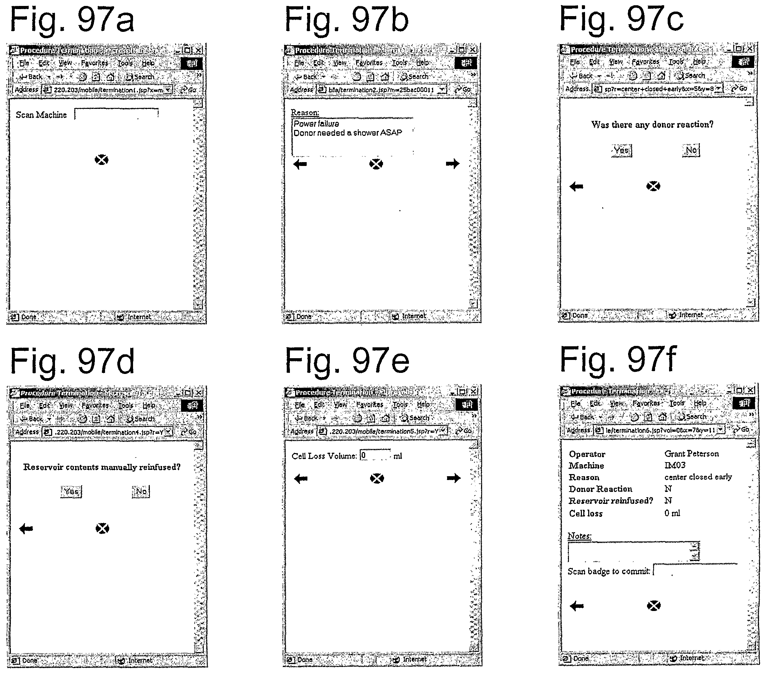

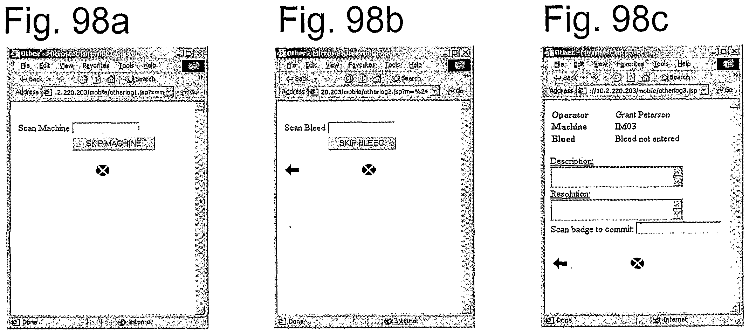

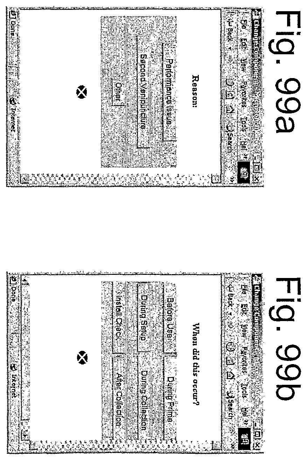

Under the Exceptions group, if the user clicks "Manual Saline," the device goes to a manual saline screen (Figures 93a-93d); if the user clicks "Donor Reaction," the device goes to a donor reaction screen (Figures 94a-94c); if the user clicks "Resync," the device goes to a resync screen (Figures 95a-95c); if the user clicks "Move Donor to different Machine," the device goes to a move donor screen (Figures 96a-96i); if the user clicks "Procedure Termination," the device goes to a procedure termination screen (Figures 97a-97f); if the user clicks "Other Log Entry," the device goes to an other log entry screen (Figures 98a-98c); if the user clicks "Change Kit Component," the device goes to a discard product submenu, page 1 (Figures 99a-99o); and if the user clicks "Product Performance," the device goes to a discard product submenu, page 2 (Figures 99b-99o).

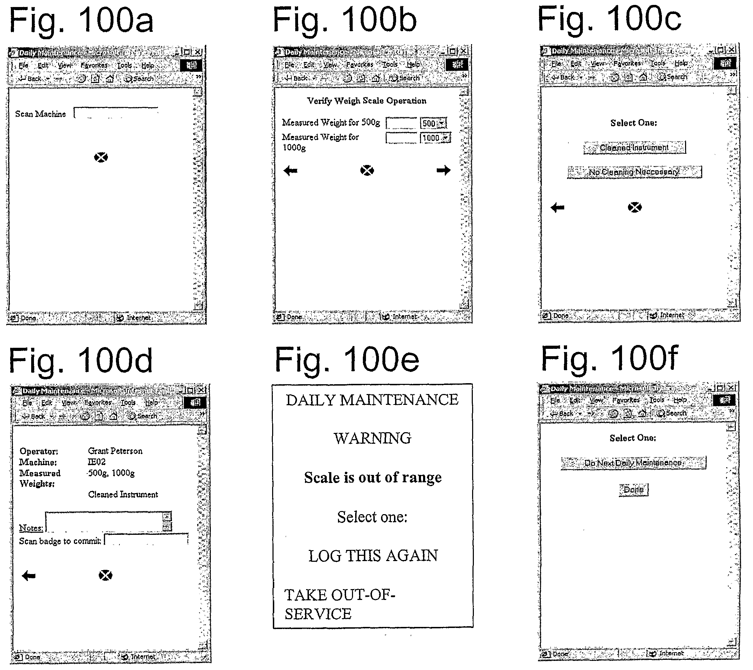

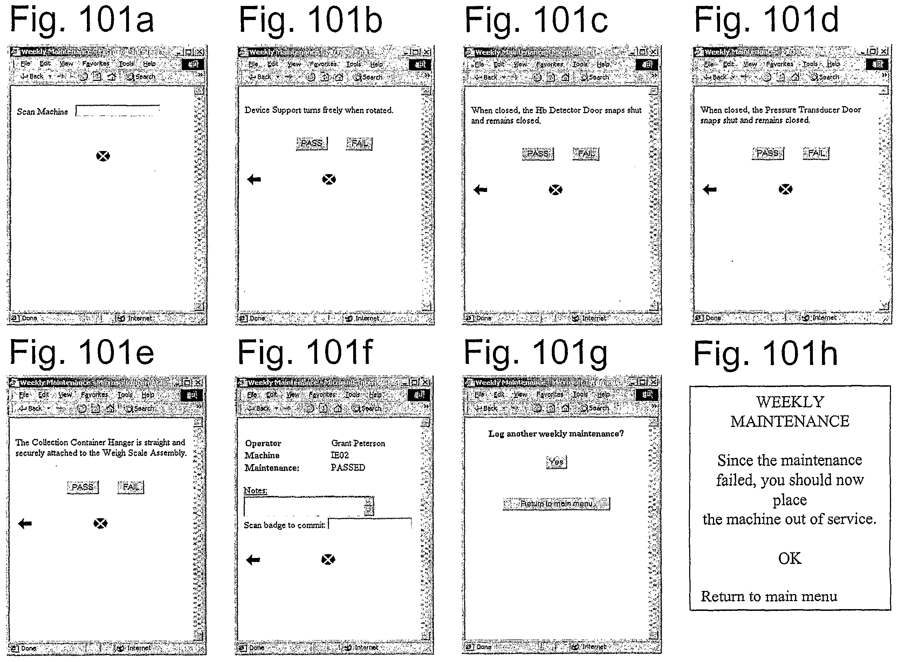

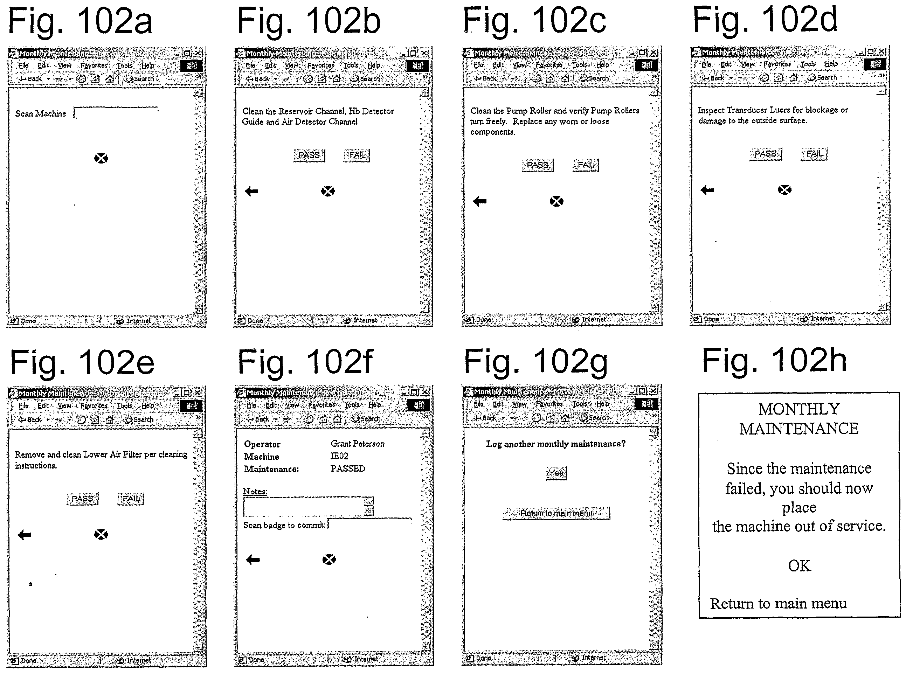

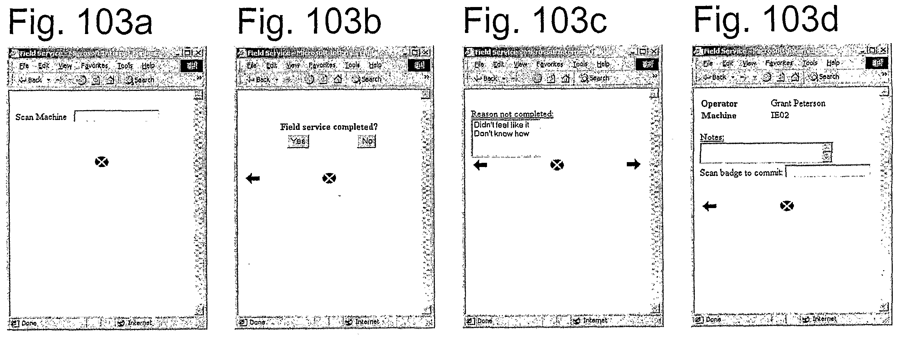

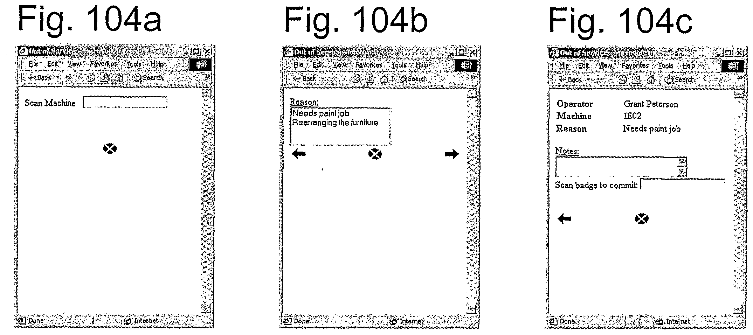

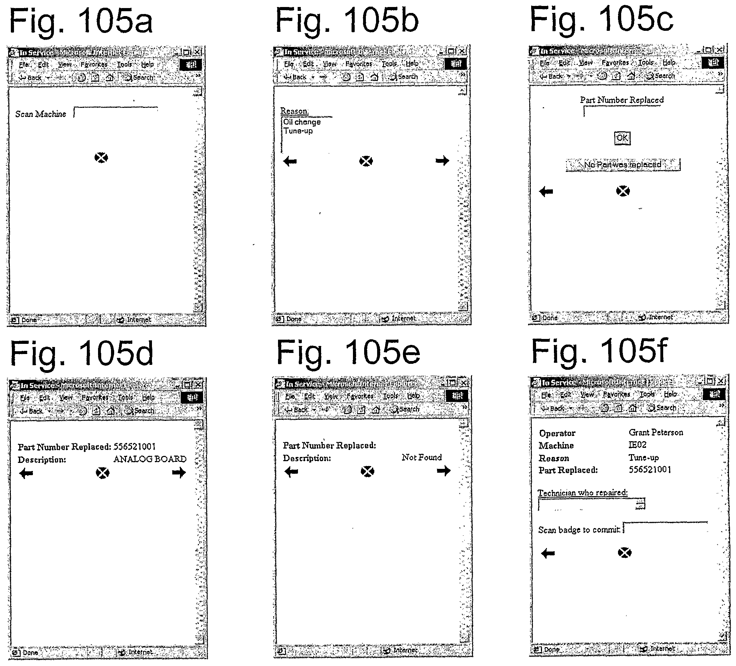

Under the Maintenance group, if the user clicks "Daily Maintenance," the device goes to a daily maintenance screen (Figures 100a-100f); if the user clicks "Weekly Maintenance," the device goes to a weekly maintenance screen (Figures 101a-101h); if the user clicks "Monthly Maintenance," the device goes to a monthly maintenance screen (Figures 102a-102h); if the user clicks "Field Service," the device goes to a field service screen (Figures 103a-103d); if the user clicks "Out of Service," the device goes to an out of service screen (Figures 104a-104c); if the user clicks "Back in Service," the device goes to a back in service screen (Figure 105a-105f).

Now referring to Figures 82a-82h, the "ALERT" button is highlighted, and an alarm/alert log is displayed at the top of the screen (as shown in Figure 82b) when there are active alarm/alerts for the operator. Alarms and alerts are errors, warnings, or notices on any instrument assigned to a user or generated by the system server. When the user selects "More" on this screen, Figure 82d is displayed. The difference between Figures 82c and 82d is that the operator associated with the screen of Figure 82c had no active alarm/alert messages whereas the operator associated with the screen of Figure 82d had several active alarm/alert messages.

The user can resolve alarms and alerts by clicking on any of the listed messages. The details about the selected alarm/alert is then displayed as shown in Figure 82e. The alarms/alerts are listed in reverse chronological order based on when the user last visited each instrument.

To view a list of all alarms/alerts for a given instrument, the user scans the instrument's barcode while the alarm/alert log screen (Figure 82c or 82d) is displayed. The current log is replaced with a list of active alarms/alerts for that machine. To clear an alarm or alert, the user can: select it from the alarm/alert list on the main menu screen, or select it from the alarm/alert log.

When the user clicks on an alarm/alert, if the alarm/alert requires a log response, the device goes to the appropriate logging screen to log the resolution to this alarm/alert, and then clears the alarm/alert after a log is committed. The log screen that is displayed depends upon the type of alarm/alert. If the alarm/alert does not require any response, the device displays Figure 82b. When the user scans a machine, the device replaces the list of alarm/alerts with the list of active alarm/alerts associated with the instrument. When the user clicks "OK," the device clears the alarm/alert and returns to the screen of Figure 82a. However, if the user is not authorized to clear this alarm/alert, the device asks for a supervisor ID scan for validation. When the user clicks "Cancel," the device goes to the screen of Figure 82a.

Figures 83a-85 illustrate the screens under the Command group. First, referring to Figures 83a and 83b, the check log screens are displayed. The check log screen shows what has already been logged in connection with a particular instrument. The operator can view this log to determine whether he/she has forgotten to log an event.

The user scans the instrument to view all log entries made for the bleed in progress on that instrument. The bleed is determined by the bleed in progress. If no bleed is in progress, no information is displayed. Each log entry is listed first by name with no detail, then all of the entries are listed again with full detail. Clicking on the summary entry will scroll the display to the corresponding detail entry.

When the user scans an instrument, if the instrument has a bleed in progress, the device displays the screen of Figure 83b; otherwise, the device shows an error message. If the user clicks on a summary entry (e.g. machine setup), the device scrolls down to the corresponding detail entry. If the user clicks "Done," the device goes to the main menu. If the user clicks "Cancel," (also denoted in the Figures as an "X") the device goes to the main menu.

Figures 84 is an illustration of the check machine assignments screen. This screen is used to check which instruments are assigned to a particular operator. Thus, this screen allows the operator to view a list of the instruments. The machine list field displays all of the instrument numbers (both low number and serial numbers) associated with the operator. If the user clicks "Change," the device goes to the assign machines screen (see Figure 81). If the user clicks "Done," the device goes to the main menu.

The get text screen (Figures 85a and 85b) helps troubleshoot network or setup problems by testing the communication path from the scanner to the instrument. The user scans an instrument, which causes the instrument's display to show up on the PDA as shown on Figure 85b. When the clicks "Done" or "Cancel," the device goes to the main menu.

The remaining screens are event logging screens. The event logging screens are used to log operator actions on the system, e.g. a machine setup, a venipuncture, and so on. All screens contain a top banner displaying the event type. The main part of the screen varies depending on the event type. Many of the events require a series of screens to enter all of the information to log.

If the screen is in a series (except the first), it includes a "Previous" (or back arrow) button and a "Next" (or forward arrow) button, which goes to the previous screen or next screen in the series. If the screen is a single-screen entry or is the last in the series, it includes a "Verify/Scan badge" entry field, which, when filled with the correct user ID for the current user, logs/verifies the event. The "Cancel" (or "X") button takes the user back to the main menu without logging anything.

Log entries require scanning the operator badge to commit/verify. For each attempted log entry, the system matches the scanned badge ID with the ID that was used when the operator logged in initially. If there is a match, the log entry is committed, and the subsequent action is taken (usually to return to the main menu). If it does not match, an error is presented, such as "You did not scan the correct operator badge," and the user returns to the main menu without logging anything. This prevents accidental swapping of devices between operators. If this occurs, the operator will have to start over with the log entry.

Figures 87-92 are related to the Standard Procedure group. First, Figures 87a-87c represent the machine setup screen. The machine setup screen is used for the original instrument setup as well as for replacement products. When called as a subroutine in the latter case, the title is "Replacement Product." The machine setup screen is where the user enters the data necessary for a machine setup log entry. Up to 10 product components can be entered, and the screen of Figure 87b is presented multiple times as necessary. The scan machine field comprises the scanned instrument ID. The unlabeled scan fields comprise one to three barcode scans including the product code, lot number, and expiration date. These are shown as scanned on screen and unpacked into the components when saved and shown in Figure 87c. When the user scans a machine, the device displays the screen of Figure 87b. When the user clicks "NEXT" or "More Products," the device repeats the screen of Figure 87b for another component. When the user clicks "Done," the device shows the screen of Figure 87c. When the user clicks "Re-scan," the device deletes the component and returns to Figure 87b to allow the operator to re-scan the component. The operator may simply press "DONE" again to remove the component without replacing it with a new one. When the user scans the correct operator ID, the device commits the log entry and goes to the main menu.

The procedure program screen is illustrated in Figures 88a-88e. The procedure program screen is where the user enters the data necessary for a procedure-program log entry. If after the screen of Figure 88c, the system detects that the bleed and donor do not match a bleed/donor pair passed from the facility's system, a warning is presented, and the user may not continue. The plasma volume programmed must match the value passed from the facility system, but this check is done in the background, not as part of the sequence of these screens. If there is a mismatch, a new alarm/alert is generated to this effect. The scan machine, scan bleed, and scan donor contain the scanned IDs for the instrument, bleed number, and donor, respectively.

If the user scans the instrument, the device goes to the page of Figure 88b. If user scans the bleed number, the device goes to the page of Figure 88c. If the user scans the donor number, the device goes to the page of Figure 87d. If the user clicks "Log Arm Prep," the device goes to page 2 (Figure 89b) of the arm prep log screen (assume the same machine). If the user clicks "Log Venipuncture," the device goes to page 2 (Figure 90b) of the venipuncture log screen (assume the same machine). If the user clicks "Done," the device goes to the main menu. If the user scans the correct operator ID, the device logs the entries and returns to main menu.

Figures 89a-89d represent the arm prep screen. The user enters data necessary for an arm prep log entry on the arm prep screen. The screen comprises the scan machine field which contains the scanned instrument ID.

If the user scans the instrument, the device goes to the page of Figure 89b. If the user clicks "Left" or "Right," the device goes to the page of Figure 89c. If the user scans the correct operator ID, the device logs the entries and goes to the venipuncture screen. If the user clicks "Log Venipuncture," the device goes to the page 2 (Figure 90b) of the venipuncture screen (assume the same machine). If the user clicks "Log Procedure Program," the device goes to page 2 (Figure 88b) of the procedure program log screen (assume the same machine).

The user enters the data necessary for a venipuncture log entry on the venipuncture screen (Figures 90a-90d). If after page 3 (Figure 90c), the system detects that a blood sample is required (based on inputs from the facility system), the device presents page 4 (Figure 90d). Else, the device skips page 4. The scan machine and the scan bleed fields comprise the instrument ID and the bleed number, respectively.

If the user scans instrument, the device goes to page 2 (Figure 90b). If the user scans the bleed number, the device checks that the bleed number is correct (the same bleed that was scanned for the procedure-program log entry on the same machine) and displays an error message if not. If OK, the device goes to page 3 (Figure 90c). If the user scans the correct operator ID, the device logs the entries. If a blood sample is needed, the device goes to page 4 (Figure 90d). Else, the device goes to the main menu.

The user enters data necessary for a remove plasma log entry on the remove plasma screen (Figures 91a-91e). If this screen is accessed from the main menu, page 1 (Figure 91a) is shown first. If the screen is accessed from the alert screen, page 2 (Figure 91b) is shown first (since the machine is already known based on the alert). Page 3 (Figure 91c) is only shown if the system detects an over or under-draw (UON or UUN).

The calculated nomogram is displayed in the calculated nomogram field. This is the value passed from the facility system. The actual collection field is an entry field. The default value displayed in the field is the value calculated by the instrument. The user can override this. The reason for over/under-draw field is a pick list field which contains user defined reasons.

If the user scans the instrument, the device checks whether there is a remove plasma alert active for that instrument. If there is no active alert, the device goes to page 2 (Figure 91b). If there is an active alert, the device gives an error message. If the user clicks "OK" (Figure 91c) the device calculates whether the default or corrected actual volume constitutes a UUN/UON. If so, the device goes to page 4 (Figure 91d). Else, the device goes to page 5 (Figure 91e). If the user clicks a reason from page 4 (figure 91d), the device goes to page 5 (Figure 91e). If the user scans the correct operator ID, the device logs the entries and goes to the main menu.

The user enters data necessary for a disconnect donor log entry on the disconnect donor screen (Figures 92a-92c). If this screen is accessed from the main menu, page 1 is shown first. If the screen is accessed from the alert screen, page 2 (Figure 92b) is shown first (since the machine is already known based on the alert).

If the user scans the instrument, the device checks whether there is a procedure-end alert active for that instrument. If there is no active alert, the device goes to page 2 (Figure 92b). Otherwise, the device shows error message. If the user clicks "Yes" or "No," the device goes to page 3 (Figure 92c). If the user scans the correct operator ID, the device logs the entries and goes to the main menu.

The screens of the Exceptions group are illustrated in Figures 93-99. First, the manual saline screen comprises the data necessary for a manual saline log entry (Figures 93a-93d). The reason and outcome fields are a pick list fields. The notes field is a free-form entry field.

If the user scans the instrument, the device goes to page 2 (Figure 93b). If the user selects a reason, the device goes to page 3 (Figure 93c). If the user selects an outcome, the device goes to page 4 (Figure 93d). If user scans the correct operator ID, the device logs the entries and displays the main menu.

The donor reaction screen (Figures 94a-94c) comprises the data necessary to produce a donor reaction log entry. This log type requires the entry of a bleed number instead of an instrument ID due to the possibility that the reaction occurred after the donor left the instrument, and the instrument may be in use with another donor. The scan bleed field comprises the scanned bleed number. The outcome field is a pick list field. The notes field is a free-form entry field.

If the user scans the bleed number, or manually enters it, the device displays page 2 (Figure 94b). If the user clicks an outcome, the device displays page 3 (Figure 94c). If user scans the correct operator ID, the device logs the entries and goes to the main menu.

The reboot-resync screen (Figures 95a-95c) allows the user to enter the data necessary for a reboot-resync log entry, which is required only in failure cases when the system server is rebooted while a donation is in progress. However, the instrument is identified by the system and does not have to be scanned because this log entry is made in response to an alert that is based on the machine. The scan bleed field comprises the scanned bleed number, and the scan donor comprises the scanned donor ID.

If the user scans the bleed number, the device advances to page 2 (Figure 95b). If the user scans the donor number, the device advances to page 3 (Figure 95c). If user scans the correct operator ID, the device logs the entries and goes to the main menu.

The move to a different machine screen (Figures 96a-96i) provides the means for the user to enter the data necessary for a move-donor log entry. The reason and outcome fields are facility definable pick list fields. The plasma volume and notes field are entry fields.

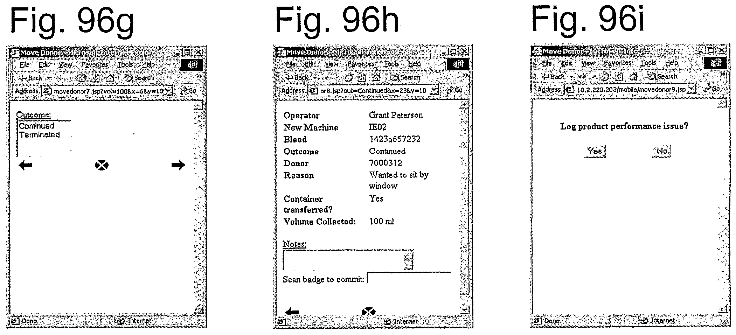

When the user scans the instrument, the device goes to page 2 (Figure 96b). When the user scans the bleed number, the device goes to page 3 (Figure 96c). When the user scans the donor ID, the device goes to page 4 (Figure 96d). When the user selects a reason, the device goes to page 5 (Figure 96e). If the collection container was transferred to a new machine, the user selects "Yes," and page 6 (Figure 96f) is displayed. If the collection container was not transferred to the new machine, the user selects "No," and page 7 (Figure 96g) is displayed.

If page 6 (Figure 96f) is displayed, the user enters the volume of plasma collected before moving the donor. The user then selects "Next," and page 7 (Figure 96g) is displayed. When the user selects an outcome, the device goes to page 8 (Figure 96h). If user scans the correct operator ID, the device logs the entries and goes to page 9 (Figure 96i). If the user wishes to log a product performance issue, he/she selects "Yes" on page 9 (Figure 96i), the product performance issue routine (described below) is displayed. If the user does not wish to log a product performance issue, he/she selects "No", and the main menu is displayed.

The procedure termination screen allows the user to enter the data necessary for a procedure-termination log entry (Figures 97a-97f). This is used for an abnormal disconnect, not for a successful completion. The reason field is a facility-definable pick list field. The cell loss volume field is a numeric entry field. The default value displayed is calculated from instrument data.

When the user scans an instrument, the device goes to page 2 (Figure 97b). When the user selects a reason, the device goes to page 3 (Figure 97c). If the user clicks "Yes" or "No" on page 3, the device goes to page 4 (Figure 97d). If the user clicks "Yes" or "No" on page 4, the device goes to page 5 (Figure 97e). The cell loss volume as calculated by the machine is displayed on page 5, if available. If this volume is incorrect or if no cell loss volume is displayed, the user enters the volume manually. If the user clicks "Next" on page 5, the device goes to page 6 (Figure 97f). If the user scans the correct operator ID, the device logs the entries and goes to the main menu.

The other log entry screen (Figures 98a-98c) allows the user to enter the data necessary for a miscellaneous log entry. This would be used for unusual occurrences for which no log entry type was defined.

If the user scans the instrument or clicks "Skip Machine," the device displays page 2 (Figure 98b) . If the user scans the bleed number or clicks "Skip Bleed," the device displays page 3 (Figure 98c). If user scans the correct operator ID, the device logs the entries and goes to the main menu.

Referring to Figures 99a-99o, the screens associated with a discarded product (disposables/soft goods) log entry are illustrated. Because the scenarios that occur when products are discarded are especially complex, they are broken out into sub-menus and several subroutines. The required data items that must be stored with each of the three kinds of log entries used within the change-kit scenarios are product performance (records when any product is discarded for any reason), second venipuncture (records when a second stick is made for any reason), and machine setup (records when a new product is used for any reason).

If the user selects the "Change Disposable Component" button under Exceptions from the main menu, the change disposable screen is activated. When the user clicks the "Change Disposable Component" button, page 1 (Figure 99a) is displayed. Upon selecting any of the three logging options on page 1, page 2 (Figure 99b) is displayed. Depending on the logging option selected on page 1 (Figure 99a), the specific screens that the device displays will vary depending on the reason for the change and when the change occurred (from page 2), the reason for the discard, performance issue, second venipuncture, or other. If the disposable is discard for a performance issue, there are three types of information to log: the reason for the change; information about the component being discarded; and information about the replacement component.

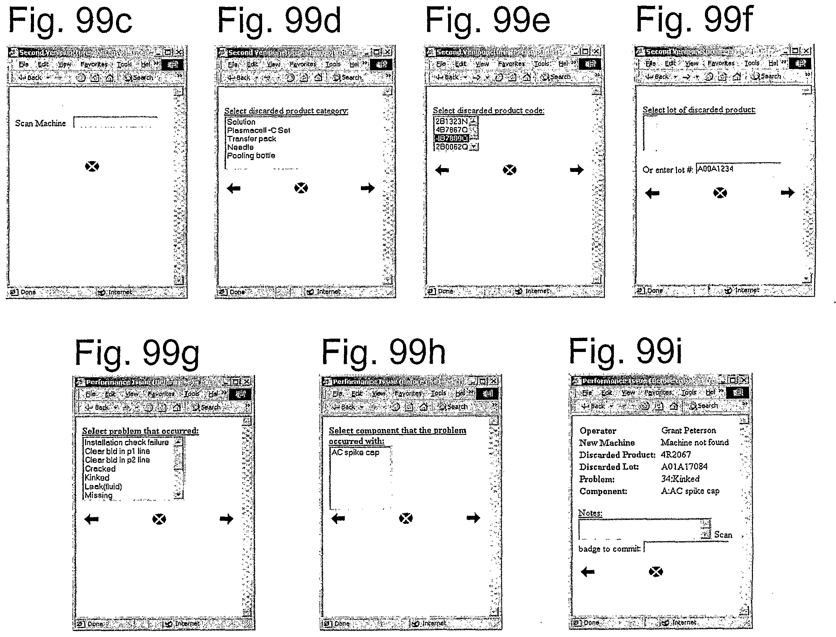

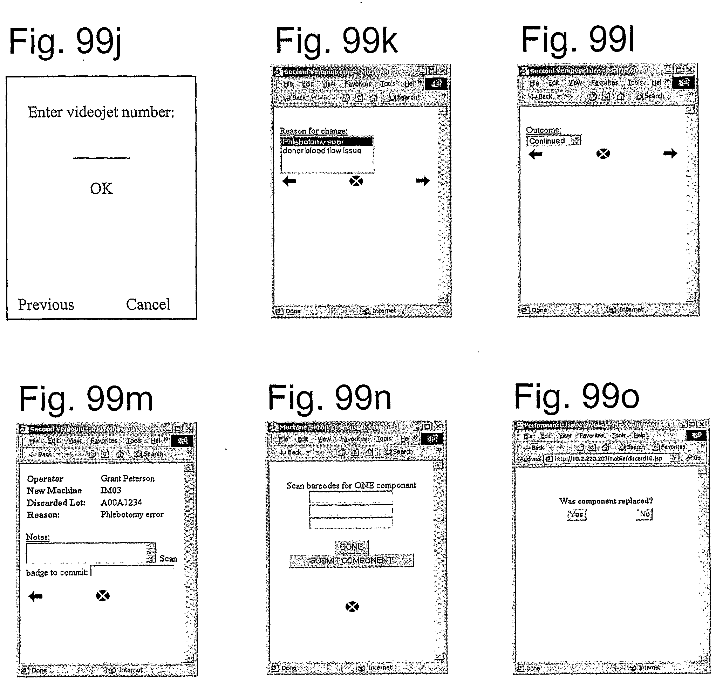

If the disposable is changed for a performance issue prior to the bleed procedure, the following procedure is followed. The user selects "Performance Issue" on page 1 (Figure 99a), and page 2 (Figure 99b) is displayed. The user selects "Before Use" on page 2, and page 3 (Figure 99c) is displayed. The user scans the instrument's barcode, and page 4 (Figure 99d) is displayed. A discarded product category is selected from a pick list on page 4, and page 5 (Figure 99e) is displayed. Once the user has selected a discarded product code from the list provided on page 5, page 6 (Figure 99f) is displayed. On page 6, the user either selects the lot number of the discarded product or enters the lot number manually. After the lot number is entered, page 7 (Figure 99g) is displayed. On page 7, the user selects the problem that occurred with the disposable product, and page 8 (Figure 99h) is displayed. On page 8, the user selects the component that the problem occurred with from the list provided. If the problem occurred with a separation device, then page 10 (Figure 99j) is displayed. Otherwise, page 9 (Figure 99i) is displayed.

On page 10, the user enters the videojet number in the spaced provided and selects "OK." Page 9 (Figure 99i) is then displayed.

On page 9, the user verifies the information that is displayed and uses the space provided to enter additional notes about the discarded component. The information is verified and committed to the system when the user scans his/her ID badge. The main menu is then displayed.

In a second scenario, the disposable product is discarded for a performance issue after the bleed has begun. In this scenario, the user selects "Performance Issue" from page 1 (Figure 99a), page 2 (Figure 99b) is displayed. On page 2, the user selects when the products was changed (other than "Before Use"), page 3 (Figure 99c) is displayed. On page 3, the user scans the instrument's barcode number, and page 4 (Figure 99d) is displayed. A discarded product category is selected from a pick list on page 4, and page 5 (Figure 99e) is displayed. Once the user has selected a discarded product code from the list provided on page 5, page 6 (Figure 99f) is displayed. On page 6, the user either selects the lot number of the discarded product or enters the lot number manually. After the lot number is entered, page 7 (Figure 99g) is displayed. On page 7, the user selects the problem that occurred with the disposable product, and page 8 (Figure 99h) is displayed. On page 8, the user selects the component that the problem occurred with from the list provided. If the problem occurred with a separation device, then page 10 (Figure 99j) is displayed. Otherwise, page 9 (Figure 99i) is displayed.

On page 10, the user enters the videojet number in the spaced provided and selects "OK." Page 9 (Figure 99i) is then displayed.

On page 9, the user verifies the information that is displayed and uses the space provided to enter additional notes about the discarded component. The information is verified and committed to the system when the user scans his/her ID badge, and page 15 (Figure 99o) is displayed. On page 15, if a component was replaced, the user selects "Yes," and page 14 (Figure 99n) is displayed. On page 14, the user scans the barcodes for the replaced component, selects "Done," and the main menu is displayed. If the component was not replaced, the user selects "No," and the main menu is displayed.

In the next scenario, the user selects "Second Venipuncture" from page 1 (Figure 99a), and page 3 (Figure 99c) is displayed. On page 3, the user scans the instrument's barcode number, and page 4 (Figure 99d) is displayed. A discarded product category is selected from a pick list on page 4, and page 5 (Figure 99e) is displayed. Once the user has selected a discarded product code from the list provided on page 5, page 6 (Figure 99f) is displayed. On page 6, the user either selects the lot number of the discarded product or enters the lot number manually. Once the lot number is entered, page 11 (Figure 99k) is displayed. The reason for changing the component is selected from the list provided on page 11, and page 12 (Figure 99l) is displayed. The outcome of the second venipuncture is selected from the list provided on page 12, and page 13 (Figure 99m) is displayed. The user verifies the information displayed and uses the space provided to enter additional notes about the discarded component. The user scans his/her ID badge to commit/verify the information, and page 15 (Figure 99o) is displayed. On page 15, if a component was replaced, the user selects "Yes," and page 14 (Figure 99n) is displayed. On page 14, the user scans the barcodes for the replaced component, selects "Done," and the main menu is displayed. If the component was not replaced, the user selects "No," and the main menu is displayed.

The final scenario is when a product is changed for any other reason. Here, the user selects "Other" from page 1 (Figure 99a), and page 3 is displayed. On page 3, the user scans the instrument's barcode number, and page 4 (Figure 99d) is displayed. A discarded product category is selected from a pick list on page 4, and page 5 (Figure 99e) is displayed. Once the user has selected a discarded product code from the list provided on page 5, page 6 (Figure 99f) is displayed. On page 6, the user either selects the lot number of the discarded product or enters the lot number manually. Once the lot number is entered, page 11 (Figure 99k) is displayed. The reason for changing the component is selected from the list provided on page 11, and page 13 (Figure 99m) is displayed. The user verifies the information displayed and uses the space provided to enter additional notes about the discarded component. Next, the user scans his/her ID badge to commit/verify the information, and page 15 (Figure 99o) is displayed. On page 15, if a component was replaced, the user selects "Yes," and page 14 (Figure 99n) is displayed. On page 14, the user scans the barcodes for the replaced component, selects "Done," and the main menu is displayed. If the component was not replaced, the user selects "No," and the main menu is displayed.

The screens of the Maintenance group are shown in Figures 100-105. First, the daily maintenance screen (Figures 100a-100f) is where the user enters the data necessary for a daily maintenance log entry. The measured weight for 333g field is an entry screen. The weight, 333g, is an example of the calibration weigh used. The field would show the actual facility-defined weight #1. Likewise, the measured weight for 666g is a similar entry field. The notes field is a free-form entry field.

When the user scans the instrument, the device goes to page 2 (Figure 100b). If the user clicks "Next," the device goes to page 3 (Figure 100c). If the user clicks "Cleaned instrument" or "cleaning not necessary," the device goes to page 4 (Figure 100d). If user scans the correct operator ID, the device logs the entries. If the weights are within the facility-defined % acceptable error from the actual weights, then the device goes to page 6 (Figure 100f). Else the device goes to page 5 (warning) (Figure 100e). If the user clicks "Again," the device goes to page 1 (for a new log entry). If the user clicks "Out of Service," the device goes to the out of service screen. If the user clicks "Done," the device goes to the main menu.

The weekly maintenance screen (Figure 101a-101h) allows the user to enter the data necessary for a weekly maintenance log entry. Pages 2-5 (Figures 101b-101e) do not result in any logged data. They are reminders only. However, if any of the items fail, that item is logged as the failure.

The scan machine field comprises the scanned instrument ID. The notes field is a free-form entry field. The maintenance field is a display comprising the either "PASSED" or "FAILED."

If the user clicks "PASS," the device goes to the next screen in sequence. If the user clicks "FAIL," the device skips directly to page 6 (Figure 101f) and remembers the description of the failed procedure. If the user scans the correct operator ID, the device logs the entries. If "FAIL" had been clicked for any item, the device goes to page 8 (Figure 101h). If all items passed, the device goes to page 7 (figure 101g). If the user clicks "Yes," the device goes to page 1 (for anew log entry). If the user clicks "Return to menu," the device goes to the main menu. If the user clicks "OK" on page 8, the device goes to the out of service screen, page 2 (assumes same instrument).

The monthly maintenance screen (Figures 102a-102h) allows the user to enter the data necessary for a monthly maintenance log entry. Pages 2-5 (Figures 102b-102f) do not result in any logged data. Rather, they are reminders only. However, if any items failed, the description of the failed item is logged.

The scan machine field comprises the scanned instrument ID. The notes field is a free-form entry field. The maintenance field is a display comprising the either "PASSED" or "FAILED."

If the user clicks "PASS," the device goes to the next screen in sequence. If the user clicks "FAIL," the device skips directly to page 6 (Figure 102f) and remembers the description of the failed procedure. If the user scans the correct operator ID, the device logs the entries. If "FAIL" had been clicked for any item, the device goes to page 8 (Figure 102h). If all items passed, the device goes to page 7 (Figure 102g). If the user clicks "Yes," the device goes to page 1 (for a new log entry). If the user clicks "Return to menu," the device goes to the main menu. If the user clicks "OK" on page 8, the device goes to the out of service screen, page 2 (assumes same instrument).

The field service screen (Figures 103a-103d) is where the user enters the data necessary for a field service log entry. The scan machine field comprises the scanned instrument ID. The reason field is a facility-definable pick list field. The notes field is a free-form entry field.

If the user clicks "Yes," the device goes to page 4 (Figure 103d) (skips page 3). If the user clicks "No," the device goes to page 3 (Figure 103c). If the user clicks on a reason on page 3, the device goes to page 4. If user scans the correct operator ID, the device logs the entries and goes to the main menu.

The out of service screen (Figures 104a-104c) is where the user enters the data necessary for an out of service log entry. The log entry disables further use of the instrument. The scan machine field comprises the scanned instrument ID. The reason field is a facility-definable pick list field. The notes field is a free-form entry field.

If the user scans the instrument, the device goes to page 2 (Figure 104b). If the user selects a reason, the device goes to page 3 (Figure 104c). If the user scans the correct operator ID, the device logs the entries and goes to the main menu.

The back in service screen (Figures 105a-105f) allows the user it enter the data necessary to enter the data for a back-in-service log entry. The scan machine field comprises the scanned instrument ID. The part number replaced screen is an entry field where the user enters a part number. The description field is a display field based on a lookup using the part number entered or "not found" if the part number is not found. The technician field is an entry field comprising the name of the technician. The reason and part fields are a facility-definable pick list fields.

If the user scans the instrument, the device checks if a field service report was logged since the last time an out-of-service was logged. If so, the device goes to page 3 (Figure 105c). If not (error condition), the device goes to page 2 (Figure 105b). If the user clicks "Ignore warning," the device goes to page 3 (Figure 105c). If the user selects a reason, the device goes to page 4 (Figure 105d). If the user clicks "OK" on page 4, the device looks up the part number entered, and goes to page 5 (Figure 105e). If the part number is not found, the device displays "Not found" for description. If the user clicks "No part replaced," the device goes to page 6 (Figure 105f). If the user scans the correct operator ID, the device logs the entries and goes to the main menu.

### Instrument Data

The system 10 also allows a facility to gather data from the laboratory instruments. This data can be monitored in real time, or near real time, from remote locations, the workstation(s), or the PDAs. The present system has the ability to convert parallel data to Ethernet which allows the data to be seen using a common web browser. This enables present system to be integrated into existing blood collection facilities that utilize legacy apheresis instruments having a proprietary pin arrangement, such as the Autopheresis-C plasmapheresis instrument supplied by the Fenwal Division of Baxter International. The data conversion is accomplished by the serial/parallel to Ethernet converters or NetDev™ devices 24a, 24b, 24c.

Typically, an Autopheresis-C apheresis instrument transmits parallel data at fixed intervals. Each data sample consists of a binary stream. The data transmission limitations of an Autopheresis-C communication are overcome by the serial/parallel to Ethernet converters 24a, 24b, 24c. The converters 24a, 24b, 24c are add-on circuit boards that repackage the Autopheresis-C data sample into an IP/UDP packet for transmission over the first Ethernet 30. From a network point of view, the converters 24a, 24b, 24c are nodes, although the source of data comes from the instruments 20a, 20b, 20c. The converters 24a, 24b, 24c act as a pass-through of instrument 20a, 20b, 20c data samples to the system server 34.

An Autopheresis-C is typically designed to operate in transmit mode only, although a hardwired signal line between each Autopheresis-C and each converter can be used to communicate network data to the Autopheresis-C. Such data may originate from the system server 34 in the form of acknowledgment data, which then passes through the converter to the Autopheresis-C.

There are several types of data packets transmitted from the instruments 20a, 20b, 20c to the system server 34. The formats for all of the packets contain header information about the actual length of data that they encapsulate. A first type of packet contains diagnostic information, including crash reports, stack and ram dump. The data are sent once in continuous blocks at power-up of the apheresis instrument.

A second type of packet contains run information and is sent is sent approximately 10 times per second (unless replaced by a configuration packet, as described below) after power up and initial start up (lasting approximately 5 seconds) of the instruments 20a, 20b, 20c. This occurs throughout the power-on state of the instruments 20a, 20b, 20c, whether it is in a bleed or not. A modecode field of the packet of the second type is examined to determine the state of the machine. Each run packet includes a first echo field that the instruments 20a, 20b, 20c expect the system server 34 to echo back in a data packet within 10 seconds after it was transmitted by an instrument 20a, 20b, 20c.

A third type of packet contains configuration data. This packet is sent in place of a run packet at predetermined intervals. At each of the intervals, the configuration packet is sent 5 times, once per second (over a 5 second total) every 6 minutes, and at specific states of the machine, such as power-up, before the venipuncture is expected, and at the end of a bleed.

Additional data packets, called network management packets, are transmitted on different ports than the diagnostic, run, and configuration packets. A first network management packet is transmitted from the converters 24a, 24b, 24c to the system server 34. This packet is generated by a converter and received by the system server 34 and is the first packet to be sent after a converter reboot (after bootp reply). Thereafter it is repeated every 8 seconds (with redundant duplicates). This packet provides information about communication status.

A second network management packet, or reset packet, is transmitted from the system server 34 to each converter 24a, 24b, 24c. This packet is sent as a broadcast out of a port on the system server 34 when the system server 34 boots, or can be sent to an individual device. The reset packet causes the converter to reboot.

A third network management packet, or parameter packet, is sent by the system server 34 primarily to signal to an instrument 20a, 20b, 20c that the system server 34 has received the run packets. The system server 34 is expected to the parameter packet every 3 seconds. The parameter packet contains the first echo field response to the run packet. Each parameter packet further includes a second echo field that the instruments 20a, 20b, 20c echo in the run packet, and a third echo that the converters 24a, 24b, 24c echo in the information packet. The system server 34 can use these echo fields to determine of the last parameter packet transmitted by the system server 34 was received by either the converters 24a, 24b, 24c or the instruments 20a, 20b, 20c.

In a typical scenario, an apheresis instrument is booting when the system server 34 is already running. First, the converter 24 boots. At reset, an indicator lamp flashes. The converter 24 sends bootp requests until it gets a broadcast reply from the system server 34 assigning it an IP address. Once the converter has an IP address, it will send an information packet to gather apheresis instrument data and transmit the data to the system server 34. The converter will timeout and reboot when it detects no apheresis instrument activity after 3 seconds, or no system server 34 activity after 8 seconds.

The converter boot is relatively quick. Once the converter boot is completed, the converter waits for the apheresis instrument to boot.

Once the converter boot (bootp) is completed and after the system server 34 has received the information packet from the converter, the system server 34 sends a parameter packet to the converter.

On power up, the apheresis instrument sends a diagnostic packet to the system server 34. If the diagnostic packet includes a non-functional report, the system server 34 disables the apheresis instrument.

Next, the apheresis instrument sends a configuration packet to the system server 34. Thus, the system server 34 receives an information packet, a diagnostic packet, and a configuration packet shortly after the apheresis instrument boots.

Generally during a bleed procedure, a first indicator lamp or LED flashes as the converter sends Ethernet packets to the system server 34. A second indicator lamp or LED signals when data collisions occur. An apheresis instrument sends a run or configuration packet at predetermined intervals. The majority of the data traffic on the first network 12 is made up of run packets. Configuration packets are interleaved at specific intervals as described above. The system server 34 sends a parameter packet at predetermined intervals (asynchronously with run packets), and the converter sends an information packet at predetermined intervals (asynchronously with the run packets).

The converters 24a, 24b, 24c reset on several conditions. In every case, an indicator lamp or LED will signal when a reset occurs. The converters 24a, 24b, 24c reset when no apheresis instrument packets are received for 3 seconds. The converters 24a, 24b, 24c also reset when the system server 34 sends a reset packet, when an internal software fault causes a hardware reset, and when no system server 34 packets are seen for 8 seconds.

During a system server 34 outage, the converters 24a, 24b, 24c will continuously reset until the system server 34 application starts up again. When the system server 34 application broadcasts a reset packet, all of the converters 24a, 24b, 24c reset, and the boot procedure continues as if the system server 34 was booted first, and all the converters 24a, 24b, 24c were booted later.

### Practical Example

Referring to Figure 95, a method of using the apparatus of the present invention for automating the workflow within a blood collection facility is illustrated in flowchart format. This method includes monitoring the apheresis processes for the occurrence of specific events, and routing data regarding these events to PDAs or personal mobile devices for operator-assisted logging. The data provides a snapshot of the operational status of the apheresis instrument and is stored along with the logged event. Additional events can be logged directly on the PDAs through menu selections. The system also provides a means for performing verification of input data logged with each event.

The data snapshots can be displayed on a web browser in real time or near real time. The system also provides HTML-based query and reporting of aggregate data
from the logged events and snapshots. Open standard protocol is provided for
transferring the aggregate data from/to other blood facilities' computer systems. The aggregate data can be used to drive business decisions in operator training, inventory management, donor recruitment, and machine maintenance and servicing.

The method of the present invention begins with a donor walking into the donation site. The donor produces his ID card. The receptionist scans the donor ID card. An RF handheld terminal/web client, a PDA, with a built in bar code scanner is used to prompt the screener to scan the donor ID card bar code. The system server verifies the donor ID by searching a database and displays an eligibility outcome. The database search is made automatically when the donor ID card is read. The donor ID input causes a uniform resource locator (URL) to run and connects the web client to a hypertext transfer protocol (HTTP) server via a wireless link. The system server runs an application to search against a national donor eligibility database or, alternatively, the central server's DMS. The result is returned to the client application. The result of the search is returned instantaneously. If the donor is not eligible, the computer displays the eligibility date for this donor, and the donor is deferred. Otherwise, the site receptionist prepares to take the vital signs and medical history of the donor.

Next, the vital signs and medical history of the donor are transmitted to a donor screening database. The donor vital signs are input and stored in a donor screening database. This is accomplished by entering the vital sign data in an HTML form located on the handheld terminal. The form is then submitted to the system server via the mobile module.

Similarly, responses to a donor medical history checklist are entered electronically. The medical history data are entered on an HTML form on the handheld terminal. The form is then submitted to the system server.

Donor eligibility is automatically derived based on the input donor ID, the input donor vital sign data, and the donor's medical history data. A set criteria for eligibility is predetermined and configurable within the database on the system server. The system server runs an application to verify against the set of criteria and returns the outcome to the client.

If the donor's vital signs are acceptable and the donor is eligible, an identifier such as a bleed number is generated and queued in a scheduling database. The scheduling database now points to or has access to donor demographics, vital signs, and medical history, and the donor's weight is used by the system server to calculate a nomogram; i.e. the volume of blood or blood component to collect. However, if the vital signs are unacceptable, the donor is again deferred.

The bleed number and the donor ID may be identical. However, the bleed number is preferably a unique identifier that is generated once for each separate donation. A unique bleed number allows the system to differentiate between different bleed or collection procedures performed at different times on the same donor. This is important for traceability and donor evaluations, for example, tracking when the donor last underwent a bleed or collection procedure.

If the donor is accepted, the system server runs an application generated for a qualified donor and returns the approval to the client. The bleed number is printed on a bar code label using a portable printer attached to the handheld terminal. The bleed number is linked to the DMS. The system server creates a donation data file indexed on the bleed number. The donation data comprises the bleed number, the donor's vital signs, and the donor ID. The bleed number is queued and maintained electronically for daily scheduling. The system server maintains a FIFO list of active bleed numbers for the daily schedule. A count of the number of pending donations is also maintained. Bleed number ranges can be allotted at regular intervals before given out, and can be tracked based on lots, among other tracking methods described herein.

All PDA/scanners and apheresis instruments have network access to the database server(s), including the daily scheduling data. Accordingly, each instrument is a net appliance with an IP address. The instruments communicate over wire links and wireless links with a bridge sitting on a wired network. Each PDA/scanner and monitoring personal computer, thus, functions as a web client. In a preferred embodiment, the instruments are set up to send out data, including events and other data taken during the collection process, at regular time intervals to the facility server, which in-turn can send such information to the main system server, and related databases. Another contemplated embodiment includes using a server directly on the instrument. One PDA/scanner function includes being able to mimic the actual screen display at any point in real time, of each networked instruments, when the PDA/scanner is set to view such information. If an apheresis instrument is available, and the bleed number queue is not empty, a phlebotomist transmits a request from the apheresis instrument to the database. The first available bleed number from the queue is then removed from the queue and allocated to the available apheresis instrument.

An LED display at the apheresis center's reception area blinks the current bleed number and the apheresis instrument that is available. This instructs the waiting donors to proceed to the available instrument.

Therefore, each apheresis instrument includes a method for the phlebotomist to send a request for a donor to the scheduling database. The phlebotomist pushes a request button which causes a URL to run and connect to the system server. In response to the request for a donor, the scheduling database returns a bleed number to the apheresis instrument. This is accomplished as an application runs on the server to retrieve the next available bleed number. The relevant donor information is retrieved and returned to the apheresis instrument or PDA by another application.

In addition, the apheresis instrument or a PDA has a method to notify the scheduling application the apheresis instrument's readiness to accept a donor. Here, the phlebotomist pushes a button which causes a URL to run and connect to the system server. In response to the apheresis instrument ready notification, the instrument identity and bleed number is broadcast on a public display system. Here, an application is run on the system server to output the instrument identity and the bleed number to the public display system.

Future apheresis instruments may be capable of configuring themselves based on the donor information received. The instrument would read the donor information received from the system server and load a system configuration file. Here, the bleed number is inputted into the ready apheresis instrument for verification, instead of the PDA/scanner.

Before each action is taken by each operator (such as a phlebotomist) at each instrument, the operator scans (can be a requirement) their own ID card/badge, and the system tracks all actions performed by that operator, and at each instrument, are associated with the operator ID code. The operator typically must scan the machine code first, or vice versa.

Next, at the apheresis instrument or PDA/scanner, the bleed number is scanned by the phlebotomist. This bleed number must match the information retrieved by the PDA or the instrument from the system server. The bleed number is read into the apheresis instrument or PDA/scanner and compared with the donor information previously downloaded from the server.

The phlebotomist via the PDA or the apheresis instrument notifies the scheduling database when a donor has been accepted at the apheresis instrument. A bleed number match causes a URL to run and connect to the system server. The system server then updates the waiting list of donors by removing the donor from the list by running an application to remove the bleed number from the queue. Thus, the daily donor schedule is updated.

Some of the donor acceptance criteria are input from a separate customer system and others are set up as a general rule for the facility. For example, the facility system communicates information to the central server such as the donor ID that has just been registered, the unique bleed number, and the donor's weight. When the donor arrives at the bedside, his barcoded ID and bleed number are scanned by the PDA/scanner to match what the server is expecting. The donor's weight is used by the operator to calculate a nomogram. When the operator enters the nomogram, the input is electronically transmitted to the system server which compares the nomogram to the system server's calculation. If a match is not found, the system server warns the operator of the error.

The phlebotomist then scans a blood collection kit's (i.e. a disposable kits/solutions/transfer pack) identification code, usually a bar code, and enters the relevant information to begin the apheresis procedure. All input and output data associated with the apheresis procedure are stored in the database. These include the access site, the collection volumes, solution volumes, start and end times, donor reactions, and blood/plasma losses. A bar code scanner is, thus, either attached directly to the apheresis instrument or the PDA is used to collect and transmit the information.

The system and method of the invention also provides a precheck of all of the disposable kits/solutions/transfer packs used in the blood collection for acceptability before a bleed (procedure) can begin. The system server also verifies that the disposable kits are the right type and within expiration date, the operator is authorized to perform the procedure, and the apheresis instrument is approved. This accomplished by comparing this data with the data configured in the system server for the facility. The lot information may also be verified against a valid lot list on the system server, if one is maintained. This way the apheresis instrument can reject a lot if it has been recalled. If the disposable kit fails the precheck, a warning is transmitted by the system server.

The apparatus employed in the system also tracks usage and inventory of disposable kits/solutions/transfer packs. Inventory of the disposable kits/solutions/transfer packs is tracked via an inventory application. The instrument immediately notifies an inventory application of the disposable kits/solutions/transfer packs' usage. The bar code input cause URLs to run and connect to the system server. The inventory application updates the supply level based on usage. The system server runs an application to update a supplies inventory database. The system server invokes an additional connection to an external server at a supplier's facility. The supplier's server in turn updates its orders database.

As described above, the operator logs his/her identity into the system server through the PDA/scanner. The system also includes a database of the phlebotomists' qualifications. For example, certain operators are only qualified to perform certain procedures. When an operator attempts to log in his/her identity and perform a planned blood collection related procedure for which he/she is not unqualified to perform, the system server will produce a warning, or alternatively, prevent the procedure from continuing altogether. rhe apparatus provides an automatic trace from the bleed number to all inputs and outputs that are involved in the collection of blood or blood components 136. Thus, the bleed number can be traced to the donor, the disposable kits, the facility operator, and the apheresis instrument. This has not been possible, or was a very laborious task. For the traceability to be accurate, all information must be linked or synched with the apheresis instrument and the bleed number. Therefore, each apheresis instrument has a unique serial number programmed into its memory. Each instrument also has a bar code label associated with it which may or may not be the same as its serial number, and a media access control (MAC) address programmed into the NetDev connected to the apheresis instrument. As phlebotomist goes through the administrative set up function which sets up the apheresis instrument. The serial number, bar code and MAC address are entered into a table within the system server to identify the apheresis instrument. When the apheresis instrument is powered up, it sends the bootp request to the system server, which in turn returns an IP address to the instrument. From then on, the data packets from the instrument are identified by the system server through apheresis instrument's IP address.

This is all transparent to the phlebotomist. From the phlebotomist's point of view, he/she scans the bar code on the apheresis instrument to set up the apheresis instrument and prepares for a bleed procedure. The system server looks up the serial number of the apheresis instrument from the bar code, and creates a bleed record with the donor bleed number, donor ID, the instrument serial number, and the identity of the disposable kit(s) used. Every time a data packet is received from the apheresis instrument, the system server also looks up the apheresis instrument's serial number (IP to MAC to serial number) and inserts any relevant information into the corresponding bleed record.

The system also routes apheresis instrument information to the PDAs based on the apheresis instrument that is assigned to or associated with an individual PDA. This is used like a paging system. This enables the phlebotomists to log predefined events (such as instrument alarms) with only a few key strokes. The system is flexible enough to log events from different vendor's instruments. The specific target routing is performed when the phlebotomist scan the apheresis instrument bar codes at the beginning of a procedure. Since each data packet is traceable to the ID of the originating apheresis instrument, and each PDA has its own network ID, the system server can identify which PDA to route the information to.

The pre-processing (pre-venipuncture) configuration of the apheresis instrument is recorded electronically. The information is stored locally on the instrument and transmitted to the system server at the end of the collections. Alternatively, just prior to venipuncture, the instrument configuration is submitted as an HTML form to the system server. The system server then runs an application to write the information into a configuration database file.

The statistics and summaries at the end of the collection are also stored electronically. The information is submitted as an HTML form to the system server. The system server then runs an application to write the information into a donation database file. The system server also maintains a count of the total number of blood product units collected for the day.

The system may provide statistics of a procedure execution that is linked to a phlebotomist or phlebotomists, e.g., the events the operator handled, the operator's error rate (whether they resulted in instrument alarms or not), how many procedures the operator has performed, etc. This provides a means for focused training of individual operators.

The system also captures the history of keystrokes performed on the instrument. This can be used later for troubleshooting of instruments or procedures.

Procedural exceptions (alarms and/or adjustments) during collection are recorded electronically. The information is stored locally on the apheresis instrument and transmitted at the end of the collection. Alternatively, whenever an exception is performed, the information is submitted as an HTML form to the system server. The server then runs an application to write the information into a procedure exception database file.

The apparatus also logs the apheresis instrument's cumulative operating hours, calibration, and any service maintenance performed, as well as any malfunctions and/or error occurrences. Each instrument records its cumulative operating hours, calibration and other service maintenance performed, as well as malfunction and error occurrences in its internal memory. On a weekly base or otherwise, an application runs to collect these records from the instruments. This is done by running a URL which connects to the instrument. The instrument in turn runs an application to return the records. Based on the returned records, the application makes a recommendation on service call on each instrument.

The apparatus records blood/plasma loss during a collection. Automatic reports are generated to the appropriate establishments when the loss is greater than acceptable limits. The instrument monitors and calculates blood/plasma loss during a collection. The information is transmitted as part of the summary record at the end of a collection. When an unacceptable blood/plasma loss record is received, the system server connects to the central server and submits a donor deferment report. Other reports pertaining to any of the inputs or outputs may also be requested and generated.

Next, each apheresis instrument's operation can be monitored at a central workstation, generally a web client or the system server, or through the PDA/scanner. The operator configures a list of parameters for real time display. If necessary a message or instruction can be transmitted by the operator to a specific instrument for display. The workstation can also check the total units of blood components collected for the day, as well as the number of donors on the waiting list. The workstation monitors parameters associated with the collection process on each apheresis instrument. Accordingly, the monitoring workstation communicates to all of the PDA/scanners and apheresis instruments within the apparatus via a wired or wireless network.

The apparatus routes instrument information to the PDAs based on the apheresis instruments that are assigned to the individual PDAs. This allows operators to view and log predefined events (such as instrument alarms) with only a few key strokes. The specific target routing by the system server is done is accomplished when the operator scans the apheresis instrument barcodes at the beginning of a procedure. Since each data packet for the apheresis instrument is traceable to the identity of the apheresis instrument, and each PDA has its own network identity, the system server can route the information to the proper PDA.

The workstation application sets up a list of parameters to monitor based on user preference. The application runs a URL/web query to the system server running on the instrument. The instrument runs an application to read the requested parameters and returns them to the application. A java applet runs at the workstation to provide continuous trending of the data received. Alternatively, a stream server is implemented on the instrument and data are continuously streamed to the application and updated in real time. ASP's are implemented on the instrument. An ASP is a specification for a dynamically created web page that utilizes ActiveX scripting. Dynamic hypertext markup language (DHTML) is run on the workstation to display data dynamically.

The workstation also communicates messages to the apheresis instrument. The workstation application submits an HTML form containing messages to the instrument. An application runs on the instrument to display a pop up message.

The workstation also has a function to display the cumulative blood product units collected, as well as the total number of donors in the waiting list. The workstation application displays live real time statistics through a java applet, streaming, or DHTML.

Blood and blood component inventory can be tracked by the system. When the apheresis collection is completed, a bar code label identifying the product and volume is printed and affixed to the product. The blood product then moves to a post-processing area such as pathogen inactivation. A different bar code label can be printed and affixed to identify it as having been post-processed. The blood product then moves into the freezer storage area, where each incoming unit is scanned and the corresponding product inventory database is updated.

The instrument prints a bar code label containing necessary information identifying the product. The instrument includes a function to print bar code labels on an attached printer. The apheresis instrument prints a bar code label identifying the product as being processed. Accordingly, the apheresis instrument also includes a function to print bar code labels on an attached printer.

Incoming blood products are identified electronically prior to entering the freezer. A bar code scanner is provided to scan incoming blood products. The blood product inventory is updated for each unit stored in the freezer. Each bar code read from a product unit triggers a URL with the bar code information sent to the system server. The system server then runs an application to update the product inventory.

Next, the blood product stays in the freezer area until lab test results of the blood or blood component collected are received. At the shipping area, each blood product unit is scanned and compared with the lab test database and other release requirements. All approved product units are then packaged into a shipping box designated for a certain destination. The center inventory database is updated with the outgoing units, while the end user gets informed of the shipment immediately.

A sample test report is used to electronically identify blood product units for release. The sample test report is downloaded to an application on a daily basis. The bleed number on each blood product unit is scanned and verified with that of the approved test sample list. A unit can be released if a match is found.

The contents and destination of each shipping box is identified. Each blood product unit is scanned before going into the shipping box. When the capacity of the box is reached, a shipping label is printed. A record of each shipping box identification and its contents are also stored in a database

Finally, the blood product inventory is maintained up-to-date. A blood product inventory database is updated with each outgoing unit. When a box is scaled and confirmed, a URL runs to connect to an external server at a customer's site. A data file containing these records is transferred to the external server. The external server then updates its inventory receivables list.

It should be understood that when the word "scanned" or "scanner"is used herein, it is contemplated that such actions and information can be entered in another a manner, such as through a touch screen keyboard, and vice versa.

The above-described invention can also be implemented and used within a blood testing and pathogen inactivation facility/system, and/or within a fluid tracking system.

It is further contemplated that all of the features and advantages of the PDA/scanner type device can be implemented directly into the instrument (apheresis or other instrument).

## Claims

1. A system for managing inventory of blood component collection soft goods in a blood component collection facility and for preventing the use of quarantined blood component collection soft goods, the system comprising:
an operator identifier corresponding to a blood component collection instrument operator;
a blood component collection instrument for collecting a blood component from a blood component donor in a blood component collection soft good;
a system computer in data communication with a system database;
an interface operably connected to the system computer and having a reader for receiving the operator identifier and transmitting the operator identifier to the system computer and for receiving separate input of a blood component collection soft good identifier and transmitting the blood component collection soft good identifier to the system database for managing inventory;
wherein the system computer is operably connected to the blood component collection instrument, the system computer running a blood component collection application for at least a portion of a blood component collection process, wherein the system database has a blood component collection soft good inventory and quarantine blood component collection soft good information, said system computer is adapted to process said inventory and quarantine blood component collection soft good information prior to the use of the blood component collection soft good and the system computer is further adapted to transmit a warning to the blood component collection instrument and cause the blood component collection instrument to reject the blood component collection soft good upon detection of a quarantined blood component collection soft goods identifier.

2. The system of Claim 1, wherein the blood component collection soft good is selected from a group consisting of a blood component collection kit, a blood component collection solution, and a blood component collection transfer pack.

3. The system of Claim 1, wherein the blood component collection instrument further comprises a blood component instrument identifier, wherein the reader receives an input of the blood component collection instrument and wherein the blood collection component application associates the blood component collection instrument identifier with the blood component donor identifier and the operator identifier.

4. The system of Claim 3, wherein the reader receives separate input of the blood component collection instrument identifier, the blood component donor identifier and the operator identifier.

5. The system of Claim 3 further comprising a blood component collection donor identifier corresponding to a blood component donor, wherein the blood component collection donor identifier is transmitted to the system computer for storing the blood component collection donor identifier in the system database and for associating the blood component collection donor identifier with at least one of the blood component collection soft good identifier and the blood component collection instrument identifier.

6. The system of Claim 1 further comprising:
a system communication conduit for operably connecting the system computer to the blood component collection instrument; and
a system communication protocol for facilitating communication on the communication conduit between the system computer and the blood component collection instrument.

7. The system of Claim 6, wherein the system communication protocol is Ethernet.

8. The system of Claim 6, wherein the system communication protocol is TCP/IP.

9. The system of Claim 6, further comprising:
a network server being operably connected to the system computer via a network communication conduit; and
a web interface being operably connected to the system computer for facilitating access to the blood component collection process, wherein the interface receives data from the system computer.

10. The system of Claim 9, further comprising a web server being operably connected to the system computer and a web browser, the web server being operably responsive to a web browser wherein the information stored in the system computer can be accessed.

11. The system of Claim 1, wherein the system database further comprises separate inventory data for each of a plurality of different types of soft goods.

12. The system of Claim 1 which is configured to generate a notification when the blood component collection soft good inventory data is modified to a value which is lower than a predetermined value.

13. The system of Claim 12 which is configured to transmit the notification to a remote location for restocking blood component collection soft good inventory and wherein the notification comprises providing a reorder option corresponding to the blood component collection soft good associated with the blood component collection soft good identifier.

14. A method for managing inventory of blood component collection soft goods in a blood component collection facility and for preventing use of quarantined blood component collection soft goods in a system including a blood component collection instrument for collecting a blood component from a blood component donor, a system computer in data communication with a system database and an interface operably connected to the system computer, the interface having a reader, the method comprising the steps of:
receiving a an operator identifier via the reader corresponding to a blood component collection instrument operator and transmitting the operator identifier to the system computer;
accessing a system database having a blood component collection soft good inventory;
receiving separate input of a blood component collection soft good identifier; and
transmitting the blood component collection soft good identifier to the system database for managing inventory;
processing the blood component collection soft good inventory and any quarantine blood component collection soft good information prior to use of the blood component collection soft good;
indicating that at least a portion of the blood component collection soft good is quarantined based on said processing and prior to the use of the blood component collection soft good; and
detecting quarantined blood component collection soft goods such that, upon detection of a quarantined blood component collection soft goods identifier, the system computer transmits a warning to the blood component collection instrument and causes the blood component collection instrument to reject the blood component collection soft good.

15. The method of claim 14, further comprising the steps of:
receiving a blood component collection instrument identifier and,
associating the blood component collection instrument identifier with the blood component donor identifier and the operator identifier.

16. The method of Claim 14, further comprising the step of:
generating a notification when the blood component collection soft good inventory data is modified to a value which lower than a predetermined value.

17. The method of Claim 16, further comprising the steps of:
generating a notification comprising providing a reorder option corresponding to the blood component collection soft good associated with the blood component collection soft good identifier; and
transmitting the notification to a remote location for restocking blood component collection soft good inventory.

## Patentansprüche

1. System zum Verwalten des Bestands von Blutkomponentenentnahme-Soft-Goods bzw. -Einwegartikeln in einer Blutkomponentenentnahmeanlage und zum Verhindern der Verwendung von unter Quarantäne gestellten Blutkomponentenentnahme-Soft-Goods bzw. -Einwegartikeln, wobei das System umfasst:
eine Operator- bzw. Bedienerkennung, die einem Blutkomponentenentnahme-Instrumentenoperator bzw. -bediener entspricht;
ein Blutkomponentenentnahmeinstrument zum Entnehmen einer Blutkomponente von einem Blutkomponentenspender in einem Blutkomponentenentnahme-Soft-Good bzw. -Einwegartikel;
einen Systemcomputer in Datenkommunikation mit einer Systemdatenbank;
eine Schnittstelle, die operabel mit dem Systemcomputer verbunden ist und ein Lesegerät zum Empfangen der Bedienerkennung und zum Übertragen der Bedienerkennung an den Systemcomputer und zum Empfangen einer separaten Eingabe einer Blutkomponentenentnahme-Soft-Good- bzw. - Einwegartikelkennung und zum Übertragen der Blutkomponentenentnahme-Soft-Good-Kennung an die Systemdatenbank zum Verwalten des Bestands aufweist;
wobei der Systemcomputer operabel mit dem Blutkomponentenentnahmeinstrument verbunden ist, wobei der Systemcomputer eine Blutkomponentenentnahmeanwendung für zumindest einen Teil eines Blutkomponentenentnahmeprozesses ausführt, wobei die Systemdatenbank einen Blutkomponentenentnahme-Soft-Good bzw. - Einwegartikelbestand und Informationen zu unter Quarantäne gestellten Blutkomponentenentnahme-Soft-Goods bzw. -Einwegartikeln aufweist, wobei der Systemcomputer ausgelegt ist, den Bestand und die Informationen zu den unter Quarantäne gestellten Blutkomponentenentnahme-Soft-Goods vor der Verwendung des Blutkomponentenentnahme-Soft-Goods zu verarbeiten, und wobei der Systemcomputer ferner ausgelegt ist, eine Warnung an das Blutkomponentenentnahmeinstrument zu übertragen und das Blutkomponentenentnahmeinstrument zu veranlassen, das Blutkomponentenentnahme-Soft-Good bei Detektion einer Quarantäne-Blutkomponentenentnahme-Soft-Good- bzw. -Einwegartikelkennung abzulehnen.

2. System nach Anspruch 1, wobei das Blutkomponentenentnahme-Soft-Good aus einer Gruppe bestehend aus einem Blutkomponentenentnahme-Kit, einer Blutkomponentenentnahmelösung und einem Blutkomponentenentnahme-Übertragungspack ausgewählt ist.

3. System nach Anspruch 1, wobei das Blutkomponentenentnahmeinstrument ferner eine Blutkomponenteninstrumentenkennung umfasst, wobei das Lesegerät eine Eingabe des Blutkomponentenentnahmeinstruments empfängt und wobei die Blutkomponentenentnahmeanwendung die Blutkomponentenentnahme-Instrumentenkennung mit der Blutkomponentenspenderkennung und der Bedienerkennung verknüpft.

4. System nach Anspruch 3, wobei das Lesegerät eine separate Eingabe der Blutkomponentenentnahme-Instrumentenkennung, der Blutkomponentenspenderkennung und der Bedienerkennung erhält.

5. System nach Anspruch 3, ferner umfassend eine Blutkomponentenentnahme-Spenderkennung, die einem Blutkomponentenspender entspricht, wobei die Blutkomponentenentnahme-Spenderkennung an den Systemcomputer übertragen wird, um die Blutkomponentenentnahme-Spenderkennung in der Systemdatenbank zu speichern und die Blutkomponentenentnahme-Spenderkennung mit zumindest einer der Blutkomponentenentnahme-Soft-Good-Kennung und der Blutkomponentenentnahme-Instrumentenkennung zu verknüpfen.

6. System nach Anspruch 1, ferner umfassend:
eine Systemkommunikationsleitung zum operablen Verbinden des Systemcomputers mit dem Blutkomponentenentnahmeinstrument; und
ein Systemkommunikationsprotokoll zum Erleichtern der Kommunikation auf der Kommunikationsleitung zwischen dem Systemcomputer und dem Blutkomponentenentnahmeinstrument.

7. System nach Anspruch 6, wobei das Systemkommunikationsprotokoll Ethernet ist.

8. System nach Anspruch 6, wobei das Systemkommunikationsprotokoll TCP/IP ist.

9. System nach Anspruch 6, ferner umfassend:
einen Netzwerkserver, der über eine Netzwerkkommunikationsleitung operabel mit dem Systemcomputer verbunden ist; und
eine Webschnittstelle, die operabel mit dem Systemcomputer zum Erleichtern des Zugriffs auf den Blutkomponentenentnahmeprozess verbunden ist, wobei die Schnittstelle Daten von dem Systemcomputer empfängt.

10. System nach Anspruch 9, ferner umfassend einen Webserver, der operabel mit dem Systemcomputer und einem Webbrowser verbunden ist, wobei der Webserver operabel auf einen Webbrowser anspricht, wobei auf die in dem Systemcomputer gespeicherten Informationen zugegriffen werden kann.

11. System nach Anspruch 1, wobei die Systemdatenbank ferner separate Bestandsdaten für jedes einer Mehrzahl unterschiedlicher Arten von Soft Goods bzw. Einwegartikeln umfasst.

12. System nach Anspruch 1, das konfiguriert ist, eine Benachrichtigung zu erzeugen, wenn die Blutkomponentenentnahme-Soft-Good-Bestandsdaten auf einen Wert geändert werden, der niedriger als ein vorbestimmter Wert ist.

13. System nach Anspruch 12, das konfiguriert ist, die Benachrichtigung an einen entfernten Ort zum Wiederauffüllen des Blutkomponentenentnahme-Soft-Good-Bestands zu übertragen, und wobei die Benachrichtigung das Bereitstellen einer Nachbestellungsoption entsprechend dem Blutkomponentenentnahme-Soft-Good umfasst, das mit der Blutkomponentenentnahme-Soft-Good-Kennung verknüpft ist.

14. Verfahren zum Verwalten des Bestands von Blutkomponentenentnahme-Soft-Goods bzw. -Einwegartikeln in einer Blutkomponentenentnahmeanlage und zum Verhindern der Verwendung von unter Quarantäne gestellten Blutkomponentenentnahme-Soft-Goods bzw. -Einwegartikeln in einem System, das ein Blutkomponentenentnahmeinstrument zum Entnehmen einer Blutkomponente von einem Blutkomponentenspender, einen Systemcomputer in Datenkommunikation mit einer Systemdatenbank und eine Schnittstelle enthält, die operabel mit dem Systemcomputer verbunden ist, wobei die Schnittstelle ein Lesegerät aufweist, wobei das Verfahren die Schritte umfasst:
Empfangen einer Operator- bzw. Bedienerkennung über das Lesegerät, die einem Blutkomponentenentnahme-Instrumentenoperator bzw. -bediner entspricht, und Übertragen der Bedienerkennung an den Systemcomputer;
Zugreifen auf eine Systemdatenbank mit einem Blutkomponentenentnahme-Soft-Good- bzw. -Einwegartikelbestand;
Empfangen einer separaten Eingabe einer Blutkomponentenentnahme-Soft-Good- bzw. -Einwegartikelkennung; und
Übertragen der Blutkomponentenentnahme-Soft-Good-Kennung an die Systemdatenbank zum Verwalten des Bestands;
Verarbeiten des Blutkomponentenentnahme-Soft-Good-Bestands und jeglicher Informationen zu unter Quarantäne gestellten Blutkomponentenentnahme-Soft-Goods bzw. -Einwegartikeln vor der Verwendung des Blutkomponentenentnahme-Soft-Goods;
Angeben, dass zumindest ein Teil des Blutkomponentenentnahme-Soft-Goods basierend auf der Verarbeitung und vor der Verwendung des Blutkomponentenentnahme-Soft-Goods unter Quarantäne gestellt wird; und
Detektieren von unter Quarantäne gestellten Blutkomponentenentnahme-Soft-Goods, so dass bei Detektion einer Quarantäne-Blutkomponentenentnahme-Soft-Good- bzw. -Einwegartikelkennung der Systemcomputer eine Warnung an das Blutkomponentenentnahmeinstrument sendet und das Blutkomponentenentnahmeinstrument veranlasst, das Blutkomponentenentnahme-Soft-Good abzulehnen.

15. Verfahren nach Anspruch 14, ferner umfassend die Schritte:
Empfangen einer Blutkomponentenentnahme-Instrumentenkennung und
Verknüpfen der Blutkomponentenentnahme-Instrumentenkennung mit der Blutkomponentenspenderkennung und der Bedienerkennung.

16. Verfahren nach Anspruch 14, ferner umfassend den Schritt:
Erzeugen einer Benachrichtigung, wenn die Blutkomponentenentnahme-Soft-Good-Bestandsdaten auf einen Wert geändert werden, der niedriger als ein vorbestimmter Wert ist.

17. Verfahren nach Anspruch 16, ferner umfassend die Schritte:
Erzeugen einer Benachrichtigung, die das Bereitstellen einer Nachbestellungsoption entsprechend dem Blutkomponentenentnahme-Soft-Good umfasst, das mit der Blutkomponentenentnahme-Soft-Good-Kennung verknüpft ist; und
Übertragen der Benachrichtigung an einen entfernten Ort zum Wiederauffüllen des Blutkomponentenentnahme-Soft-Good-Bestands.

## Revendications

1. Système permettant de gérer l'inventaire de biens non durables issus d'un prélèvement de composants sanguins dans une installation de prélèvement de composants sanguins et permettant d'éviter l'utilisation de biens non durables issus d'un prélèvement de composants sanguins mis en quarantaine, le système comprenant :
un identifiant opérateur correspondant à un opérateur d'instrument de prélèvement de composants sanguins ;
un instrument de prélèvement de composants sanguins pour prélever un composant sanguin sur un donneur de composant sanguin dans un bien non durable issu d'un prélèvement de composants sanguins ;
un ordinateur système en communication, par transmission de données, avec une base de données système ;
une interface connectée de manière opérationnelle à l'ordinateur système et possédant un lecteur pour recevoir l'identifiant de l'opérateur et transmettre l'identifiant de l'opérateur à l'ordinateur système et pour recevoir une entrée distincte d'un identifiant de biens non durables issus d'un prélèvement de composants sanguins et transmettre l'identifiant de biens non durables issus d'un prélèvement de composants sanguins à la base de données système permettant de gérer l'inventaire ;
dans lequel l'ordinateur système est connecté de manière opérationnelle à l'instrument de prélèvement de composants sanguins, l'ordinateur système exécutant une application de prélèvement de composants sanguins pour au moins une partie d'un procédé de prélèvement de composants sanguins, dans lequel la base de données système possède un inventaire de biens non durables issus d'un prélèvement de composants sanguins et des informations relatives aux biens non durables issus d'un prélèvement de composants sanguins en quarantaine, ledit ordinateur système est adapté pour traiter lesdits inventaire et informations relatives aux biens non durables issus d'un prélèvement de composants sanguins en quarantaine avant l'utilisation du bien non durable issu d'un prélèvement de composants sanguins et l'ordinateur système est en outre adapté pour transmettre un avertissement à l'instrument de prélèvement de composants sanguins et amener l'instrument de prélèvement de composants sanguins à rejeter le bien non durable issu d'un prélèvement de composants sanguins lors de la détection d'un identifiant de biens non durables issus d'un prélèvement de composants sanguins mis en quarantaine.

2. Système selon la revendication 1, dans lequel le bien non durable issu d'un prélèvement de composants sanguins est choisi dans un groupe constitué d'un kit de prélèvement de composants sanguins, d'une solution de prélèvement de composants sanguins et d'un sac de transvasement de prélèvement de composants sanguins.

3. Système selon la revendication 1, dans lequel l'instrument de prélèvement de composants sanguins comprend en outre un identifiant d'instrument de composants sanguins, dans lequel le lecteur reçoit une entrée de l'instrument de prélèvement de composants sanguins et dans lequel l'application de prélèvement de composants sanguins associe l'identifiant de l'instrument de prélèvement de composants sanguins à l'identifiant du donneur de composant sanguin et à l'identifiant de l'opérateur.

4. Système selon la revendication 3, dans lequel le lecteur reçoit une entrée distincte de l'identifiant de l'instrument de prélèvement de composants sanguins, de l'identifiant du donneur de composant sanguin et de l'identifiant de l'opérateur.

5. Système selon la revendication 3 comprenant en outre un identifiant donneur de prélèvement de composants sanguins correspondant à un donneur de composant sanguin, dans lequel l'identifiant du donneur de prélèvement de composants sanguins est transmis à l'ordinateur système pour stocker l'identifiant du donneur de prélèvement de composants sanguins dans la base de données système et pour associer l'identifiant du donneur de prélèvement de composants sanguins à au moins l'un de l'identifiant de biens non durables issus d'un prélèvement de composants sanguins et de l'identifiant de l'instrument de prélèvement de composants sanguins.

6. Système selon la revendication 1 comprenant en outre :
un canal de communication système pour connecter de manière opérationnelle l'ordinateur système à l'instrument de prélèvement de composants sanguins ; et
un protocole de communication système pour faciliter la communication sur le canal de communication entre l'ordinateur système et l'instrument de prélèvement de composants sanguins.

7. Système selon la revendication 6, dans lequel le protocole de communication système est Ethernet.

8. Système selon la revendication 6, dans lequel le protocole de communication système est TCP/IP.

9. Système selon la revendication 6, comprenant en outre :
un serveur de réseau étant connecté de manière opérationnelle à l'ordinateur système via un canal de communication de réseau ; et
une interface Web étant connectée de manière opérationnelle à l'ordinateur système pour faciliter l'accès au procédé de prélèvement de composants sanguins, dans lequel l'interface reçoit des données à partir de l'ordinateur système.

10. Système selon la revendication 9, comprenant en outre un serveur Web étant connecté de manière opérationnelle à l'ordinateur système et un navigateur Web, le serveur Web répondant de manière opérationnelle à un navigateur Web dans lequel les informations stockées dans l'ordinateur système sont accessibles.

11. Système selon la revendication 1, dans lequel la base de données système comprend en outre des données d'inventaire distinctes pour chacun d'une pluralité de différents types de biens non durables.

12. Système selon la revendication 1 qui est configuré pour générer une notification lorsque les données de l'inventaire de biens non durables issus d'un prélèvement de composants sanguins sont modifiées à une valeur qui est inférieure à une valeur prédéterminée.

13. Système selon la revendication 12 qui est configuré pour transmettre la notification à un site distant pour restocker l'inventaire de biens non durables issus d'un prélèvement de composants sanguins et dans lequel la notification comprend la fourniture d'une option classement correspondant au bien non durable issu d'un prélèvement de composants sanguins associé à l'identifiant de biens non durables issus d'un prélèvement de composants sanguins.

14. Procédé permettant de gérer l'inventaire de biens non durables issus d'un prélèvement de composants sanguins dans une installation de prélèvement de composants sanguins et permettant d'éviter l'utilisation de biens non durables issus d'un prélèvement de composants sanguins mis en quarantaine dans un système incluant un instrument de prélèvement de composants sanguins pour prélever un composant sanguin sur un donneur de composant sanguin, un ordinateur système en communication, par transmission de données, avec une base de données système et une interface connectée de manière opérationnelle à l'ordinateur système, l'interface possédant un lecteur, le procédé comprenant les étapes de :
réception d'un identifiant opérateur via le lecteur correspondant à un opérateur d'instrument de prélèvement de composants sanguins et transmission de l'identifiant de l'opérateur à l'ordinateur système ;
accès à une base de données système possédant un inventaire de biens non durables issus d'un prélèvement de composants sanguins ;
réception d'une entrée distincte d'un identifiant de biens non durables issus d'un prélèvement de composants sanguins ; et
transmission de l'identifiant de biens non durables issus d'un prélèvement de composants sanguins à la base de données système permettant de gérer l'inventaire ;
traitement de l'inventaire de biens non durables issus d'un prélèvement de composants sanguins et de toutes informations relatives aux biens non durables issus d'un prélèvement de composants sanguins en quarantaine avant l'utilisation du bien non durable issu d'un prélèvement de composants sanguins ;
indication qu'au moins une partie du bien non durable issu d'un prélèvement de composants sanguins est mis en quarantaine sur la base dudit traitement et avant l'utilisation du bien non durable issu d'un prélèvement de composants sanguins ; et
détection de biens non durables issus d'un prélèvement de composants sanguins mis en quarantaine de sorte que, lors de la détection d'un identifiant de biens non durables issus d'un prélèvement de composants sanguins mis en quarantaine, l'ordinateur système transmette un avertissement à l'instrument de prélèvement de composants sanguins et amène l'instrument de prélèvement de composants sanguins à rejeter le bien non durable issu d'un prélèvement de composants sanguins.

15. Procédé selon la revendication 14, comprenant en outre les étapes de :
réception d'un identifiant instrument de prélèvement de composants sanguins et,
association de l'identifiant de l'instrument de prélèvement de composants sanguins à l'identifiant du donneur de composant sanguin et l'identifiant de l'opérateur.

16. Procédé selon la revendication 14, comprenant en outre l'étape de :
génération d'une notification lorsque les données de l'inventaire de biens non durables issus d'un prélèvement de composants sanguins sont modifiées à une valeur qui est inférieure à une valeur prédéterminée.

17. Procédé selon la revendication 16, comprenant en outre les étapes de :
génération d'une notification comprenant la fourniture d'une option classement correspondant au bien non durable issu d'un prélèvement de composants sanguins associé à l'identifiant de biens non durables issus d'un prélèvement de composants sanguins ; et
transmission de la notification à un site distant pour restocker l'inventaire de biens non durables issus d'un prélèvement de composants sanguins.
